# EUROPEAN PATENT APPLICATION

(11) **EP 4 442 681 A1**
(43) Date of publication of application: **09.10.2024**
(21) Application number: 22901597.9
(22) Date of filing: 04.11.2022
(51) Int. Cl.: C07D 251/24, C07D 405/04, C07D 409/04, H10K 99/00, H10K 50/00, C09K 11/06

(54) **HETEROCYCLIC COMPOUND, ORGANIC LIGHT-EMITTING ELEMENT COMPRISING SAME, AND COMPOSITION FOR ORGANIC MATERIAL LAYER**

(30) Priority: 03.12.2021 KR 20210172049
(71) Applicant: LT Materials Co., Ltd., Yongin-si, Gyeonggi-do 17118 (KR)
(72) Inventor: PARK, Geon-Yu, Yongin-si, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2022/017207
(87) International publication number: WO 2023/101238

(57) **Abstract**

The present specification relates to a heterocyclic compound represented by Chemical Formula 1, and an organic light emitting device and a composition for an organic material layer, including the same. Chemical Formula 1 is characterized in that at least one of the cores of the triphenylenyl group is substituted with deuterium, and has a feature capable of improving the service life of the organic light emitting device because the stability is improved.

## Description

### [Technical Field]

The present specification relates to a heterocyclic compound, and an organic light emitting device and a composition for an organic material layer, including the same.

### <Cross-Reference to Related Applications>

This application claims priority to and the benefit of Korean Patent Application No. 10-2021-0172049 filed in the Korean Intellectual Property Office on December 3, 2021, the entire contents of which are incorporated herein by reference.

### [Background Art]

An organic electroluminescence device is a kind of self-emitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multiple layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may perform a function such as hole injection, hole transport, electron blocking, hole blocking, electron transport or electron injection.

In order to improve the performance, service life, or efficiency of the organic light emitting device, there is a continuous need for developing a material for an organic thin film.

### <Related Art Document>

(Patent Document) US Patent No. 4,356,429

### [Disclosure]

### [Technical Problem]

The present invention has been made in an effort to provide a heterocyclic compound, and an organic light emitting device and a composition for an organic material layer, including the same.

### [Technical Solution]

In an exemplary embodiment of the present application, provided is a heterocyclic compound represented by the following Chemical Formula 1.

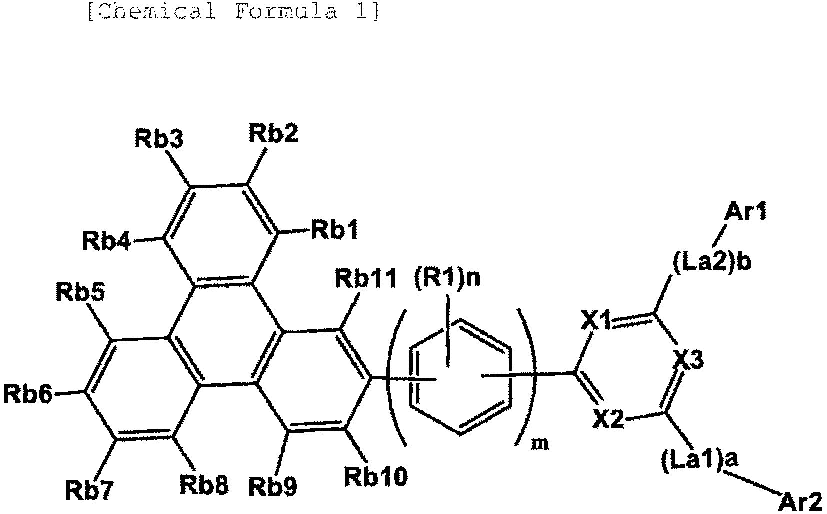

In Chemical Formula 1,
X1 to X3 are the same as or different from each other, and are each independently N; or CRa1, and at least one is N,
La1 and La2 are the same as or different from each other, and are each independently a direct bond; a substituted or unsubstituted C6 to C60 arylene group; or a substituted or unsubstituted C2 to C60 heteroarylene group,
R1 is hydrogen; or deuterium,
Ra1, Ar1 and Ar2 are the same as or different from each other, and are each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR',
Rb1 to Rb11 are the same as or different from each other, and are each independently hydrogen; or deuterium, and at least one of Rb1 to Rb11 is deuterium,
R, R' and R" are the same as or different from each other, and are each independently hydrogen; deuterium; a substituted or unsubstituted C1 to C60 alkyl group; or a substituted or unsubstituted C6 to C60 aryl group,
a, b and m are an integer from 1 to 4,
n is an integer from 0 to 4, and
when a, b, n and m are 2 or higher, substituents in the parenthesis are the same as or different from each other.

Further, in an exemplary embodiment of the present application, provided is an organic light emitting device including: a first electrode; a second electrode provided to face the first electrode; and an organic material layer having one or more layers provided between the first electrode and the second electrode, in which one or more layers of the organic material layer include the heterocyclic compound represented by Chemical Formula 1.

In addition, an exemplary embodiment of the present application provides an organic light emitting device in which an organic material layer including the heterocyclic compound of Chemical Formula 1 further includes a heterocyclic compound represented by the following Chemical Formula 2.

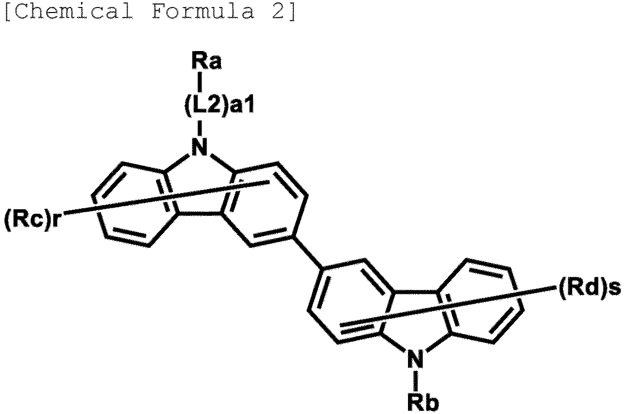

In Chemical Formula 2,
Rc and Rd are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P(=O)RR'; and -NRR', or two or more adjacent groups are bonded to each other to form a substituted or unsubstituted aromatic hydrocarbon ring or a substituted or unsubstituted hetero ring,
L2 is a direct bond; a substituted or unsubstituted C6 to C60 arylene group; or a substituted or unsubstituted C2 to C60 heteroarylene group,
Ra and Rb are the same as or different from each other, and are each independently -CN; -SiRR'R"; -P(=O)RR'; a substituted or unsubstituted C6 to C60 aryl group; or a substituted or unsubstituted C2 to C60 heteroaryl group,
R, R' and R" are the same as or different from each other, and are each independently hydrogen; deuterium; a substituted or unsubstituted C1 to C60 alkyl group; or a substituted or unsubstituted C6 to C60 aryl group,
a1 is an integer from 0 to 4,
r and s are an integer from 0 to 7, and
when a1, s and r are 2 or higher, substituents in the parenthesis are the same as or different from each other.

Further, another exemplary embodiment of the present application provides a composition for an organic material layer of an organic light emitting device, which includes the heterocyclic compound represented by Chemical Formula 1 and the heterocyclic compound represented by Chemical Formula 2.

### [Advantageous Effects]

A heterocyclic compound according to an exemplary embodiment of the present application can be used as a material for an organic material layer of an organic light emitting device. The heterocyclic compound can be used as a material for a hole injection layer, a hole transport layer, a light emitting layer, an electron transport layer, an electron injection layer, a charge generation layer, and the like in an organic light emitting device.

Specifically, the heterocyclic compound represented by Chemical Formula 1 corresponds to a compound which has a linking group of an arylene group in the core structure of a triphenylenyl group in which at least one is substituted with deuterium, and has an azine substituent. First, compounds substituted with deuterium, whose atomic mass is twice that of hydrogen, can have lower ground-state energy due to lower zero-point energy and vibrational energy than compounds substituted with hydrogen, and can result in a thin film in an amorphous state due to a reduction in collision caused by intermolecular vibration, and thus, have characteristics capable of improving the service life of an organic light emitting device.

That is, as shown in Chemical Formula 1, at least one of the core structures of the triphenylenyl group is substituted with deuterium to lower the ground state energy, thereby improving the stability of the compound and increasing the dissociation energy of the C-D bond, so that the compounds have characteristics capable of improving the service life of the organic light emitting device because the stability of the molecule is improved.

Further, both the heterocyclic compound represented by Chemical Formula 1 and the heterocyclic compound represented by Chemical Formula 2 can be used as a material for a light emitting layer of an organic light emitting device. In addition, when the heterocyclic compound represented by Chemical Formula 1 and the heterocyclic compound represented by Chemical Formula 2 are simultaneously used for the organic light emitting device, the driving voltage of the device may be lowered, the light efficiency of the device may be improved, and the service life characteristics of the device may be improved by the thermal stability of the compound.

### [Description of Drawings]

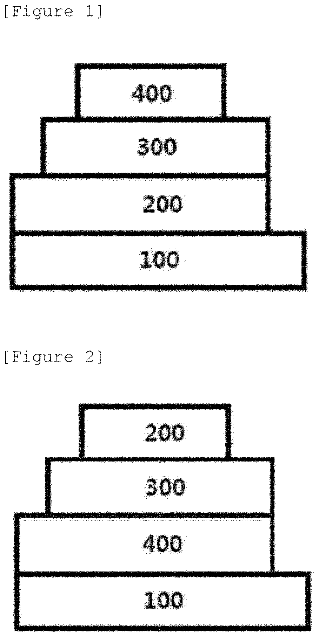

FIGS. 1 to 3 each are views schematically illustrating a stacking structure of an organic light emitting device according to an exemplary embodiment of the present application.

### [Explanation of Reference Numerals and Symbols]

- 100:: Substrate
- 200:: Positive electrode
- 300:: Organic material layer
- 301:: Hole injection layer
- 302:: Hole transport layer
- 303:: Light emitting layer
- 304:: Hole blocking layer
- 305:: Electron transport layer
- 306:: Electron injection layer
- 400:: Negative electrode

### [Mode for Invention]

Hereinafter, the present specification will be described in more detail.

When one part "includes" one constituent element in the present specification, unless otherwise specifically described, this does not mean that another constituent element is excluded, but means that another constituent element may be further included.

In the present specification, 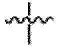 of a chemical formula means a position to which a constituent element is bonded.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, "substituted or unsubstituted" means being unsubstituted or substituted with one or more substituents selected from the group consisting of deuterium; a halogen group; -CN; a C1 to C60 alkyl group; a C2 to C60 alkenyl group; a C2 to C60 alkynyl group; a C1 to C60 haloalkyl group; a C1 to C60 alkoxy group; a C6 to C60 aryloxy group; a C1 to C60 alkylthioxy group; a C6 to C60 arylthioxy group; a C1 to C60 alkylsulfoxy group; a C6 to C60 arylsulfoxy group; a C3 to C60 cycloalkyl group; a C2 to C60 heterocycloalkyl group; a C6 to C60 aryl group; a C2 to C60 heteroaryl group; - SiRR'R"; -P(=O)RR'; and -NRR', or a substituent to which two or more substituents selected among the exemplified substituents are linked, and R, R' and R" are each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a chemical formula or compound", when the content of deuterium is 0%, the content of hydrogen is 100%, and all the substituents do not explicitly exclude deuterium such as hydrogen, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or ²H.

In an exemplary embodiment of the present application, the isotope means an atom with the same atomic number (Z), but different mass numbers (A), and the isotope may be interpreted as an element which has the same number of protons, but different number of neutrons.

In an exemplary embodiment of the present application, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 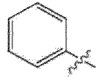 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formula.

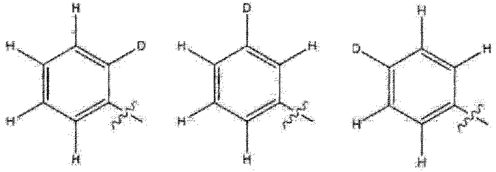

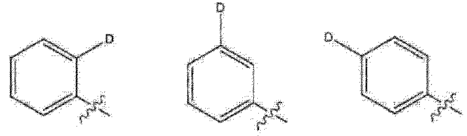

Further, in an exemplary embodiment of the present application, "a phenyl group having a deuterium content of 0%" may mean a phenyl group that does not include a deuterium atom, that is, has five hydrogen atoms.

In the present specification, the halogen may be fluorine, chlorine, bromine or iodine.

In the present specification, an alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, an alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, an alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, a haloalkyl group means an alkyl group substituted with a halogen group, and specific examples thereof include -CF₃, -CF₂CF₃, and the like, but are not limited thereto.

In the present specification, an alkoxy group is represented by -O(R101), and the above-described examples of the alkyl group may be applied to R101.

In the present specification, an aryloxy group is represented by -O(R102), and the above-described examples of the aryl group may be applied to R102.

In the present specification, an alkylthioxy group is represented by -S(R103), and the above-described examples of the alkyl group may be applied to R103.

In the present specification, an arylthioxy group is represented by -S(R104), and the above-described examples of the aryl group may be applied to R104.

In the present specification, an alkylsulfoxy group is represented by -S(=0)₂(R105), and the above-described examples of the alkyl group may be applied to R105.

In the present specification, an arylsulfoxy group is represented by -S(=0)₂(R106), and the above-described examples of the aryl group may be applied to R106.

In the present specification, a cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, a heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, an aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group includes a spiro group. The number of carbon atoms of the aryl group may be 6 to 60, specifically 6 to 40, and more specifically 6 to 25. Specific examples of the aryl group include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, the terphenyl group may be selected from the following structures.

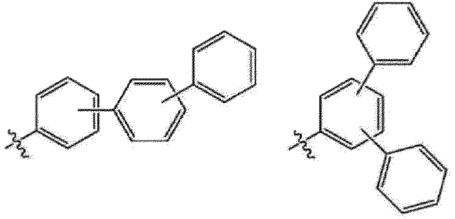

In the present specification, the fluorenyl group may be substituted, and adjacent substituents may be bonded to each other to form a ring.

When the fluorenyl group is substituted, the substituent may be 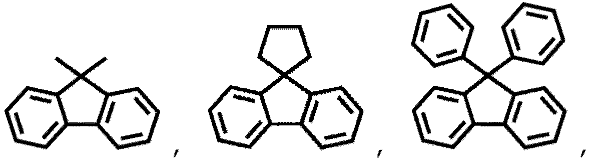 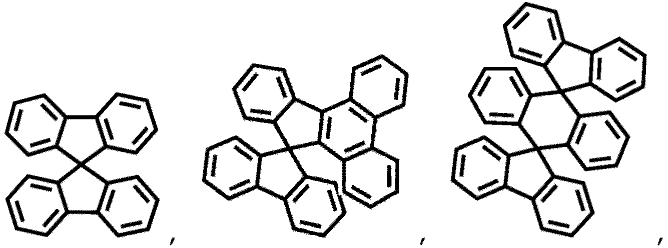 and the like, but is not limited thereto.

In the present specification, a heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridine group, a pyrrole group, a pyrimidine group, a pyridazine group, a furan group, a thiophene group, an imidazole group, a pyrazole group, an oxazole group, an isoxazole group, a thiazole group, an isothiazole group, a triazole group, a furazan group, an oxadiazole group, a thiadiazole group, a dithiazole group, a tetrazolyl group, a pyran group, a thiopyran group, a diazine group, an oxazine group, a thiazine group, a dioxin group, a triazine group, a tetrazine group, a quinoline group, an isoquinoline group, a quinazoline group, an isoquinazoline group, a quinozoline group, a naphthyridine group, an acridine group, a phenanthridine group, an imidazopyridine group, a diazanaphthalene group, a triazaindene group, an indole group, an indolizine group, a benzothiazole group, a benzoxazole group, a benzimidazole group, a benzothiophene group, a benzofuran group, a dibenzothiophene group, a dibenzofuran group, a carbazole group, a benzocarbazole group, a dibenzocarbazole group, a phenazine group, a dibenzosilole group, spirobi(dibenzosilole) group, a dihydrophenazine group, a phenoxazine group, a phenanthridine group, a thienyl group, an indolo[2,3-a]carbazole group, an indolo[2,3-b]carbazole group, an indoline group, a 10,11-dihydro-dibenzo[b,f]azepine group, a 9,10-dihydroacridine group, a phenanthrazine group, a phenothiathiazine group, a phthalazine group, a naphthobenzofuran group, a naphthobenzothiophene group, a benzo[c][1,2,5]thiadiazole group, a 2,3-dihydrobenzo[b]thiophene group, a 2,3-dihydrobenzofuran group, a 5,10-dihydrodibenzo[b,e][1,4]azasiline group, a pyrazolo[1,5-c]quinazoline group, a pyrido[1,2-b]indazole group, a pyrido[1,2-a]imidazo[1,2-e]indoline group, a 5,11-dihydroindeno[1,2-b]carbazole group, and the like, but are not limited thereto.

In the present specification, when the substituent is a carbazole group, it means being bonded to nitrogen or carbon of carbazole.

In the present specification, when a carbazole group is substituted, an additional substituent may be substituted with the nitrogen or carbon of the carbazole.

In the present specification, a benzocarbazole group may be any one of the following structures.

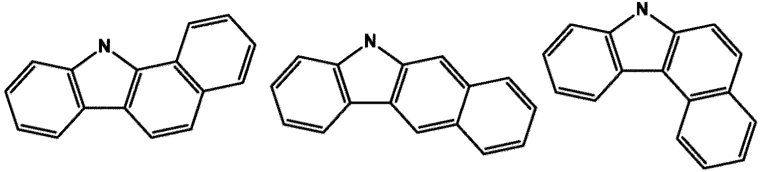

In the present specification, a dibenzocarbazole group may be any one of the following structures.

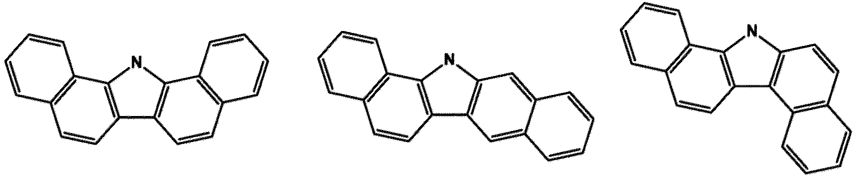

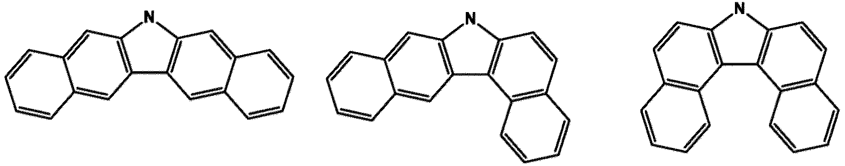

In the present specification, a naphthobenzofuran group may be any one of the following structures.

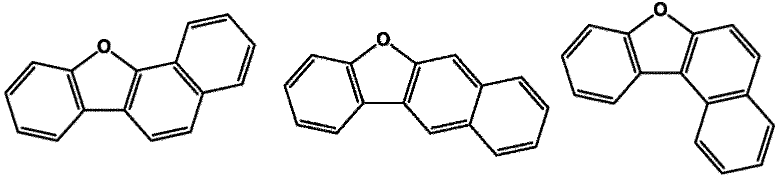

In the present specification, a naphthobenzothiophene group may be any one of the following structures.

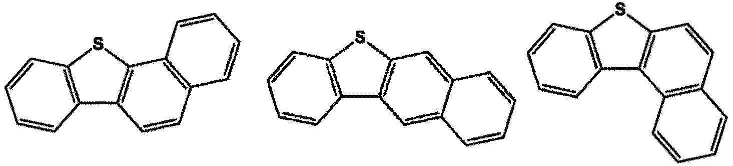

In the present specification, a silyl group includes Si and is a substituent to which the Si atom is directly linked as a radical, and is represented by - Si (R107) (R108) (R109), and R107 to R109 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. Specific examples of the silyl group include 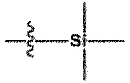 (a trimethyl silyl group), 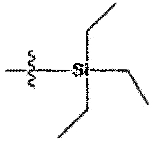 (a triethylsilyl group), 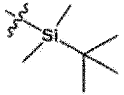 (a t-butyldimethylsilyl group), 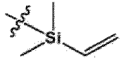 (a vinyldimethylsilyl group), 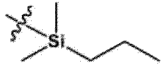 (a propyldimethylsilyl group), 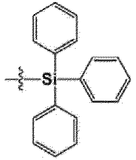 (a triphenylsilyl group), 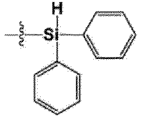 (a diphenylsilyl group), 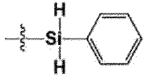 (a phenylsilyl group) and the like, but are not limited thereto.

In the present specification, a phosphine oxide group is represented by -P(=O)(R110)(R111), and R110 and R111 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. Specifically, the phosphine oxide group may be substituted with an alkyl group or an aryl group, and the above-described example may be applied to the alkyl group and the aryl group. Examples of the phosphine oxide group include a dimethylphosphine oxide group, a diphenylphosphine oxide group, dinaphthylphosphine oxide group, and the like, but are not limited thereto.

In the present specification, an amine group is represented by -N(R112) (R113), and R112 and R113 are the same as or different from each other, and are each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, the above-described examples of the aryl group may be applied to an arylene group except for a divalent arylene group.

In the present specification, the above-described examples of the heteroaryl group may be applied to a heteroarylene group except for a divalent heteroarylene group.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

Hydrocarbon rings and hetero rings that adjacent groups may form include an aliphatic hydrocarbon ring, an aromatic hydrocarbon ring, an aliphatic hetero ring and an aromatic hetero ring, and structures exemplified by the above-describe cycloalkyl group, aryl group, heterocycloalkyl group and heteroaryl group may be applied to the rings, except for those that are not monovalent groups.

In an exemplary embodiment of the present application, provided is the heterocyclic compound represented by Chemical Formula 1.

In an exemplary embodiment of the present application, X1 is N, and X2 and X3 may be CRa1.

In an exemplary embodiment of the present application, X3 is N, and X1 and X2 may be CRa1.

In an exemplary embodiment of the present application, X2 is N, and X1 and X3 may be CRa1.

In an exemplary embodiment of the present application, X1 and X2 are N, and X3 may be CRa1.

In an exemplary embodiment of the present application, X1 and X3 are N, and X2 may be CRa1.

In an exemplary embodiment of the present application, X2 and X3 are N, and X1 may be CRa1.

In an exemplary embodiment of the present application, X1 to X3 may be N.

In an exemplary embodiment of the present application, La1 and La2 are the same as or different from each other, and may be each independently a direct bond; a substituted or unsubstituted C6 to C60 arylene group; or a substituted or unsubstituted C2 to C60 heteroarylene group.

In another exemplary embodiment, La1 and La2 are the same as or different from each other, and may be each independently a direct bond; a substituted or unsubstituted C6 to C40 arylene group; or a substituted or unsubstituted C2 to C40 heteroarylene group.

In still another exemplary embodiment, La1 and La2 are the same as or different from each other, and may be each independently a direct bond; or a substituted or unsubstituted C6 to C40 arylene group.

In yet another exemplary embodiment, La1 and La2 are the same as or different from each other, and may be each independently a direct bond; or a substituted or unsubstituted C6 to C20 arylene group.

In yet another exemplary embodiment, La1 and La2 are the same as or different from each other, and may be each independently a direct bond; a substituted or unsubstituted monocyclic C6 to C10 arylene group; or a substituted or unsubstituted polycyclic C10 to C20 arylene group.

In yet another exemplary embodiment, La1 and La2 are the same as or different from each other, and may be each independently a direct bond; a monocyclic C6 to C10 arylene group; or a polycyclic C10 to C20 arylene group.

In yet another exemplary embodiment, La1 and La2 are the same as or different from each other, and may be each independently a direct bond; a substituted or unsubstituted phenylene group; or a substituted or unsubstituted biphenylene group.

In yet another exemplary embodiment, La1 and La2 are the same as or different from each other, and may be each independently a direct bond; a phenylene group; or a biphenylene group.

In yet another exemplary embodiment, La1 and La2 are the same as or different from each other, and may be each independently unsubstituted or substituted with deuterium.

In an exemplary embodiment of the present application, R1 may be hydrogen, and n may be an integer from 1 to 4.

In an exemplary embodiment of the present application, R1 may be hydrogen, and n may be an integer of 4.

In an exemplary embodiment of the present application, R1 may be deuterium, and n may be an integer of 1 to 4.

In an exemplary embodiment of the present application, R1 may be deuterium, and n may be an integer of 4.

In an exemplary embodiment of the present application, m may be 1.

In an exemplary embodiment of the present application, m may be 2.

In an exemplary embodiment of the present application, m may be 3.

In an exemplary embodiment of the present application, m may be 4.

In an exemplary embodiment of the present application, Ar1 and Ar2 are the same as or different from each other, and may be each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR'.

In another exemplary embodiment, Ar1 and Ar2 are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In still another exemplary embodiment, Ar1 and Ar2 are the same as or different from each other, and may be each independently a substituted or unsubstituted C1 to C40 alkyl group; a substituted or unsubstituted C6 to C40 aryl group; or a substituted or unsubstituted C2 to C40 heteroaryl group.

In yet another exemplary embodiment, Ar1 and Ar2 are the same as or different from each other, and may be each independently a substituted or unsubstituted C6 to C40 aryl group; or a substituted or unsubstituted C2 to C40 heteroaryl group.

In yet another exemplary embodiment, Ar1 and Ar2 are the same as or different from each other, and may be each independently a substituted or unsubstituted C6 to C20 aryl group; or a substituted or unsubstituted C2 to C20 heteroaryl group.

In yet another exemplary embodiment, Ar1 and Ar2 are the same as or different from each other, and may be each independently a C6 to C20 aryl group which is unsubstituted or substituted with a C1 to C10 alkyl group; or a C2 to C20 heteroaryl group.

In yet another exemplary embodiment, Ar1 and Ar2 are the same as or different from each other, and may be each independently a substituted or unsubstituted phenyl group; a substituted or unsubstituted biphenyl group; a substituted or unsubstituted terphenyl group; a substituted or unsubstituted dimethylfluorenyl group; a substituted or unsubstituted dibenzofuran group or a substituted or unsubstituted dibenzothiophene group.

In yet another exemplary embodiment, Ar1 and Ar2 are the same as or different from each other, and may be each independently a phenyl group; a biphenyl group; a terphenyl group; a dimethylfluorenyl group; a dibenzofuran group or a dibenzothiophene group.

In yet another exemplary embodiment, Ar1 and Ar2 may be unsubstituted or substituted with deuterium.

In an exemplary embodiment of the present application, 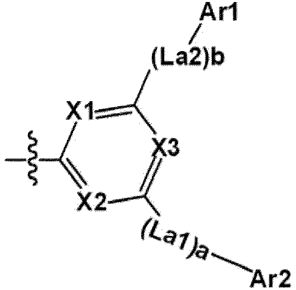 of Chemical Formula 1 may be represented by the following Chemical Formula 1-1 or 1-2.

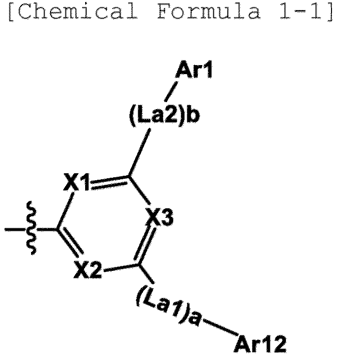

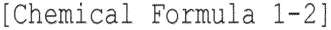

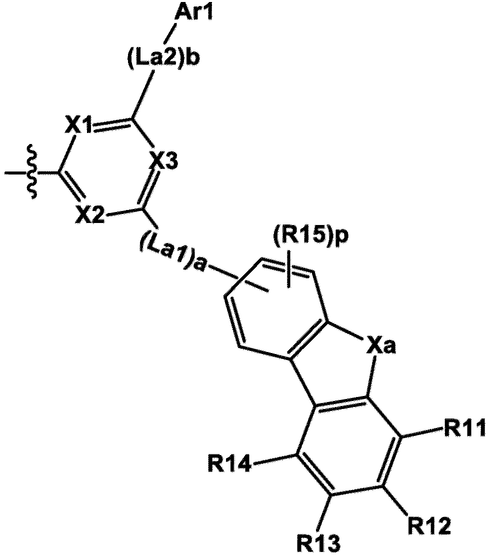

In Chemical Formulae 1-1 and 1-2,
the definitions of X1 to X3, La1, La2, a, b and Ar1 are the same as the definitions in Chemical Formula 1,
Xa is O; or S,
Ar12 is a substituted or unsubstituted C6 to C60 aryl group,
R11 to R15 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted aromatic hydrocarbon ring or a substituted or unsubstituted hetero ring, and
p is an integer from 0 to 3, and when p is 2 or higher, substituents in the parenthesis are the same as or different from each other.

In an exemplary embodiment of the present application, Ar12 is a substituted or unsubstituted C6 to C60 aryl group.

In an exemplary embodiment, Ar12 is a substituted or unsubstituted C6 to C40 aryl group.

In another exemplary embodiment, Ar12 is a substituted or unsubstituted C6 to C20 aryl group.

In still another exemplary embodiment, Ar12 is a C6 to C20 aryl group which is unsubstituted or substituted with a C1 to C10 alkyl group.

In yet another exemplary embodiment, Ar12 may be a substituted or unsubstituted phenyl group; a substituted or unsubstituted biphenyl group; a substituted or unsubstituted terphenyl group; or a substituted or unsubstituted dimethylfluorenyl group.

In yet another exemplary embodiment, Ar12 may be a phenyl group; a biphenyl group; a terphenyl group; or a dimethylfluorenyl group.

In an exemplary embodiment of the present application, Ar12 may be unsubstituted or substituted with deuterium.

In an exemplary embodiment of the present application, R11 to R15 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups may be bonded to each other to form a substituted or unsubstituted aromatic hydrocarbon ring or a substituted or unsubstituted hetero ring.

In another exemplary embodiment, R11 to R15 are the same as or different from each other, and may be each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group.

In still another exemplary embodiment, R11 to R15 are the same as or different from each other, and may be each independently selected from the group consisting of hydrogen; deuterium; a substituted or unsubstituted C1 to C40 alkyl group; a substituted or unsubstituted C6 to C40 aryl group; or a substituted or unsubstituted C2 to C40 heteroaryl group.

In yet another exemplary embodiment, R11 and R15 are the same as or different from each other, and may be each independently hydrogen; or deuterium.

In an exemplary embodiment of the present application, Chemical Formula 1 may be represented by any one of the following Chemical Formulae 3 to 5.

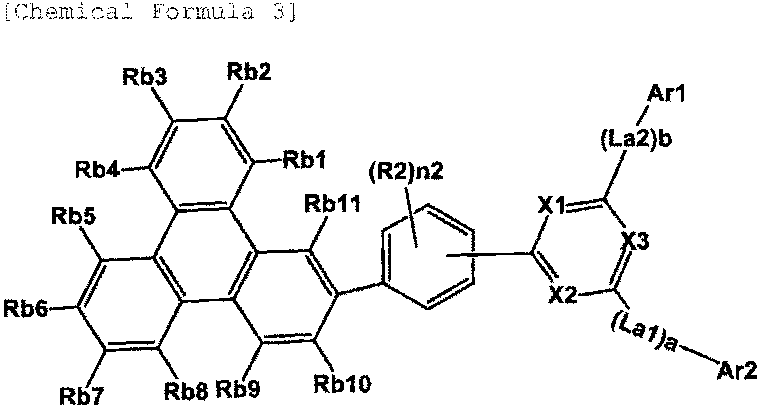

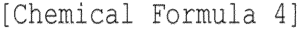

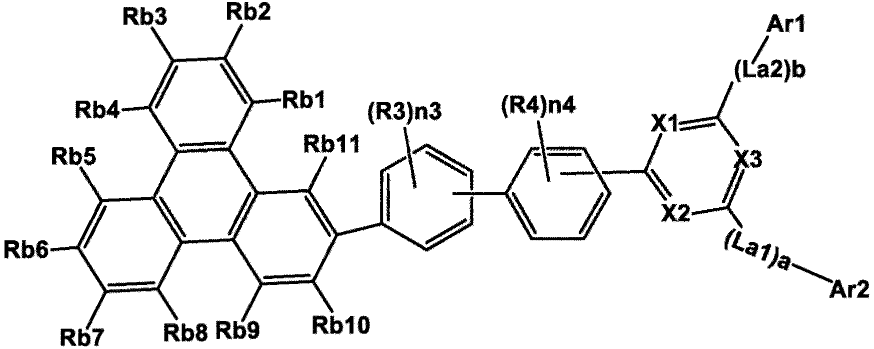

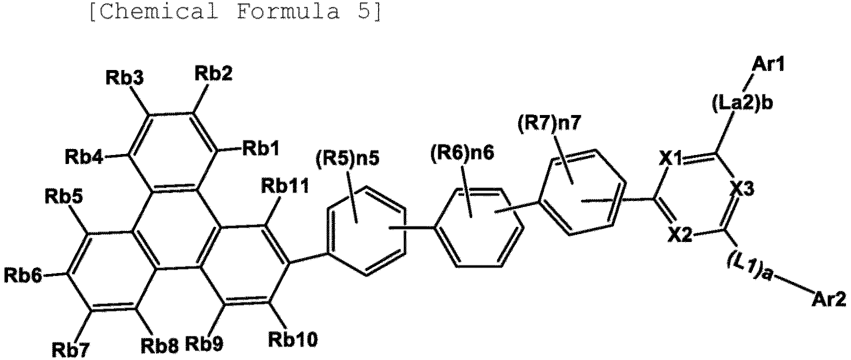

In Chemical Formulae 3 to 5,
the definitions of Rb1 to Rb11, X1 to X3, Ar1, La1, La2, a, b and Ar2 are the same as the definitions in Chemical Formula 1, and
R2 to R7 are the same as or different from each other, and are each independently hydrogen; or deuterium, n2 to n7 are the same as or different from each other, and are each independently an integer of 0 to 4, and when n2 to n7 are 2 or higher, substituents in the parenthesis are the same as or different from each other.

In an exemplary embodiment of the present application, Chemical Formula 3 may be represented by the following Chemical Formula 3-1 or 3-2.

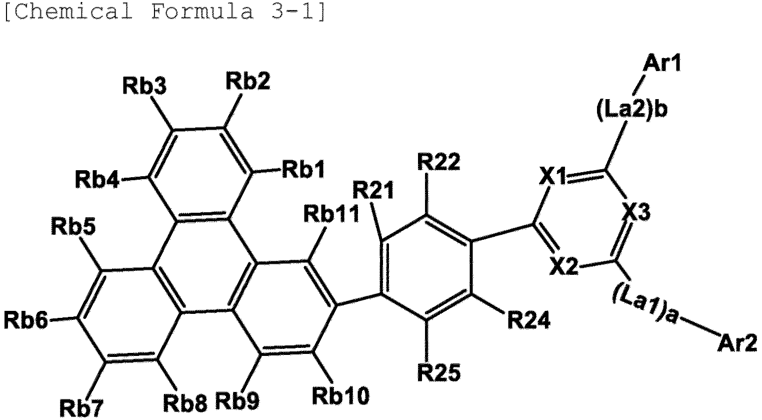

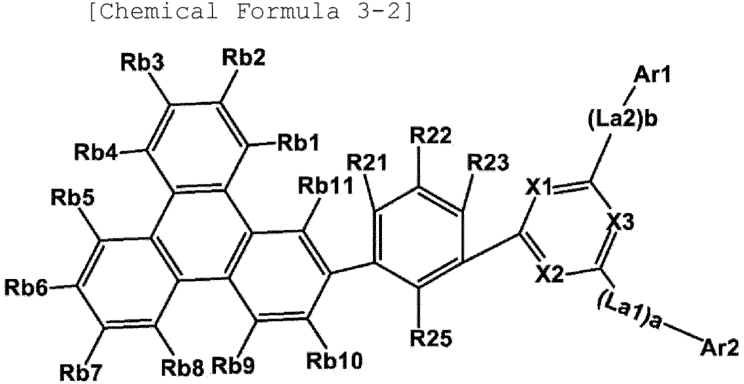

In Chemical Formulae 3-1 and 3-2,
R21 to R25 are the same as or different from each other, and are each independently hydrogen; or deuterium, and
the definitions of the other substituents are the same as the definitions in Chemical Formula 3.

In an exemplary embodiment of the present application, Chemical Formula 4 may be represented by any one of the following Chemical Formulae 4-1 to 4-4.

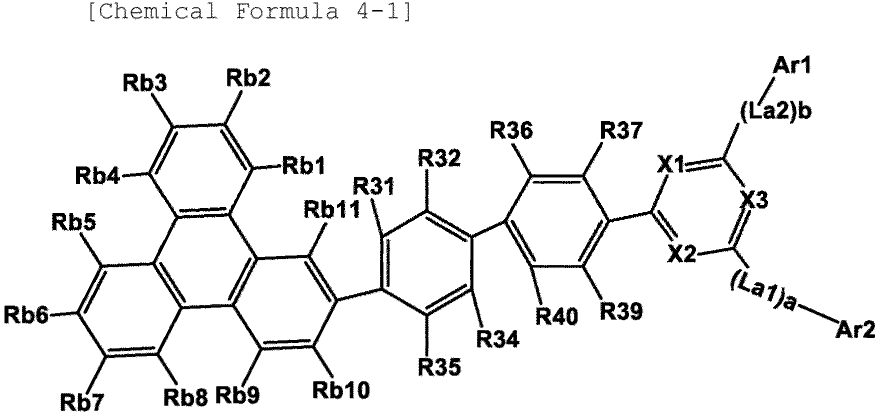

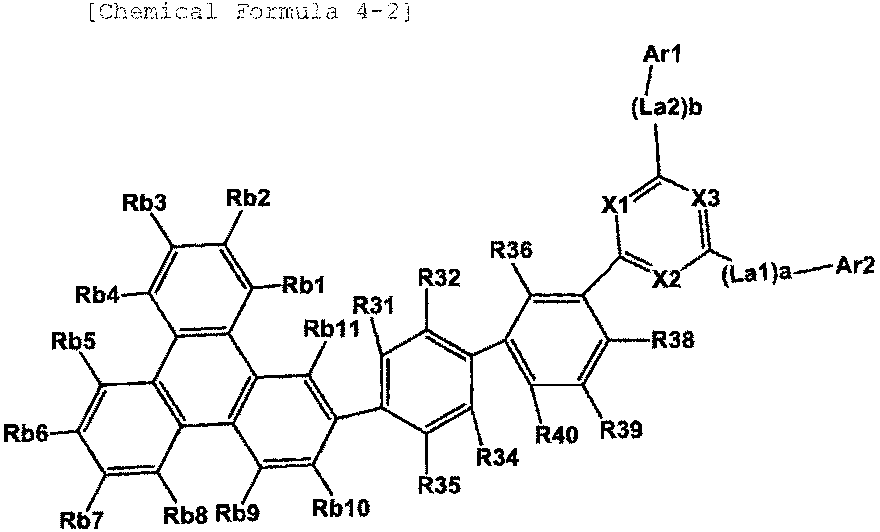

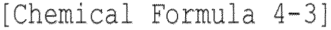

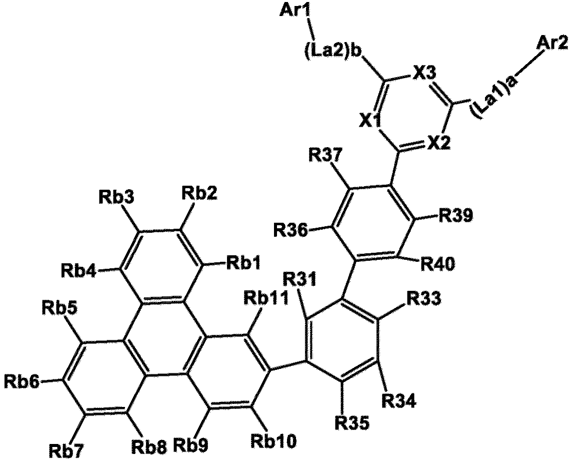

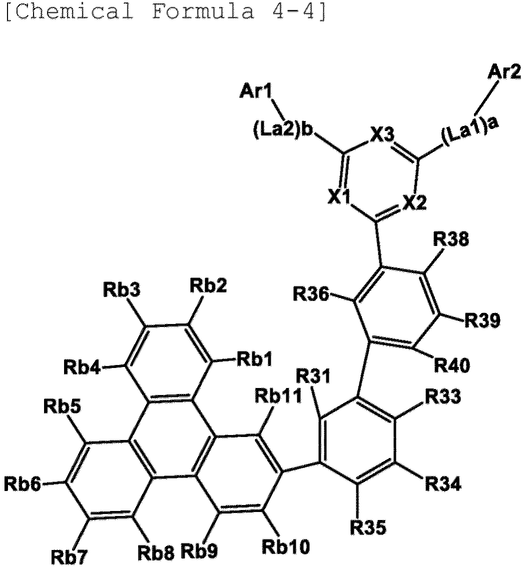

In Chemical Formulae 4-1 to 4-4,
R31 to R40 are the same as or different from each other, and are each independently hydrogen; or deuterium, and
the definitions of the other substituents are the same as the definitions in Chemical Formula 4.

In an exemplary embodiment of the present application, Chemical Formula 5 may be represented by any one of the following Chemical Formulae 5-1 to 5-4.

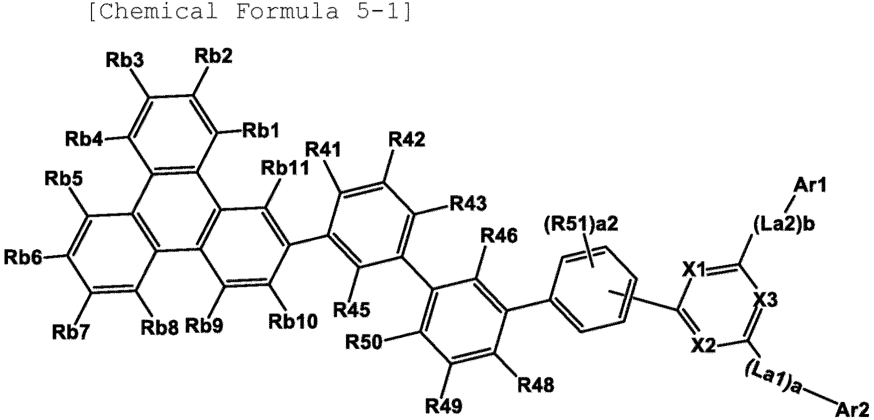

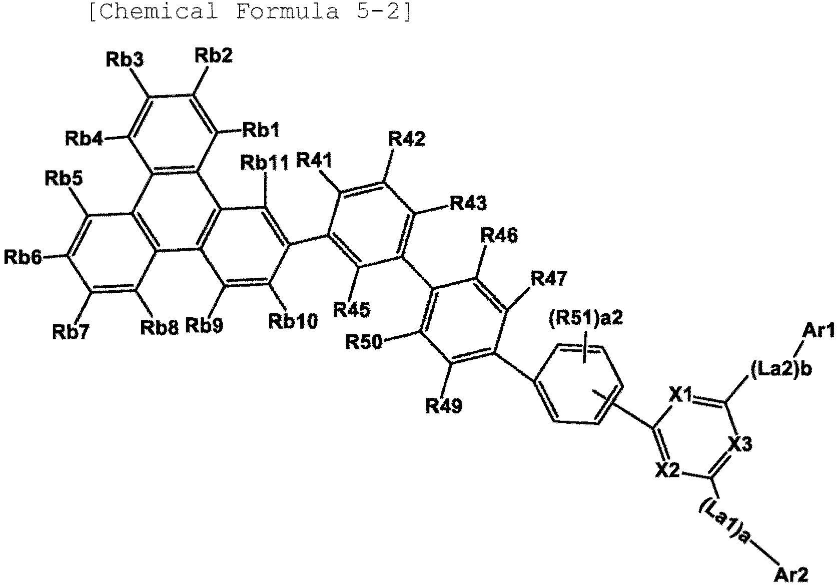

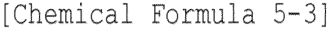

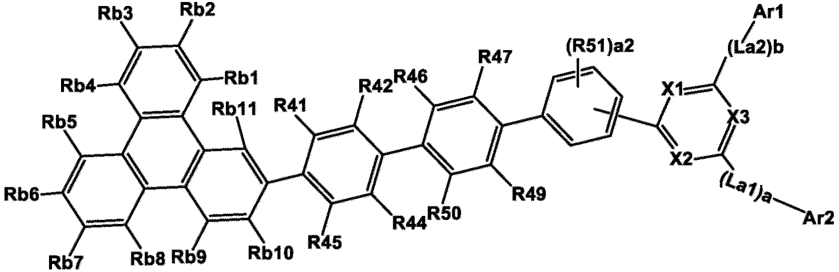

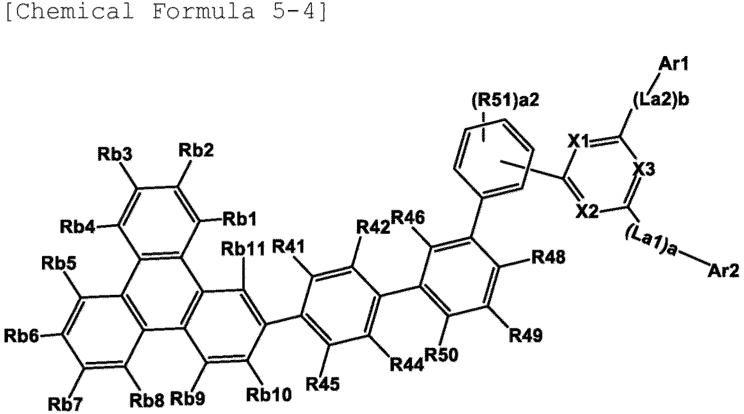

In Chemical Formulae 5-1 to 5-4,
R41 to R51 are the same as or different from each other, and are each independently hydrogen; or deuterium, a2 is an integer from 0 to 4, and when a2 is 2 or higher, substituents in the parenthesis are the same as or different from each other, and
the definitions of the other substituents are the same as the definitions in Chemical Formula 5.

In an exemplary embodiment of the present application, the deuterium content of Chemical Formula 1 may be 40% or more and 100% or less.

In an exemplary embodiment of the present application, the deuterium content of Chemical Formula 1 may be 40% or more and 100% or less, 45% or more and 100% or less, 50% or more and 100% or less, and may specifically satisfy the range of 70% or more and 90% or less.

In an exemplary embodiment of the present application, Rb1 to Rb11 are the same as or different from each other, and are each independently hydrogen; or deuterium, and at least of Rb1 to Rb11 may be deuterium.

In another exemplary embodiment, Rb1 to Rb11 are the same as or different from each other, and may be each independently deuterium.

In still another exemplary embodiment, Rb1 to Rb11 are the same as or different from each other, and are each independently hydrogen; or deuterium, and 1 or more and 10 or less of Rb1 to Rb11 may be deuterium.

The number of deuteriums in Rb1 to Rb11 is not limited as long as it satisfies the range of 1 or more and 11 or less, and may satisfy, for example, the range of 3 or more and 10 or less, and 4 or more and 9 or less.

In an exemplary embodiment of the present application, Chemical Formula 1 is represented by the following Structural Formulae A to C, the deuterium content of the following Structural Formula A is 1% to 100%, the deuterium content of the following Structural Formula B is 0% to 100%, and the deuterium content of the following Structural Formula C may be 0% to 100%.

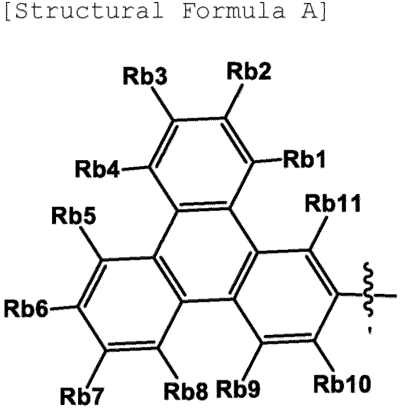

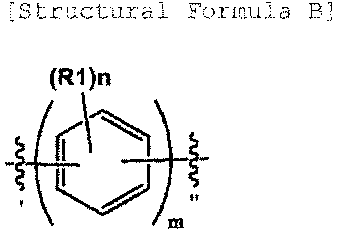

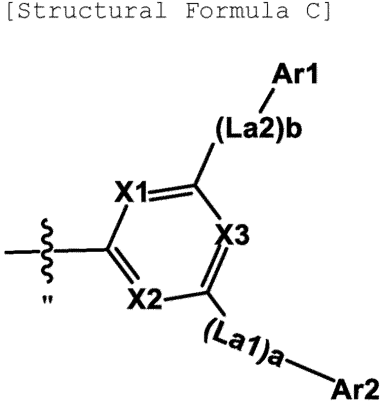

In Structural Formulae A to C,
the definition of each substituent is the same as the definition in Chemical Formula 1, and
the 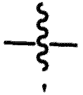 and 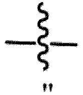 mean a position where moieties are connected to each other, and the same symbols are connected to each other.

In an exemplary embodiment of the present application, the deuterium content of Structural Formula A may be 1% to 100%; 50% to 100%; 60% to 90%; or 70% to 90%.

In an exemplary embodiment of the present application, the deuterium content of Structural Formula A may be 100%. In this case, the fact that the deuterium content of Structural Formula A is 100% may mean that all of the Rb1 to Rb11 are deuterium.

In an exemplary embodiment of the present application, the deuterium content of Structural Formula B may be 0% to 100%; 20% to 100%; 30% to 100%; 50% to 100%; or 70% to 90%.

In an exemplary embodiment of the present application, the deuterium content of Structural Formula B may be 0%.

In an exemplary embodiment of the present application, the deuterium content of Structural Formula B may be 100%.

In an exemplary embodiment of the present application, the deuterium content of Structural Formula C may be 0% to 100%; 20% to 100%; 30% to 100%; 50% to 100%; or 70% to 90%.

In an exemplary embodiment of the present application, the deuterium content of Structural Formula C may be 0%.

In an exemplary embodiment of the present application, the deuterium content of Structural Formula C may be 100%.

In another exemplary embodiment, the deuterium contents of Structural Formulae A, B and C may be increased or decreased according to the deuterium substitution process when an additional substituent is further included.

In an exemplary embodiment of the present application, the deuterium content of Structural Formulae A to C may satisfy a range of 1% or more and 100% or less, 45% or more and 100% or less, 50% or more and 100% or less, 60% or more and 90% or less; or 70% or more and 90% or less.

Specifically, although not all deuterium contents are enumerated in the present specification, the deuterium contents of Structural Formulae A to C may be specifically shown by each calculating the deuterium contents of Specific Example Compounds 1-1 to 1-200 of Chemical Formula 1 to be described below, and the deuterium content part may be expressed to the second decimal place.

In an exemplary embodiment of the present application, R, R', and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another exemplary embodiment, R, R', and R" are the same as or different from each other, and may be each independently a substituted or unsubstituted C1 to C60 alkyl group; or a substituted or unsubstituted C6 to C60 aryl group.

In still another exemplary embodiment, R, R', and R" are the same as or different from each other, and may be each independently a C1 to C60 alkyl group; or a C6 to C60 aryl group.

In yet another exemplary embodiment, R, R', and R" are the same as or different from each other, and may be each independently a methyl group; or a phenyl group.

In yet another exemplary embodiment, R, R', and R" may be a substituted or unsubstituted methyl group.

In yet another exemplary embodiment, R, R', and R" may be a substituted or unsubstituted phenyl group.

In yet another exemplary embodiment, R, R', and R" may be a phenyl group.

In yet another exemplary embodiment, R, R', and R" may be a methyl group.

In an exemplary embodiment of the present application, provided is a heterocyclic compound in which Chemical Formula 1 is represented by any one of the following compounds. Further, in an exemplary embodiment of the present application, the following compound is just one example and is not limited thereto, and may include other compounds included in Chemical Formula 1 which includes an additional substituent. That is, regarding the substitution position of deuterium in the following compound, specific positions are excluded during the process of deuterium substitution and synthesis as long as only the above-described content of deuterium is satisfied, and hydrogen and deuterium may be present in a mixed state.

| | | | |
|---|---|---|---|
| 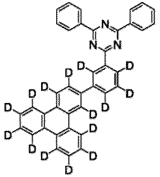 | 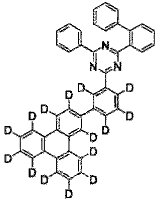 | 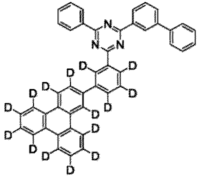 | 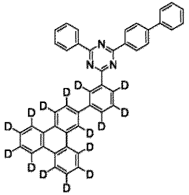 |
| 1-1 | 1-2 | 1-3 | 1-4 |
| 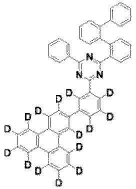 | 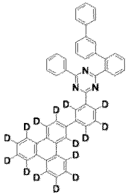 | 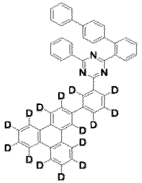 | 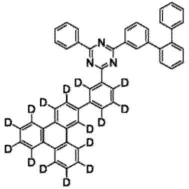 |
| 1-5 | 1-6 | 1-7 | 1-8 |
| 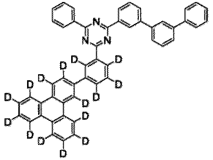 | 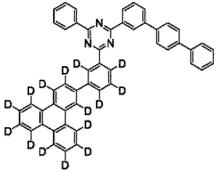 | 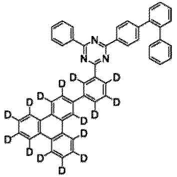 | 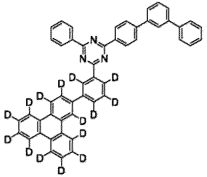 |
| 1-9 | 1-10 | 1-11 | 1-12 |
| 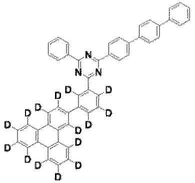 | 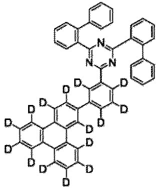 | 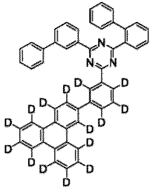 | 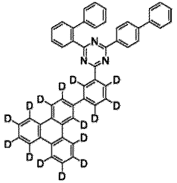 |
| 1-13 | 1-14 | 1-15 | 1-16 |
| 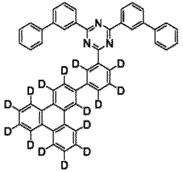 | 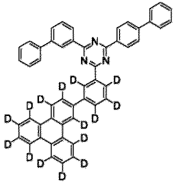 | 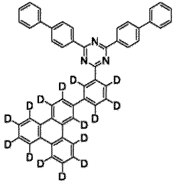 | 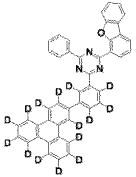 |
| 1-17 | 1-18 | 1-19 | 1-20 |
| 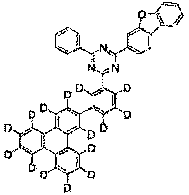 | 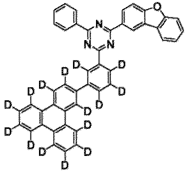 | 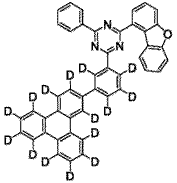 | 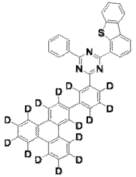 |
| 1-21 | 1-22 | 1-23 | 1-24 |
| 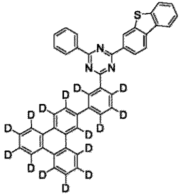 | 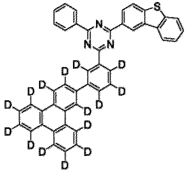 | 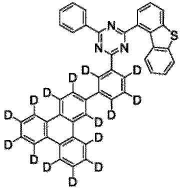 | 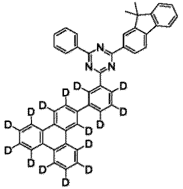 |
| 1-25 | 1-26 | 1-27 | 1-28 |
| 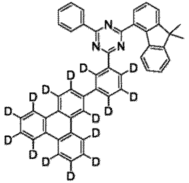 | 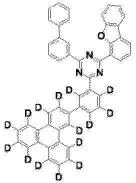 | 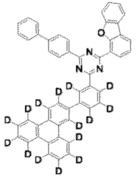 | 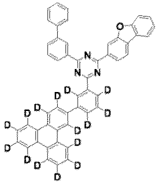 |
| 1-29 | 1-30 | 1-31 | 1-32 |
| 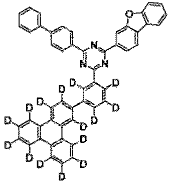 | 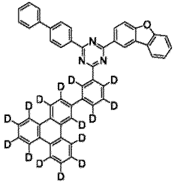 | 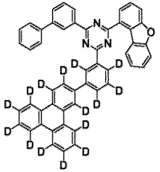 | 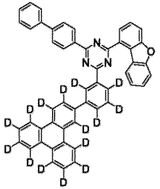 |
| 1-33 | 1-34 | 1-35 | 1-36 |
| 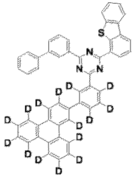 | 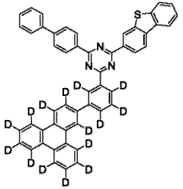 | 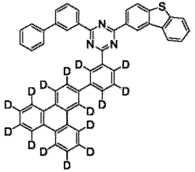 | 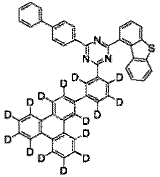 |
| 1-37 | 1-38 | 1-39 | 1-40 |
| 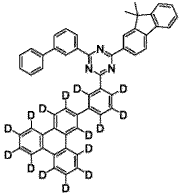 | 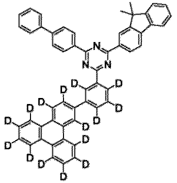 | 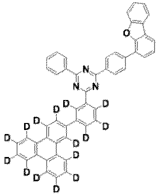 | 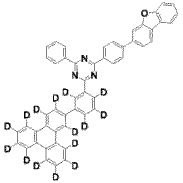 |
| 1-41 | 1-42 | 1-43 | 1-44 |
| 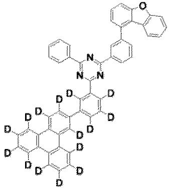 | 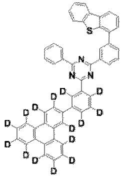 | 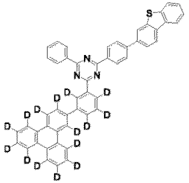 | 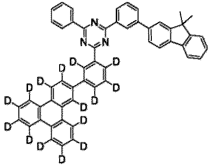 |
| 1-45 | 1-46 | 1-47 | 1-48 |
| 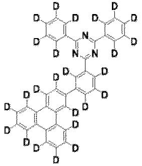 | 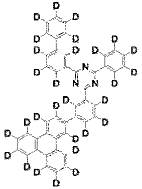 | 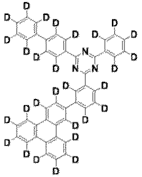 | 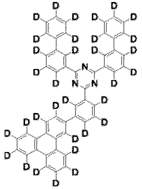 |
| 1-49 | 1-50 | 1-51 | 1-52 |
| 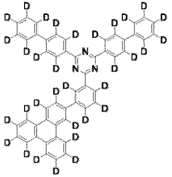 | 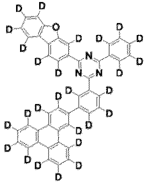 | 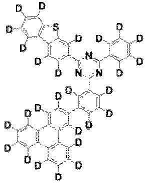 | 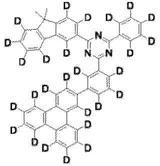 |
| 1-53 | 1-54 | 1-55 | 1-56 |
| 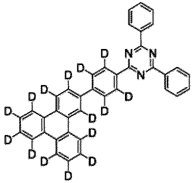 | 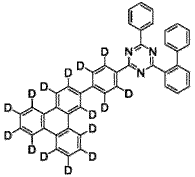 | 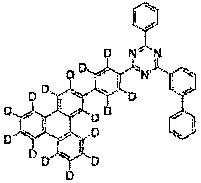 | 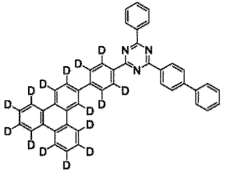 |
| 1-57 | 1-58 | 1-59 | 1-60 |
| 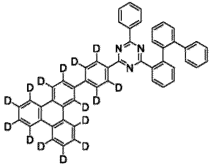 | 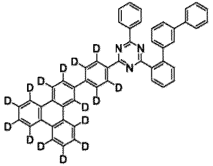 | 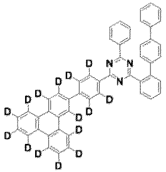 | 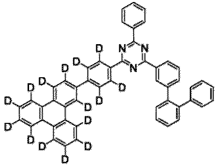 |
| 1-61 | 1-62 | 1-63 | 1-64 |
| 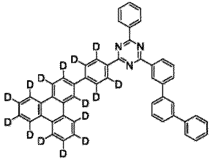 | 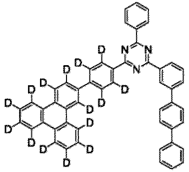 | 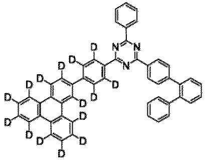 | 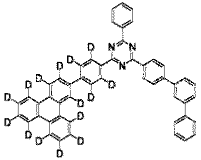 |
| 1-65 | 1-66 | 1-67 | 1-68 |
| 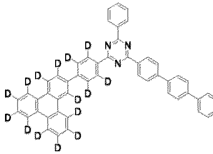 | 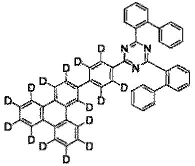 | 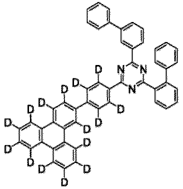 | 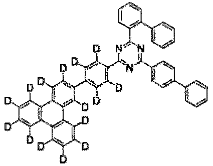 |
| 1-69 | 1-70 | 1-71 | 1-72 |
| 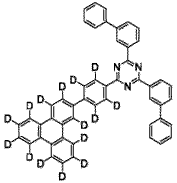 | 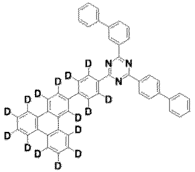 | 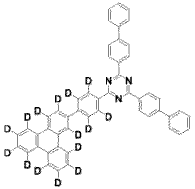 | 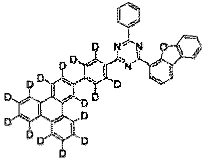 |
| 1-73 | 1-74 | 1-75 | 1-76 |
| 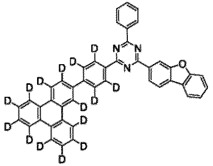 | 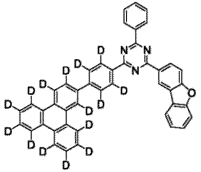 | 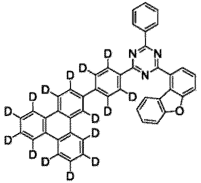 | 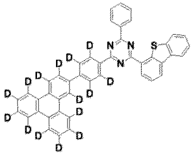 |
| 1-77 | 1-78 | 1-79 | 1-80 |
| 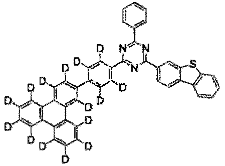 | 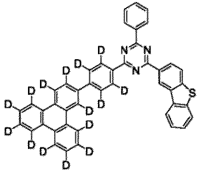 | 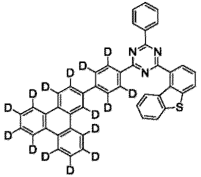 | 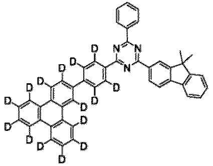 |
| 1-81 | 1-82 | 1-83 | 1-84 |
| 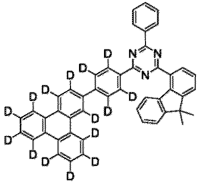 | 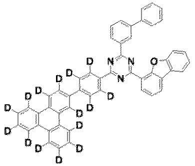 | 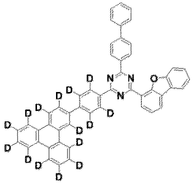 | 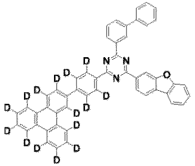 |
| 1-85 | 1-86 | 1-87 | 1-88 |
| 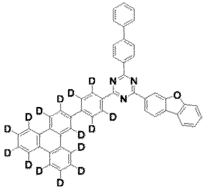 | 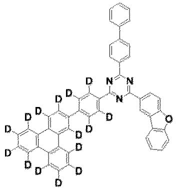 | 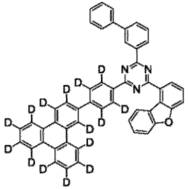 | 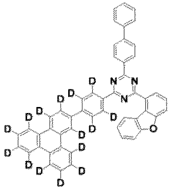 |
| 1-89 | 1-90 | 1-91 | 1-92 |
| 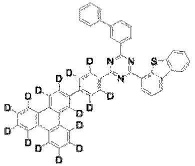 | 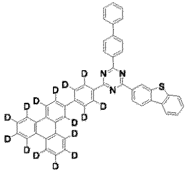 | 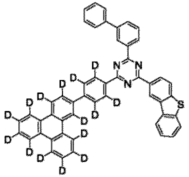 | 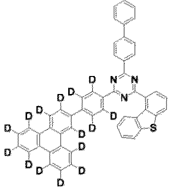 |
| 1-93 | 1-94 | 1-95 | 1-96 |
| 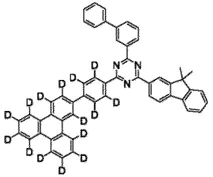 | 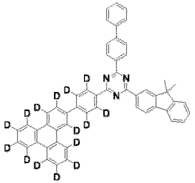 | 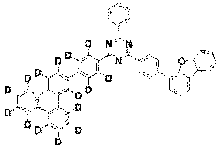 | 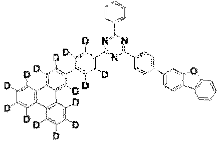 |
| 1-97 | 1-98 | 1-99 | 1-100 |
| 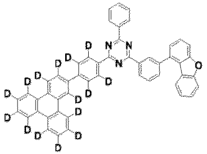 | 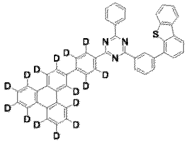 | 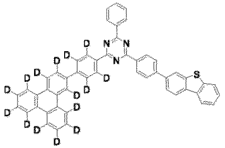 | 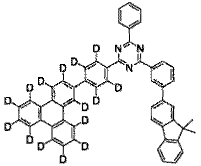 |
| 1-101 | 1-102 | 1-103 | 1-104 |
| 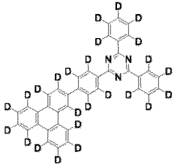 | 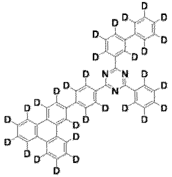 | 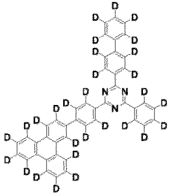 | 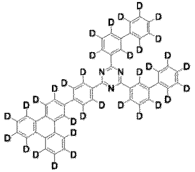 |
| 1-105 | 1-106 | 1-107 | 1-108 |
| 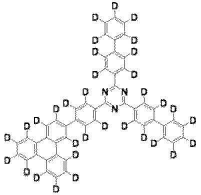 | 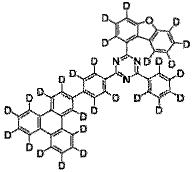 | 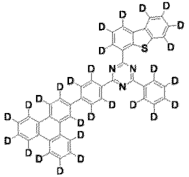 | 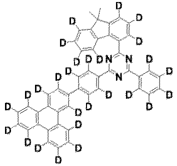 |
| 1-109 | 1-110 | 1-111 | 1-112 |
| 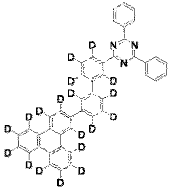 | 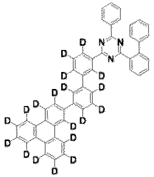 | 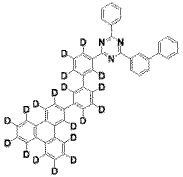 | 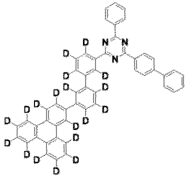 |
| 1-113 | 1-114 | 1-115 | 1-116 |
| 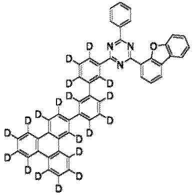 | 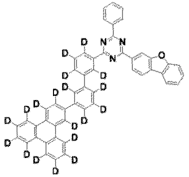 | 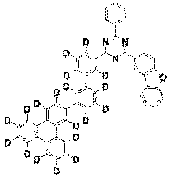 | 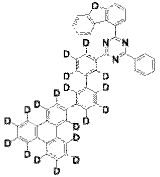 |
| 1-117 | 1-118 | 1-119 | 1-120 |
| 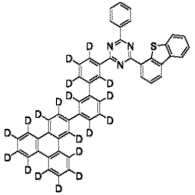 | 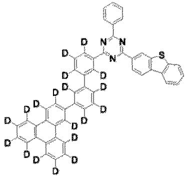 | 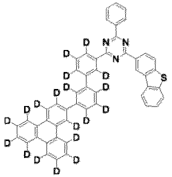 | 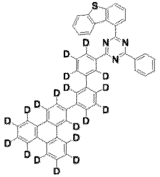 |
| 1-121 | 1-122 | 1-123 | 1-124 |
| 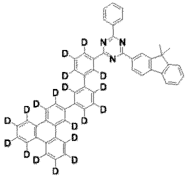 | 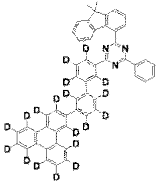 | 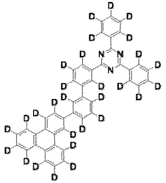 | 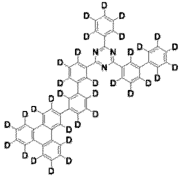 |
| 1-125 | 1-126 | 1-127 | 1-128 |
| 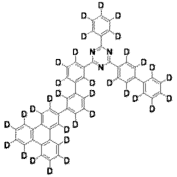 | 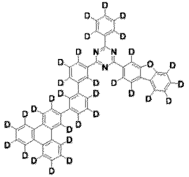 | 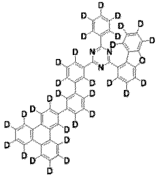 | 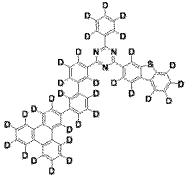 |
| 1-129 | 1-130 | 1-131 | 1-132 |
| 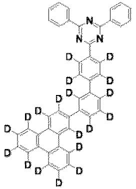 | 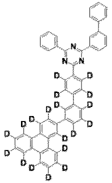 | 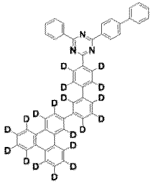 | 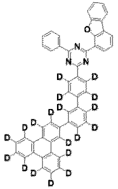 |
| 1-133 | 1-134 | 1-135 | 1-136 |
| 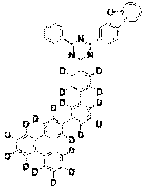 | 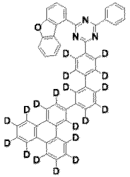 | 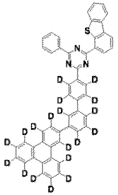 | 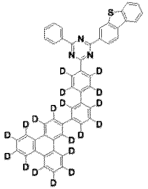 |
| 1-137 | 1-138 | 1-139 | 1-140 |
| 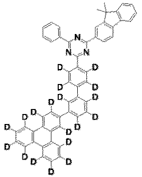 | 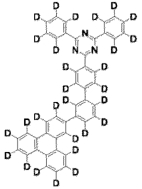 | 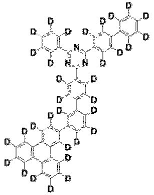 | 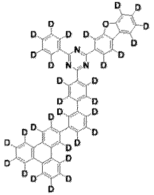 |
| 1-141 | 1-142 | 1-143 | 1-144 |
| 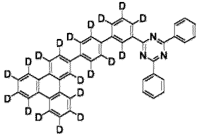 | 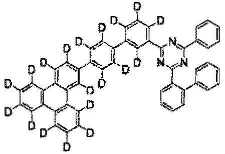 | 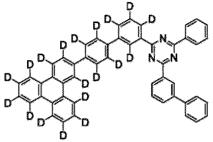 | 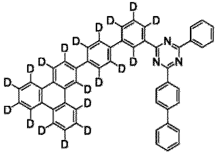 |
| 1-145 | 1-146 | 1-147 | 1-148 |
| 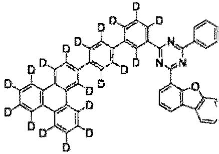 | 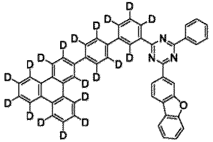 | 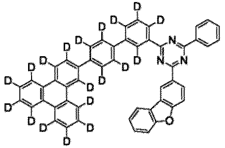 | 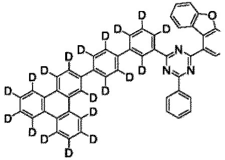 1 |
| 1-149 | 1-150 | 1-151 | 1-152 |
| 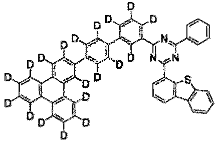 | 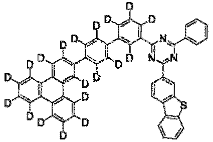 | 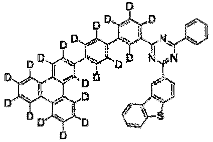 | 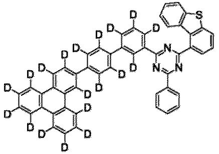 |
| 1-153 | 1-154 | 1-155 | 1-156 |
| 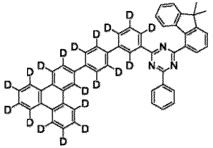 | 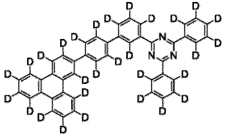 | 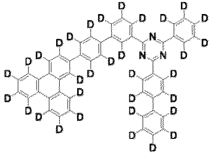 | 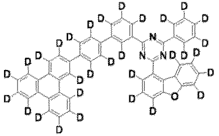 |
| 1-157 | 1-158 | 1-159 | 1-160 |
| 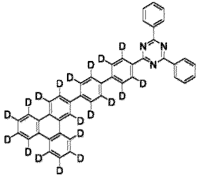 | 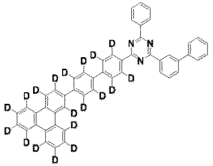 | 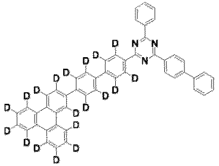 | 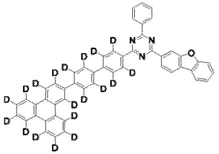 |
| 1-161 | 1-162 | 1-163 | 1-164 |
| 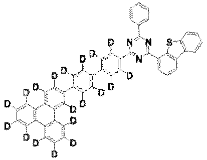 | 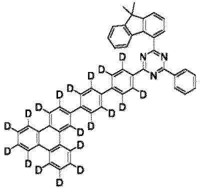 | 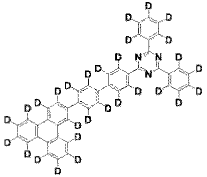 | 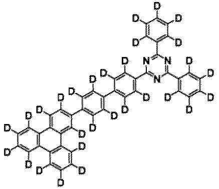 |
| 1-165 | 1-166 | 1-167 | 1-168 |
| 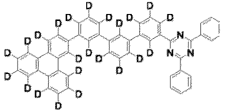 | 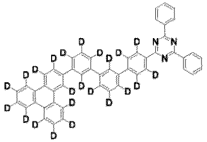 | 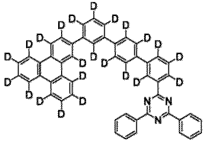 | 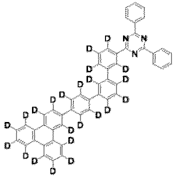 |
| 1-169 | 1-170 | 1-171 | 1-172 |
| 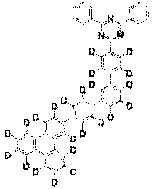 | 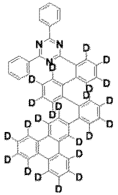 | 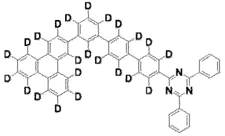 | 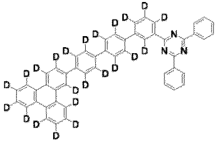 |
| 1-173 | 1-174 | 1-175 | 1-176 |
| 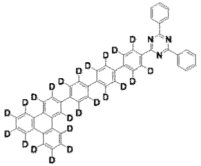 | 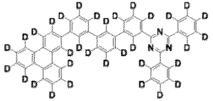 | 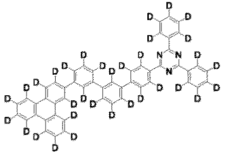 | 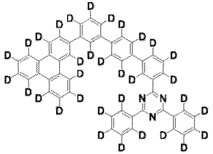 |
| 1-177 | 1-178 | 1-179 | 1-180 |
| 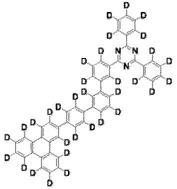 | 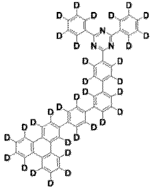 | 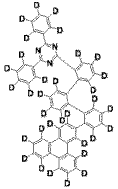 | 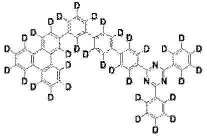 |
| 1-181 | 1-182 | 1-183 | 1-184 |
| 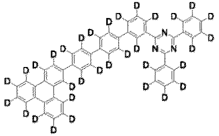 | 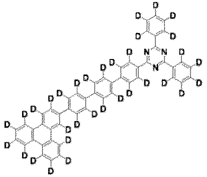 | 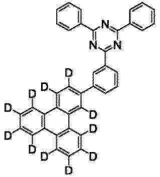 | 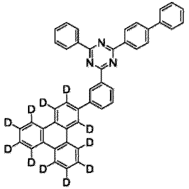 |
| 1-185 | 1-186 | 1-187 | 1-188 |
| 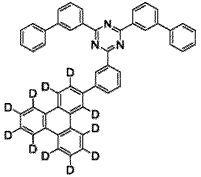 | 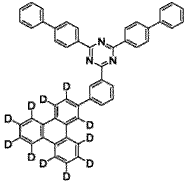 | 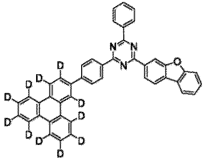 | 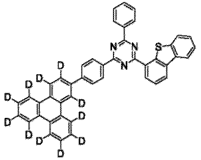 |
| 1-189 | 1-190 | 1-191 | 1-192 |
| 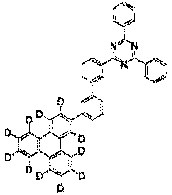 | 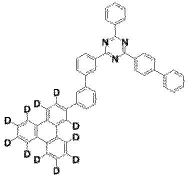 | 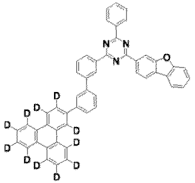 | 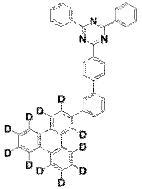 |
| 1-193 | 1-194 | 1-195 | 1-196 |
| 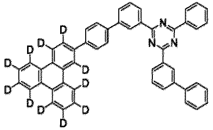 | 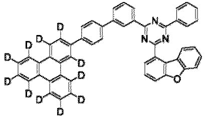 | 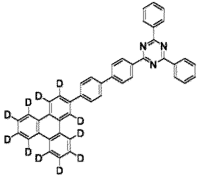 | 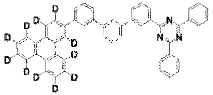 |
| 1-197 | 1-198 | 1-199 | 1-200 |

Further, various substituents may be introduced into the structure of Chemical Formula 1 to synthesize a compound having inherent characteristics of a substituent introduced. For example, it is possible to synthesize a material which satisfies conditions required for each organic material layer by introducing a substituent usually used for a hole injection layer material, a material for transporting holes, a light emitting layer material, an electron transport layer material, and a charge generation layer material, which are used for preparing an organic light emitting device, into the core structure.

In addition, it is possible to finely adjust an energy band-gap by introducing various substituents into the structure of Chemical Formula 1, and meanwhile, it is possible to improve characteristics at the interface between organic materials and diversify the use of the material.

Meanwhile, the compound has a high glass transition temperature (Tg) and thus has excellent thermal stability. The increase in thermal stability becomes an important factor for providing a device with driving stability.

Further, in an exemplary embodiment of the present application, provided is an organic light emitting device including: a first electrode; a second electrode provided to face the first electrode; and an organic material layer having one or more layers provided between the first electrode and the second electrode, in which one or more layers of the organic material layer include the heterocyclic compound represented by Chemical Formula 1.

In an exemplary embodiment of the present application, the first electrode may be a positive electrode, and the second electrode may be a negative electrode.

In another exemplary embodiment, the first electrode may be a negative electrode, and the second electrode may be a positive electrode.

In an exemplary embodiment of the present application, the organic light emitting device may be a blue organic light emitting device, and the heterocyclic compound according to Chemical Formula 1 may be used as a material for the blue organic light emitting device.

In an exemplary embodiment of the present application, the organic light emitting device may be a green organic light emitting device, and the heterocyclic compound represented by Chemical Formula 1 may be used as a material for the green organic light emitting device.

In an exemplary embodiment of the present application, the organic light emitting device may be a red organic light emitting device, and the heterocyclic compound represented by Chemical Formula 1 may be used as a material for the red organic light emitting device.

In an exemplary embodiment of the present application, the organic light emitting device may be a blue organic light emitting device, and the heterocyclic compound according to Chemical Formula 1 may be used as a material for a light emitting layer of the blue organic light emitting device.

In an exemplary embodiment of the present application, the organic light emitting device may be a green organic light emitting device, and the heterocyclic compound represented by Chemical Formula 1 may be used as a material for a light emitting layer of the green organic light emitting device.

In an exemplary embodiment of the present application, the organic light emitting device may be a red organic light emitting device, and the heterocyclic compound represented by Chemical Formula 1 may be used as a material for a light emitting layer of the red organic light emitting device.

The specific content on the heterocyclic compound represented by Chemical Formula 1 is the same as that described above.

The organic light emitting device of the present invention may be manufactured using typical manufacturing methods and materials of an organic light emitting device, except that the above-described heterocyclic compound is used to form an organic material layer having one or more layers.

The heterocyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multilayered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic material layers.

In the organic light emitting device of the present invention, the organic material layer includes a light emitting layer, and the light emitting layer may include the heterocyclic compound of Chemical Formula 1.

In the organic light emitting device of the present invention, provided is an organic light emitting device, in which the organic material layer includes a light emitting layer, the light emitting layer includes a host material, and the host material includes the heterocyclic compound.

In the organic light emitting device of the present invention, the organic material layer includes a light emitting layer, and the light emitting layer may include the heterocyclic compound of Chemical Formula 1 as a light emitting layer host.

In the organic light emitting device according to an exemplary embodiment of the present application, provided is an organic light emitting device in which the organic material layer including the heterocyclic compound represented by Chemical Formula 1 further includes a heterocyclic compound represented by the following Chemical Formula 2.

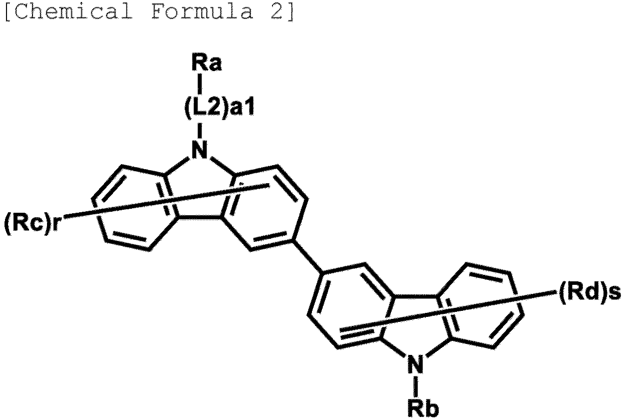

In Chemical Formula 2,
Rc and Rd are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P(=O)RR'; and -NRR', or two or more adjacent groups are bonded to each other to form a substituted or unsubstituted aromatic hydrocarbon ring or a substituted or unsubstituted hetero ring,
L2 is a direct bond; a substituted or unsubstituted C6 to C60 arylene group; or a substituted or unsubstituted C2 to C60 heteroarylene group,
Ra and Rb are the same as or different from each other, and are each independently -CN; -SiRR'R"; -P(=O)RR'; a substituted or unsubstituted C6 to C60 aryl group; or a substituted or unsubstituted C2 to C60 heteroaryl group,
R, R' and R" are the same as or different from each other, and are each independently hydrogen; deuterium; a substituted or unsubstituted C1 to C60 alkyl group; or a substituted or unsubstituted C6 to C60 aryl group,
a1 is an integer from 0 to 4,
r and s are an integer from 0 to 7, and
when a1, s and r are 2 or higher, substituents in the parenthesis are the same as or different from each other.

In an exemplary embodiment of the present application, L2 may be a direct bond; a substituted or unsubstituted C6 to C60 arylene group; or a substituted or unsubstituted C2 to C60 heteroarylene group.

In another exemplary embodiment, L2 may be a direct bond; a substituted or unsubstituted C6 to C40 arylene group; or a substituted or unsubstituted C2 to C40 heteroarylene group. In still another exemplary embodiment, L2 may be a direct bond; a C6 to C40 arylene group; or a C2 to C40 heteroarylene group.

In yet another exemplary embodiment, L2 may be a direct bond; a substituted or unsubstituted phenylene group; a substituted or unsubstituted biphenylene group; or a substituted or unsubstituted divalent dibenzofuran group. In yet another exemplary embodiment, L2 may be a direct bond; a phenylene group; a biphenylene group; a divalent dibenzothiophene group; a divalent dimethylfluorene group; or a divalent dibenzofuran group.

In an exemplary embodiment of the present application, L2 may be substituted with deuterium.

In an exemplary embodiment of the present application, Ra and Rb are the same as or different from each other, and may be each independently -CN; -SiRR'R"; -P(=O)RR'; a substituted or unsubstituted C6 to C60 aryl group; or a substituted or unsubstituted C2 to C60 heteroaryl group.

In another exemplary embodiment, Ra may be -CN; -SiRR'R"; - P(=O)RR'; a substituted or unsubstituted C6 to C60 aryl group; or a substituted or unsubstituted C2 to C60 heteroaryl group.

In still another exemplary embodiment, Ra may be -CN; - SiRR'R"; -P(=O)RR'; a C6 to C40 aryl group which is unsubstituted or substituted with a C1 to C40 alkyl group or a C6 to C40 aryl group; or a C2 to C60 heteroaryl group which is unsubstituted or substituted with a C6 to C40 aryl group or a C2 to C40 heteroaryl group.

In yet another exemplary embodiment, Ra may be -CN; - SiRR'R"; -P(=O)RR'; a phenyl group; a biphenyl group; a terphenyl group; a dimethylfluorenyl group; a diphenylfluorenyl group; a spirobifluorenyl group; a dibenzothiophene group which is unsubstituted or substituted with a phenyl group or a dibenzofuran group; or a dibenzofuran group which is unsubstituted or substituted with a phenyl group or a dibenzofuran group.

In an exemplary embodiment of the present application, Rb may be a substituted or unsubstituted C6 to C60 aryl group; or a substituted or unsubstituted C2 to C60 heteroaryl group.

In another exemplary embodiment, Rb may be a C6 to C60 aryl group which is unsubstituted or substituted with a C1 to C40 alkyl group, -CN, SiRR'R" or a C6 to C40 aryl group.

In still another exemplary embodiment, Rb may be a C6 to C40 aryl group which is unsubstituted or substituted with a C1 to C40 alkyl group, -CN, SiRR'R" or a C6 to C40 aryl group.

In yet another exemplary embodiment, Rb may be a phenyl group which is unsubstituted or substituted with -CN or SiRR'R"; a biphenyl group which is unsubstituted or substituted with a phenyl group; a terphenyl group; a dimethylfluorenyl group.

In an exemplary embodiment of the present application, Ra and Rb may be substituted with deuterium.

In an exemplary embodiment of the present application, - (L2)a-Ra and Rb of Chemical Formula 2 may be different from each other.

In an exemplary embodiment of the present application, - (L2)a-Ra and Rb of Chemical Formula 2 may be the same as each other.

In another exemplary embodiment, R, R', and R" may be a phenyl group.

In an exemplary embodiment of the present application, the deuterium content of Chemical Formula 2 may be 0% or more and 100% or less.

In another exemplary embodiment, the deuterium content of Chemical Formula 2 may be 10% or more and 100% or less.

In still another exemplary embodiment, the deuterium content of Chemical Formula 2 may be 0%, 100% or 10% to 80%. In an exemplary embodiment of the present application, Rc and Rd are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P(=O)RR'; and -NRR', or two or more adjacent groups may be bonded to each other to form a substituted or unsubstituted aromatic hydrocarbon ring or a substituted or unsubstituted hetero ring.

In another exemplary embodiment, Rc and Rd are the same as or different from each other, and may be each independently selected from the group consisting of hydrogen; deuterium; a substituted or unsubstituted C1 to C60 alkyl group; a substituted or unsubstituted C6 to C60 aryl group; a substituted or unsubstituted C2 to C60 heteroaryl group; - SiRR'R"; -P(=O)RR'; and -NRR'.

In still another exemplary embodiment, Rc and Rd are the same as or different from each other, and may be each independently selected from the group consisting of hydrogen; deuterium; a substituted or unsubstituted C1 to C40 alkyl group; a substituted or unsubstituted C6 to C40 aryl group; a substituted or unsubstituted C2 to C40 heteroaryl group; -SiRR'R"; -P(=O)RR'; and -NRR'.

In yet another exemplary embodiment, Rc and Rd are the same as or different from each other, and may be each independently selected from the group consisting of hydrogen; deuterium; a C1 to C40 alkyl group; a C6 to C40 aryl group; a C2 to C40 heteroaryl group; -SiRR'R"; - P(=O)RR'; and -NRR'.

In yet another exemplary embodiment, Rc and Rd are the same as or different from each other, and may be each independently selected from the group consisting of hydrogen; deuterium; a C1 to C20 alkyl group; a C6 to C20 aryl group; a C2 to C20 heteroaryl group; -SiRR'R"; - P(=O)RR'; and -NRR'.

In yet another exemplary embodiment, Rc and Rd are the same as or different from each other, and may be each independently hydrogen; or deuterium.

In an exemplary embodiment of the present application, r is 7, and Rc may be hydrogen.

In an exemplary embodiment of the present application, r is 7, and Rc may be deuterium.

In an exemplary embodiment of the present application, r is 7, and Rc may be hydrogen; or deuterium.

In an exemplary embodiment of the present application, s is 7, and Rd may be hydrogen.

In an exemplary embodiment of the present application, s is 7, and Rd may be deuterium.

In an exemplary embodiment of the present application, s is 7, and Rd may be hydrogen; or deuterium.

When both the compound of Chemical Formula 1 and the compound of Chemical Formula 2 are included in the organic material layer of the organic light emitting device, better efficiency and service life effects are exhibited. From this result, it can be expected that an exciplex phenomenon will occur when both two compounds are included.

The exciplex phenomenon is a phenomenon in which energy with a magnitude of the HOMO level of a donor (p-host) and the LUMO level of an acceptor (n-host) is released due to an electron exchange between two molecules. When a donor with a good hole transport capacity (p-host) and an acceptor with a good electron transport capacity (n-host) are used as hosts for the light emitting layer, holes are injected into the p-host and electrons are injected into the n-host, so that the driving voltage can be lowered, which can help to improve the service life.

In an exemplary embodiment of the present application, the heterocyclic compound of Chemical Formula 2 may be represented by any one of the following compounds.

| | | | |
|---|---|---|---|
| 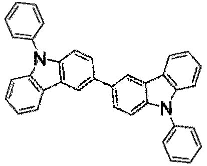 | 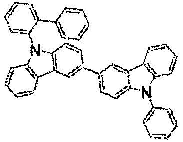 | 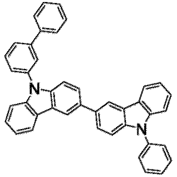 | 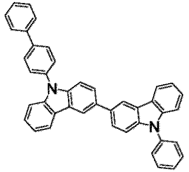 |
| 2-1 | 2-2 | 2-3 | 2-4 |
| 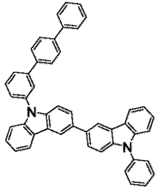 | 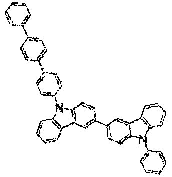 | 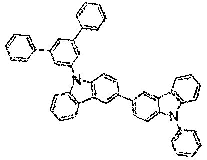 | 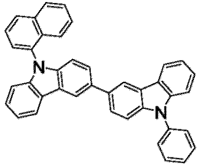 |
| 2-5 | 2-6 | 2-7 | 2-8 |
| 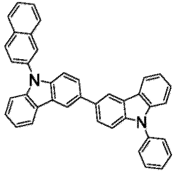 | 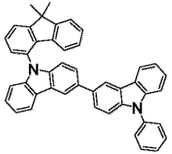 | 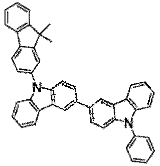 | 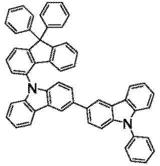 |
| 2-9 | 2-10 | 2-11 | 2-12 |
| 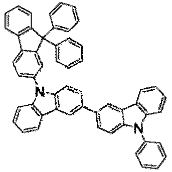 | 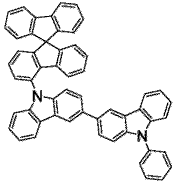 | 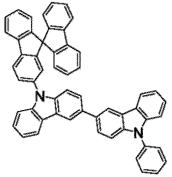 | 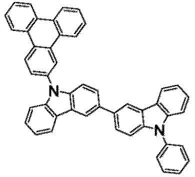 |
| 2-13 | 2-14 | 2-15 | 2-16 |
| 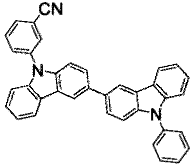 | 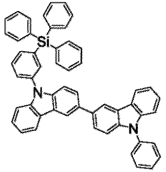 | 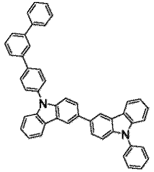 | 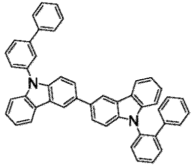 |
| 2-17 | 2-18 | 2-19 | 2-20 |
| 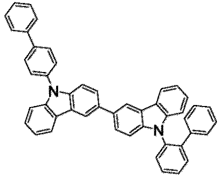 | 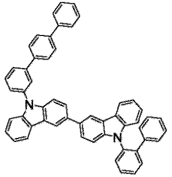 | 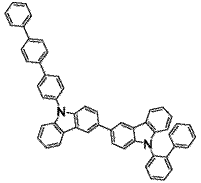 | 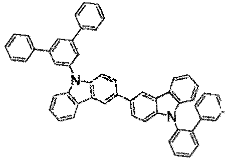 |
| 2-21 | 2-22 | 2-23 | 2-24 |
| 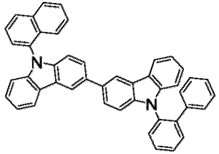 | 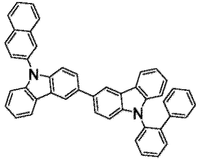 | 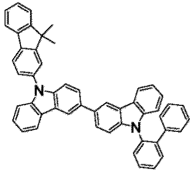 | 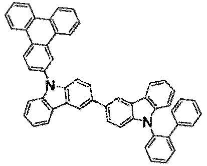 |
| 2-25 | 2-26 | 2-27 | 2-28 |
| 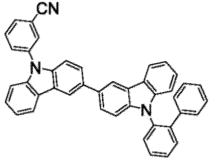 | 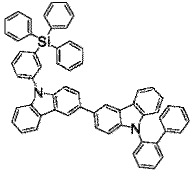 | 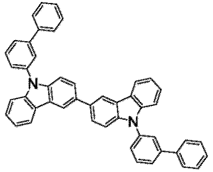 | 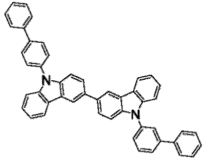 |
| 2-29 | 2-30 | 2-31 | 2-32 |
| 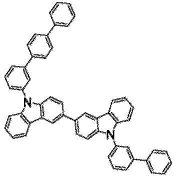 | 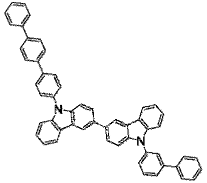 | 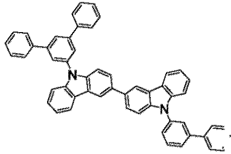 | 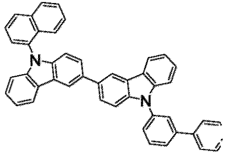 |
| 2-33 | 2-34 | 2-35 | 2-36 |
| 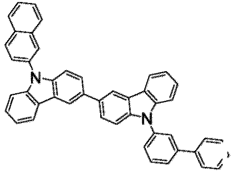 | 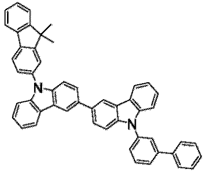 | 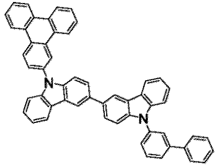 | 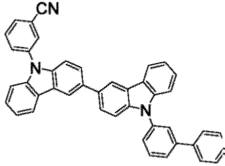 |
| 2-37 | 2-38 | 2-39 | 2-40 |
| 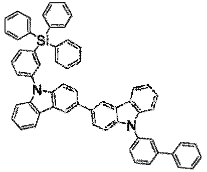 | 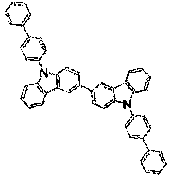 | 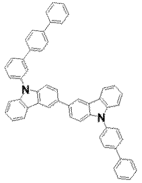 | 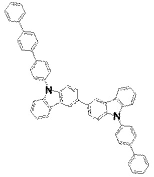 |
| 2-41 | 2-42 | 2-43 | 2-44 |
| 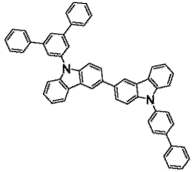 | 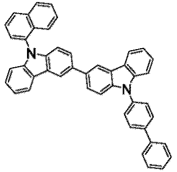 | 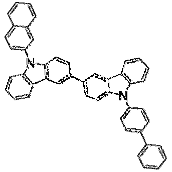 | 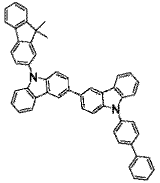 |
| 2-45 | 2-46 | 2-47 | 2-48 |
| 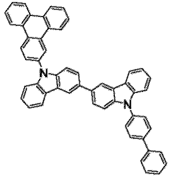 | 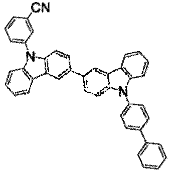 | 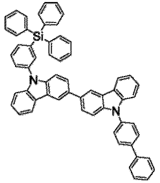 | 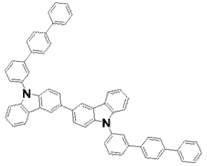 |
| 2-49 | 2-50 | 2-51 | 2-52 |
| 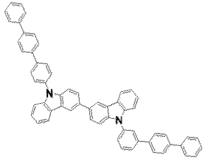 | 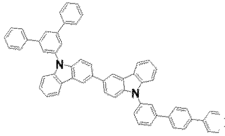 | 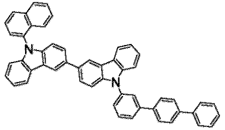 | 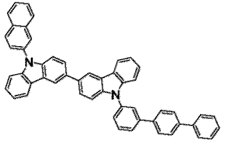 |
| 2-53 | 2-54 | 2-55 | 2-56 |
| 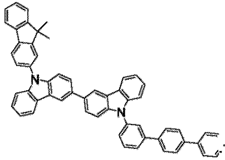 | 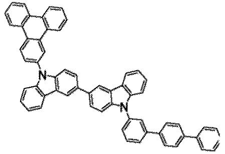 | 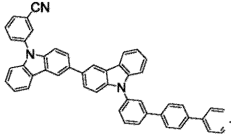 | 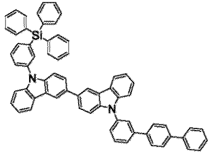 |
| 2-57 | 2-58 | 2-59 | 2-60 |
| 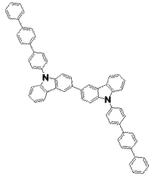 | 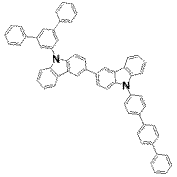 | 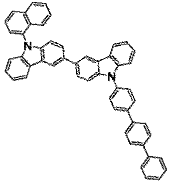 | 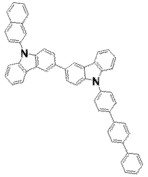 |
| 2-61 | 2-62 | 2-63 | 2-64 |
| 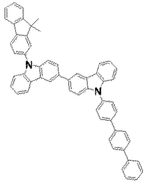 | 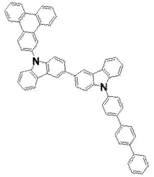 | 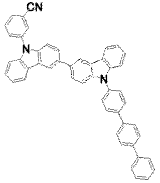 | 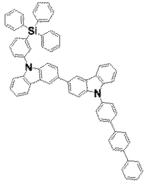 |
| 2-65 | 2-66 | 2-67 | 2-68 |
| 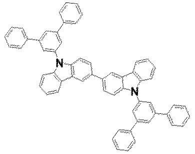 | 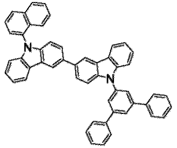 | 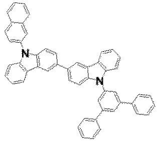 | 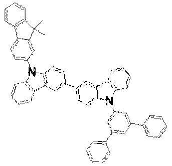 |
| 2-69 | 2-70 | 2-71 | 2-72 |
| 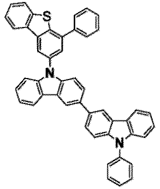 | 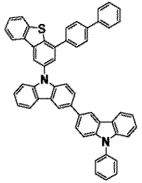 | 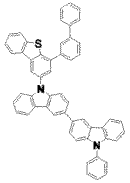 | 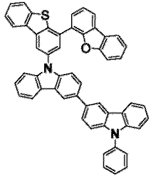 |
| 2-73 | 2-74 | 2-75 | 2-76 |

| | | | |
|---|---|---|---|
| 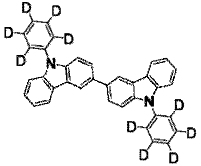 | 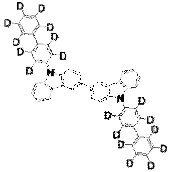 | 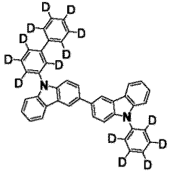 | 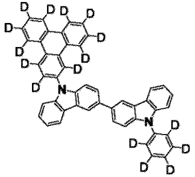 |
| 3-1 | 3-2 | 3-3 | 3-4 |
| 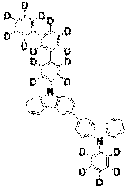 | 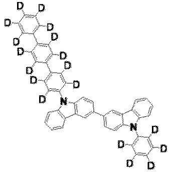 | 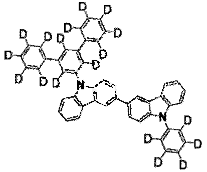 | 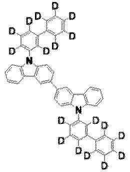 |
| 3-5 | 3-6 | 3-7 | 3-8 |
| 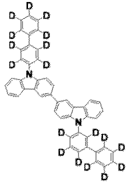 | 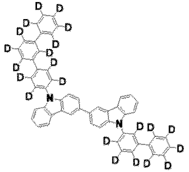 | 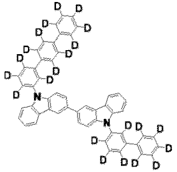 | 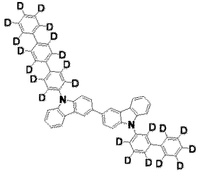 |
| 3-9 | 3-10 | 3-11 | 3-12 |
| 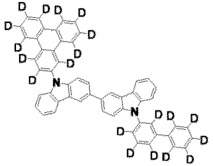 | 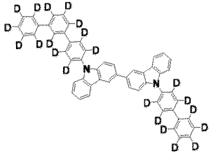 | 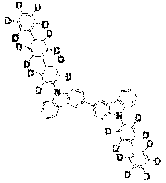 | 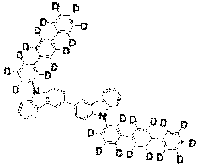 |
| 3-13 | 3-14 | 3-15 | 3-16 |
| 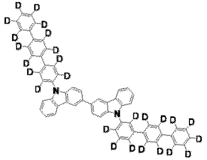 | 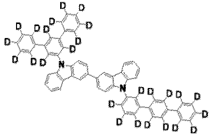 | 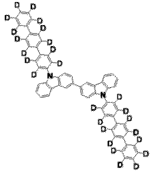 | 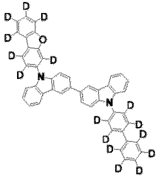 |
| 3-17 | 3-18 | 3-19 | 3-20 |
| 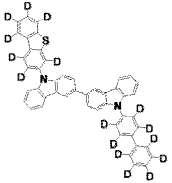 | 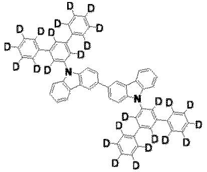 | 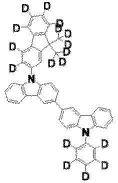 | 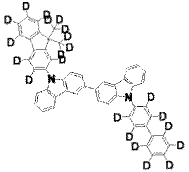 |
| 3-21 | 3-22 | 3-23 | 3-24 |
| 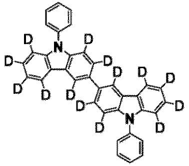 | 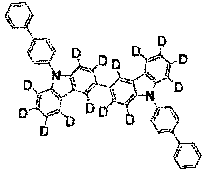 | 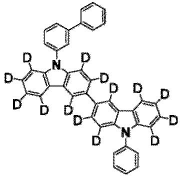 | 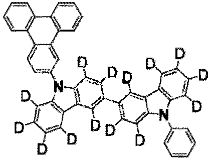 |
| 3-25 | 3-26 | 3-27 | 3-28 |
| 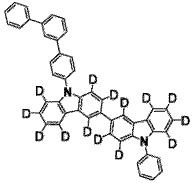 | 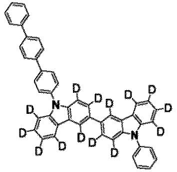 | 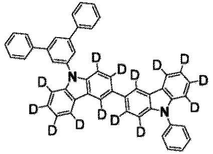 | 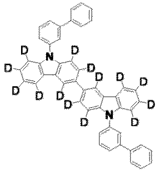 |
| 3-29 | 3-30 | 3-31 | 3-32 |
| 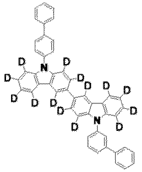 | 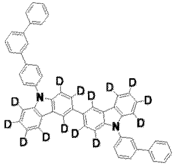 | 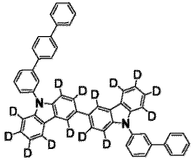 | 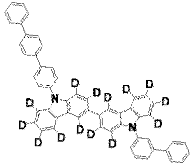 |
| 3-33 | 3-34 | 3-35 | 3-36 |
| 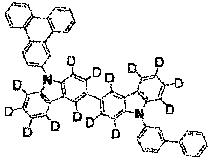 | 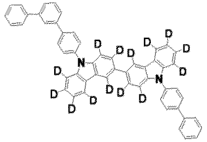 | 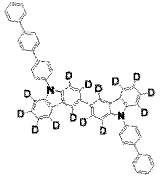 | 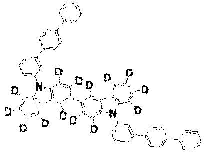 |
| 3-37 | 3-38 | 3-39 | 3-40 |
| 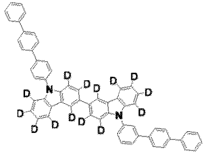 | 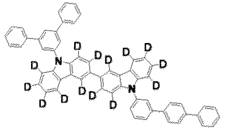 | 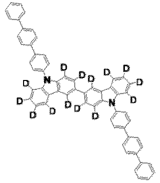 | 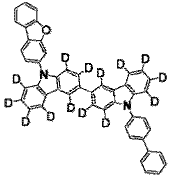 |
| 3-41 | 3-42 | 3-43 | 3-44 |
| 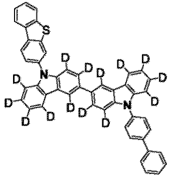 | 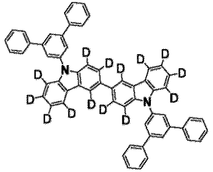 | 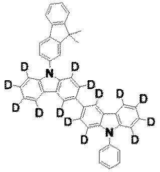 | 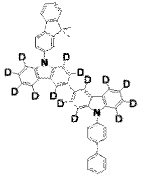 |
| 3-45 | 3-46 | 3-47 | 3-48 |
| 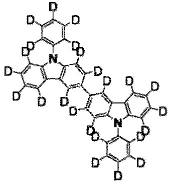 | 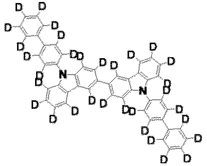 | 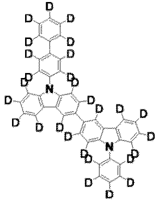 | 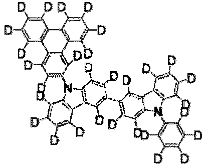 |
| 3-49 | 3-50 | 3-51 | 3-52 |
| 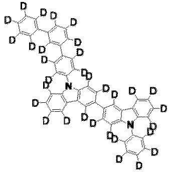 | 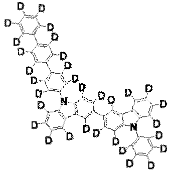 | 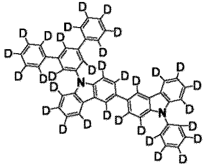 | 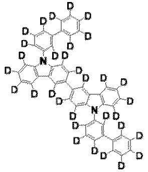 |
| 3-53 | 3-54 | 3-55 | 3-56 |
| 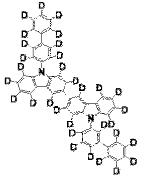 | 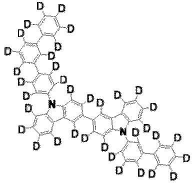 | 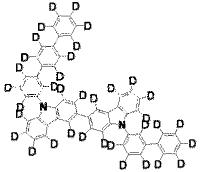 | 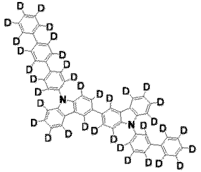 |
| 3-57 | 3-58 | 3-59 | 3-60 |
| 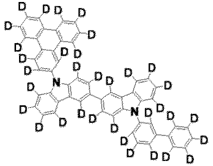 | 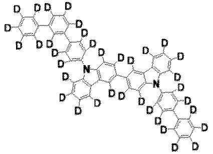 | 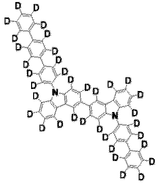 | 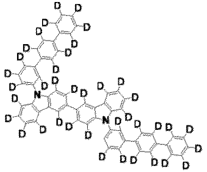 |
| 3-61 | 3-62 | 3-63 | 3-64 |
| 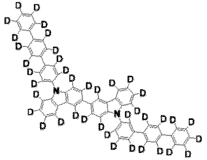 | 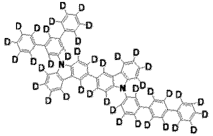 | 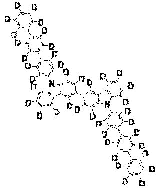 | 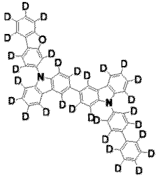 |
| 3-65 | 3-66 | 3-67 | 3-68 |
| 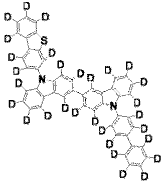 | 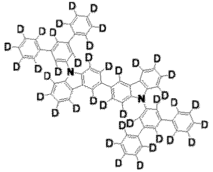 | 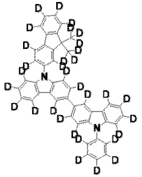 | 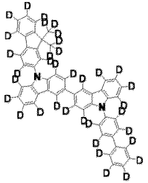 |
| 3-69 | 3-70 | 3-71 | 3-72 |
| 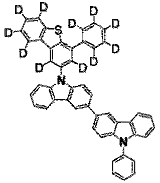 | 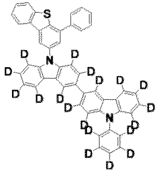 | 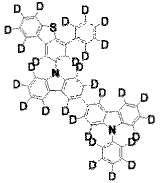 | 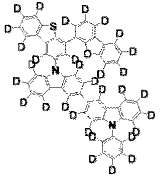 |
| 3-73 | 3-74 | 3-75 | 3-76 |

Further, another exemplary embodiment of the present application provides a composition for an organic material layer of an organic light emitting device, which includes the heterocyclic compound represented by Chemical Formula 1 and the heterocyclic compound represented by Chemical Formula 2.

The specific contents on the heterocyclic compound represented by Chemical Formula 1 and the heterocyclic compound represented by Chemical Formula 2 are the same as those described above.

The weight ratio of the heterocyclic compound represented by Chemical Formula 1 : the heterocyclic compound represented by Chemical Formula 2 in the composition may be 1 : 10 to 10 : 1, 1 : 8 to 8 : 1, 1 : 5 to 5 : 1, and 1 : 2 to 2 : 1, but is not limited thereto.

The composition may be used when an organic material for an organic light emitting device is formed, and particularly, may be more preferably used when a host of a light emitting layer is formed.

The composition is in a form in which two or more compounds are simply mixed, materials in a powder state may also be mixed before an organic material layer of an organic light emitting device is formed, and it is possible to mix compounds in a liquid state at a temperature which is equal to or more than a suitable temperature. The composition is in a solid state at a temperature which is equal to or less than the melting point of each material, and may be maintained as a liquid phase when the temperature is adjusted.

The composition may additionally include materials publicly known in the art such as solvents and additives. The organic light emitting device according to an exemplary embodiment of the present application may be manufactured by typical methods and materials for manufacturing an organic light emitting device, except that the one or more organic material layers are formed by using the heterocyclic compound represented by Chemical Formula 1 and the heterocyclic compound represented by Chemical Formula 2, which are described above.

The compound represented by Chemical Formula 1 and the heterocyclic compound represented by Chemical Formula 2 may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multilayered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic material layers.

In an exemplary embodiment of the present application, the organic light emitting device may be a blue organic light emitting device, and the heterocyclic compound according to Chemical Formula 1 and the heterocyclic compound according to Chemical Formula 2 may be used as a material for the blue organic light emitting device.

In an exemplary embodiment of the present application, the organic light emitting device may be a green organic light emitting device, and the compound represented by Chemical Formula 1 and the heterocyclic compound represented by Chemical Formula 2 may be used as a material for the green organic light emitting device.

In an exemplary embodiment of the present application, the organic light emitting device may be a red organic light emitting device, and the compound represented by Chemical Formula 1 and the heterocyclic compound represented by Chemical Formula 2 may be used as a material for the red organic light emitting device.

The organic light emitting device of the present invention may further include one or two or more layers selected from the group consisting of a light emitting layer, a hole injection layer, a hole transport layer, an electron injection layer, an electron transport layer, an electron blocking layer, and a hole blocking layer.

In an exemplary embodiment of the present application, provided is an organic light emitting device in which the organic material layer includes at least one layer of a hole blocking layer, an electron injection layer, and an electron transport layer, and at least one layer of the hole blocking layer, the electron injection layer, and the electron transport layer includes the heterocyclic compound represented by Chemical Formula 1 and the heterocyclic compound represented by Chemical Formula 2.

In an exemplary embodiment of the present application, provided is an organic light emitting device in which the organic material layer includes a light emitting layer, and the light emitting layer includes the heterocyclic compound represented by Chemical Formula 1 and the heterocyclic compound represented by Chemical Formula 2.

In an exemplary embodiment of the present application, provided is an organic light emitting device in which the organic material layer includes a light emitting layer, the light emitting layer includes a host material, and the host material includes the heterocyclic compound represented by Chemical Formula 1 and the heterocyclic compound represented by Chemical Formula 2.

FIGS. 1 to 3 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

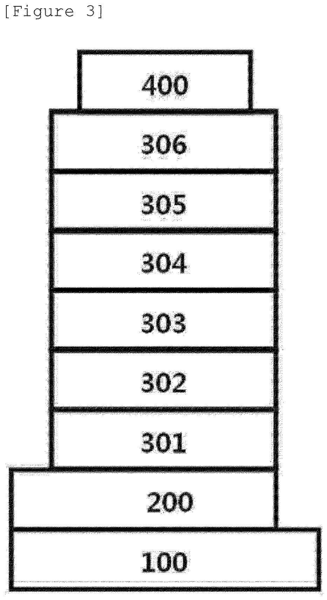

FIG. 3 exemplifies a case where an organic material layer is a multilayer. The organic light emitting device according to FIG. 3 includes a hole injection layer 301, a hole transport layer 302, a light emitting layer 303, a hole blocking layer 304, an electron transport layer 305, and an electron injection layer 306. However, the scope of the present application is not limited by the stacking structure as described above, and if necessary, the other layers except for the light emitting layer may be omitted, and another necessary functional layer may be further added.

In an exemplary embodiment of the present application, provided is a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming the organic material layer having one or more layers by using the composition for an organic material layer according to an exemplary embodiment of the present application.

In an exemplary embodiment of the present application, provided is a method for manufacturing an organic light emitting device, in which the forming of the organic material layer forms the organic material layer by pre-mixing the heterocyclic compound of Chemical Formula 1 and the heterocyclic compound of Chemical Formula 2, and using a thermal vacuum deposition method.

The pre-mixing means that before the heterocyclic compound of Chemical Formula 1 and the heterocyclic compound of Chemical Formula 2 are deposited onto an organic material layer, the materials are first mixed and the mixture is contained in one common container and mixed.

The pre-mixed material may be referred to as a composition for an organic material layer according to an exemplary embodiment of the present application.

In the organic light emitting device according to an exemplary embodiment of the present application, materials other than the heterocyclic compound of Chemical Formula 1 and the heterocyclic compound of Chemical Formula 2 will be exemplified below, but these materials are provided only for exemplification and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transport material, a pyrazoline derivative, an arylamine-based derivative, a stilbene derivative, a triphenyldiamine derivative, and the like may be used, and a low-molecular weight or polymer material may also be used.

As an electron transport material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. In this case, two or more light emitting materials may be deposited or used as an individual supply source, or pre-mixed to be deposited and used as one supply source. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

When hosts of the light emitting material are mixed and used, the same series of hosts may also be mixed and used, and different series of hosts may also be mixed and used. For example, two or more types of materials selected from n-type host materials or p-type host materials may be used as a host material for a light emitting layer.

The organic light emitting device according to an exemplary embodiment of the present application may be a top emission type, a bottom emission type, or a dual emission type according to the material to be used.

The heterocyclic compound according to an exemplary embodiment of the present application may act even in organic electronic devices including organic solar cells, organic photoconductors, organic transistors, and the like, based on the principle similar to those applied to organic light emitting devices.

Hereinafter, the present specification will be described in more detail through Examples, but these Examples are provided only for exemplifying the present application, and are not intended to limit the scope of the present application.

### <Preparation Examples>

### <Preparation Example 1> Preparation of Compound 1-1

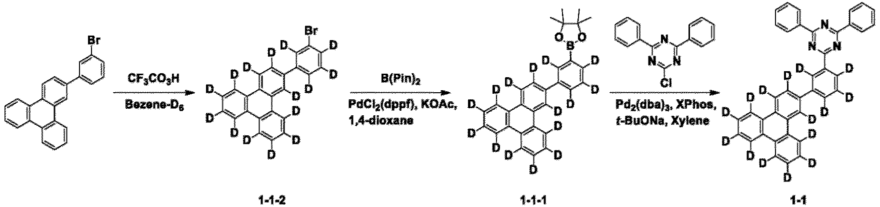

### 1) Preparation of Compound 1-1-2

After 13.9 g (36.2 mM) of 2-(3-bromophenyl)triphenylene and 9.6 mL (108.6 mM) of triflic acid were dissolved in 140 mL of benzene-d6, the resulting solution was refluxed at room temperature. After the reaction was completed, distilled water and dichloromethane (DCM) were added thereto at room temperature, extraction was performed, the organic material layer was dried over MgSO₄, and then the solvent was removed using a rotary evaporator. The product was purified by column chromatography (hexane) to obtain 11.2 g (78%) of Target Compound 1-1-2.

### 2) Preparation of Compound 1-1-1

After 11.2 g (28.2 mM) of Compound 1-1-2, 10.7 g (42.3 mM) of bis(pinacolato)diboron, 1.0 g (1.4 mM) of PdCl₂(dppf), and 8.3 g (84.6 mM) of KOAc were dissolved in 100 mL of 1,4-dioxane, the resulting solution was refluxed for 12 hours. After the reaction was completed, distilled water and dichloromethane (DCM) were added thereto at room temperature, extraction was performed, the organic material layer was dried over MgSO₄, and then the solvent was removed using a rotary evaporator. The product was purified by column chromatography (DCM:hexane = 1:2) to obtain 9.2 g (73%) of Target Compound 1-1-1.

### 3) Preparation of Compound 1-1

After 11.0 g (24.6 mM) of Compound 1-1-1, 7.9 g (29.6 M) of 2-chloro-4,6-diphenyl-1,3,5-triazine, 1.4 g (1.2 mM) of Pd(PPh₃)₄ and 6.8 g (49.2 mM) of K₂CO₃ were dissolved in 100/20 mL of 1,4-dioxane/H₂O, the resulting solution was refluxed for 12 hours. After the reaction was completed, distilled water and dichloromethane (DCM) were added thereto at room temperature, extraction was performed, the organic material layer was dried over MgSO₄, and then the solvent was removed using a rotary evaporator. The product was purified by column chromatography (DCM:hexane = 1:3) to obtain 11.0 g (81%) of Target Compound 1-1.

For reference, the reaction of substituting the hydrogen of the organic compound with deuterium increases the deuterium substitution rate as the equivalent is increased using triflic acid that is a strong acid, but it can be confirmed that when a specific equivalent or more is used, reactants and products are decomposed, so that the yield and deuterium substitution rate are also lowered. Similarly, the concentration and reaction temperature capable of increasing the reaction rate improve the deuterium substitution rate up to a specific equivalent, but when the concentration and reaction temperature are excessive, the compound may be decomposed to inhibit the yield and deuterium substitution rate.

Here, the substitution rate is calculated by [(the number of deuteriums substituted after the chemical reaction)/(the number of hydrogens in the compound before the chemical reaction)]*100.

Considering the above tendency, in the case of the preparation of Compound 1-1-2, the deuterium-substituted material synthesis method was performed according to the conditions shown in the following Table 1, and a reaction condition test experiment was conducted.

As a result, it was confirmed that Condition 2 was a condition with the highest yield and substitution rate. Accordingly, Compound 1-1-2 was synthesized under Condition 2, which has the highest substitution rate, as a reaction condition. In addition, the decomposition of compounds in strong acids is an inherent property, and the experiment was performed by varying reaction conditions as the compound is varied.

**[Table 1]**

| Condition | Compound | Benzene-D₆ | triflic acid | Temperature | Yield | Substitution rate |
|---|---|---|---|---|---|---|
| 1 | 1 g, 1 eq | 68 eq,10 mL | 1 eq | RT | 95% | 72% |
| 2 | | | 3 eq | RT | 86% | 93% |
| 3 | | | 3 eq | 40°C | 77% | 88% |
| 4 | | | 3 eq | 80°C | 68% | 84% |
| 5 | | | 5 eq | RT | 80% | 810 |

The target compound was prepared and synthesized in the same manner as in Preparation Example 1, except that Intermediate A in the following Table 2 was used instead of 2-bromotriphenylene, and Intermediate B in the following Table 2 was used instead of 2-chloro-4,6-diphenyl-1,3,5-triazine in Preparation Example 1.

**[Table 2]**

| Compound No. | Intermediate A | Intermediate B | Target compound |
|---|---|---|---|
| 1-4 | 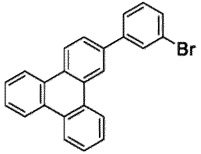 | 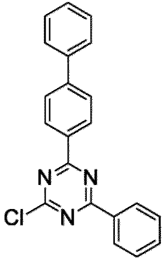 | 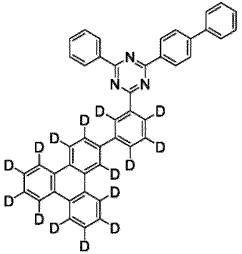 |
| 1-17 | | 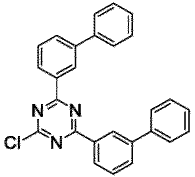 | 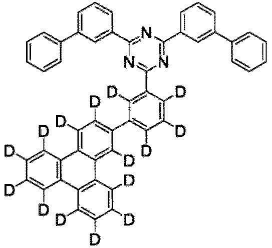 |
| 1-19 | | 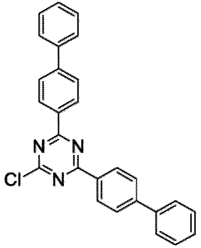 | 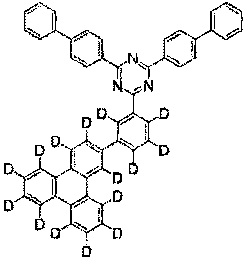 |
| 1-51 | | 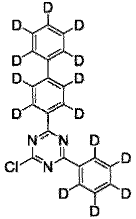 | 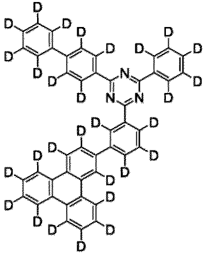 |
| 1-77 | 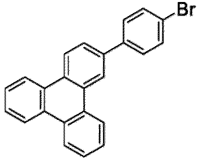 | 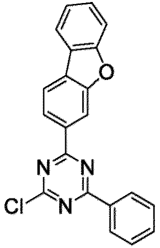 | 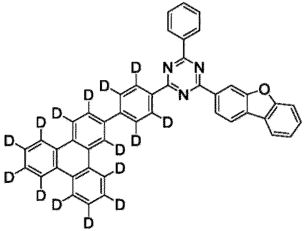 |
| 1-111 | | 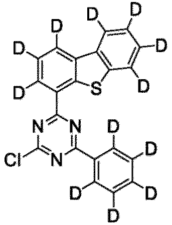 | 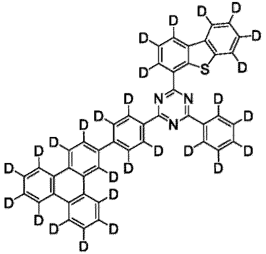 |
| 1-113 | 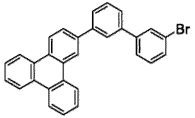 | 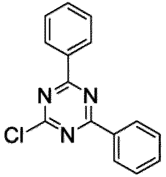 | 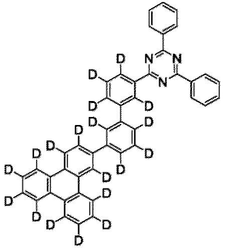 |
| 1-116 | | 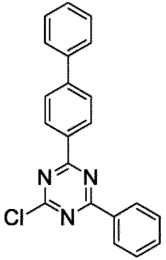 | 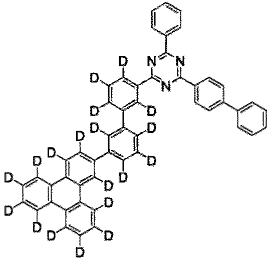 |
| 1-118 | | 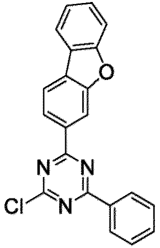 | 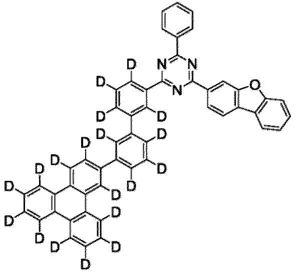 |
| 1-127 | | 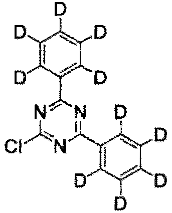 | 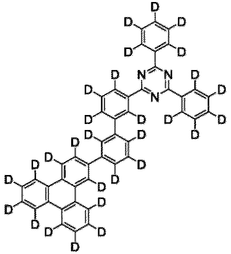 |
| 1-133 | 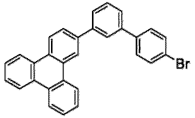 | 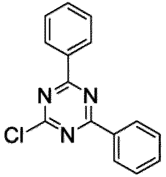 | 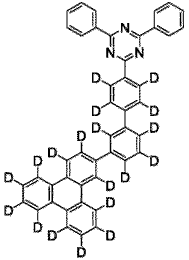 |
| 1-142 | | 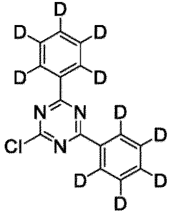 | 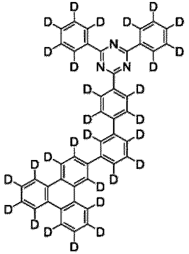 |
| 1-147 | 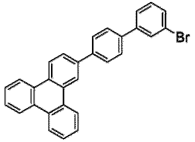 | 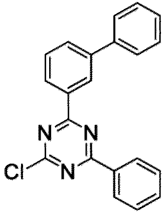 | 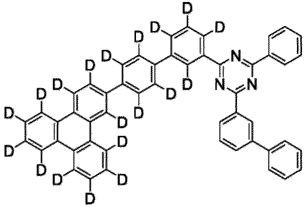 |
| 1-160 | | 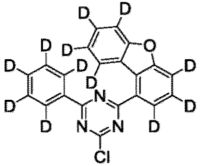 | 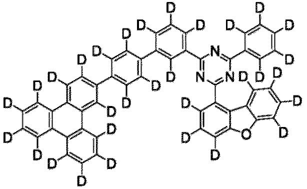 |
| 1-161 | 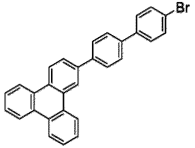 | 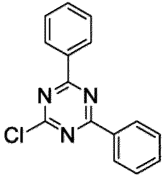 | 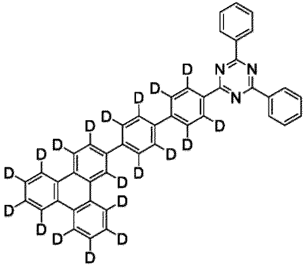 |
| 1-167 | | 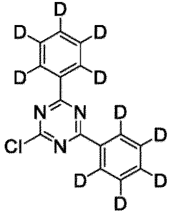 | 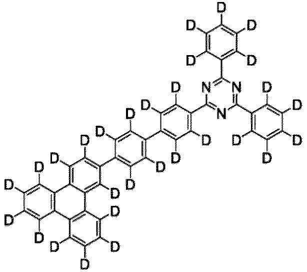 |
| 1-169 | 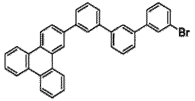 | 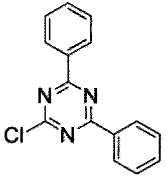 | 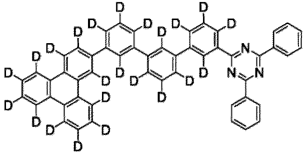 |
| 1-178 | | 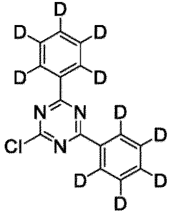 | 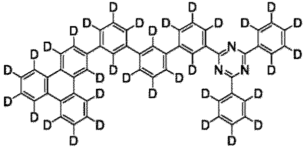 |

### <Preparation Example 2> Preparation of Compound 2-3

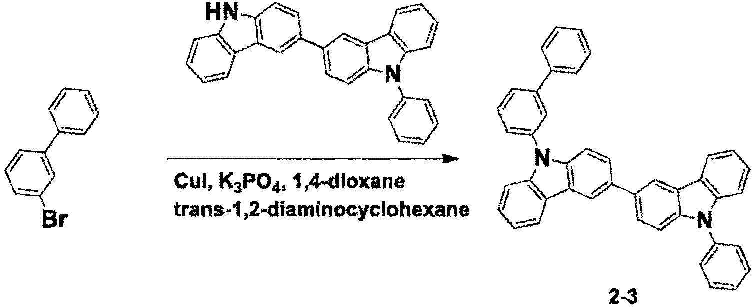

### 1) Preparation of Compound 2-3

After 3.7 g (15.8 mM) of 3-bromo-1,1'-biphenyl, 6.5 g (15.8 mM) of 9-phenyl-9H,9'H-3,3'-bicarbazole, 3.0 g (15.8 mM) of CuI, 1.9 mL (15.8 mM) of trans-1,2-diaminocyclohexane, and 3.3 g (31.6 mM) of K₃PO₄ were dissolved in 100 mL of 1,4-dioxane, the resulting solution was refluxed for 24 hours. After the reaction was completed, extraction was performed by adding distilled water and dichloromethane (DCM) thereto at room temperature, the organic material layer was dried over MgSO₄, and then the solvent was removed using a rotary evaporator. The product was purified by column chromatography (DCM:Hex = 1:3) and recrystallized with methanol to obtain 7.5 g (85%) of Target Compound 2-3.

The following target compounds were prepared and synthesized in the same manner as in Preparation Example 2, except that Intermediate A in the following Table 3 was used instead of 3-bromo-1,1'-biphenyl, and Intermediate B in the following Table 3 was used instead of 9-phenyl-9H,9'H-3,3'-bicarbazole in Preparation Example 2.

**[Table 3]**

| Compound No. | Intermediate A | Intermediate B | Target compound |
|---|---|---|---|
| 2-4 | 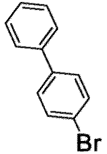 | 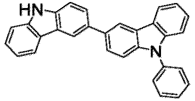 | 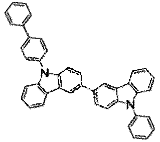 |
| 2-7 | 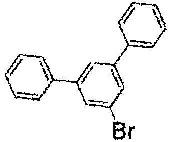 | 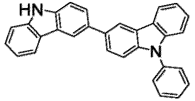 | 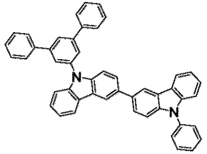 |
| 2-9 | 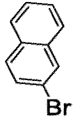 | 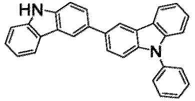 | 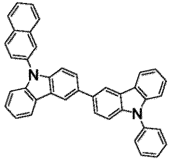 |
| 2-31 | 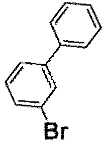 | 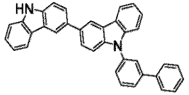 | 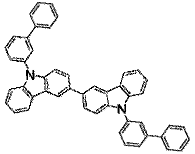 |
| 2-32 | 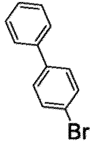 | 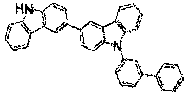 | 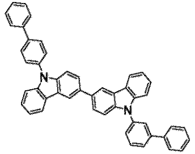 |
| 2-42 | 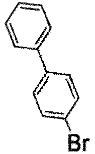 | 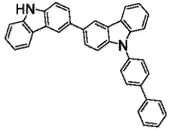 | 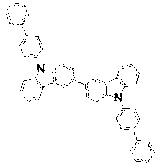 |

### <Preparation Example 3> Preparation of Compound 2-73

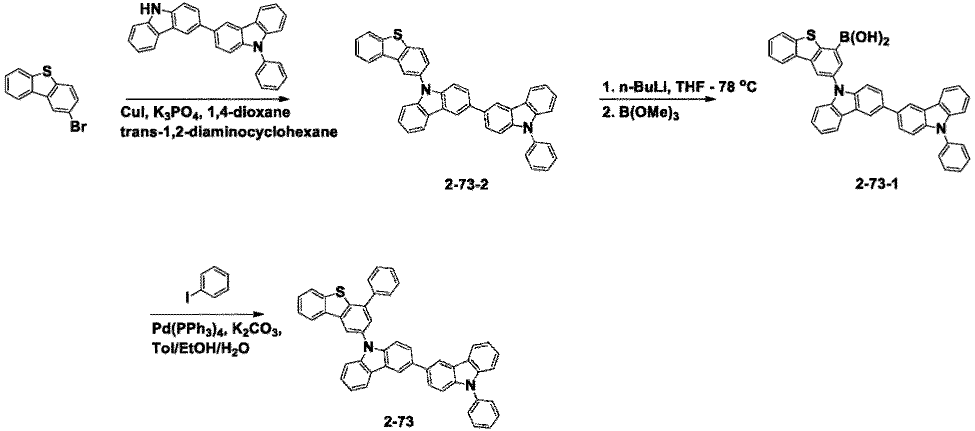

### 1) Preparation of Compound 2-73-2

After 4.2 g (15.8 mM) of 2-bromodibenzo[b,d]thiophene, 6.5 g (15.8 mM) of 9-phenyl-9H,9'H-3,3'-bicarbazole, 3.0 g (15.8 mM) of CuI, 1.9 mL (15.8 mM) of trans-1,2-diaminocyclohexane, and 3.3 g (31.6 mM) of K₃PO₄ were dissolved in 100 mL of 1,4-dioxane, the resulting solution was refluxed for 24 hours. After the reaction was completed, extraction was performed by adding distilled water and dichloromethane (DCM) thereto at room temperature, the organic material layer was dried over MgSO4, and then the solvent was removed using a rotary evaporator. The product was purified by column chromatography (DCM:Hex = 1:3) and recrystallized with methanol to obtain 7.9 g (85%) of Target Compound 2-73-2.

### 2) Preparation of Compound 2-73-1

After 8.4 g (14.3 mmol) of Compound 2-73-2 was dissolved in 100 mL of THF, the resulting solution was substituted with nitrogen at -78°C. 7.4 mL (18.6 mmol) of 2.5 M n-BuLi was added dropwise thereto, and the resulting mixture was stirred at room temperature for 1 hour. 4.8 mL(42.9 mmol) of trimethyl borate was added dropwise to the reaction mixture, and the resulting mixture was stirred at room temperature for 2 hours. After the reaction was completed, extraction was performed by adding distilled water and dichloromethane (DCM) thereto at room temperature, the organic material layer was dried over MgSO₄, and then the solvent was removed using a rotary evaporator. The product was purified by column chromatography (DCM:MeOH = 100:3) and recrystallized with DCM to obtain 3.9 g (70%) of Target Compound 2-73-1.

### 3) Preparation of Compound 2-73

After 6.7 g (10.5 mM) of Compound 2-73-1, 2.1 g (10.5 mM) of iodobenzene, 606 mg (0.52 mM) of Pd(PPh₃)₄, and 2.9 g (21.0 mM) of K₂CO₃ were dissolved in 100/20/20 mL of toluene/EtOH/H₂O, the resulting solution was refluxed for 12 hours. After the reaction was completed, extraction was performed by adding distilled water and dichloromethane (DCM) thereto at room temperature, the organic material layer was dried over MgSO₄, and then the solvent was removed using a rotary evaporator. The product was purified by column chromatography (DCM:hexane = 1:3) and recrystallized with methanol to obtain 4.9 g (70%) of Target Compound 2-73.

### <Preparation Example 4> Preparation of Compound 3-2

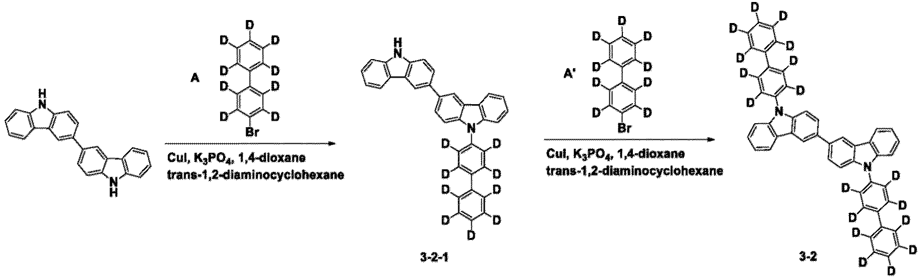

### 1) Preparation of Compound 3-2-1

After 10 g (30.0 mM) of 9H,9'H-3,3'-bicarbazole, 7.26 g (30 mM) of 4-bromo-1,1'-biphenyl-2,2',3,3',4',5,5',6,6'-D₉ [A], 0.57 g (3.0 mM) of CuI, 0.34 g (30 mM) of trans-1,2-diaminocyclohexane, and 12.7 g (60.0 mM) of K₃PO₄ were dissolved in 100 mL of 1,4-dioxane, the resulting solution was refluxed at 125°C for 8 hours. After the reaction was completed, distilled water and dichloromethane (DCM) were added thereto at room temperature, extraction was performed, the organic material layer was dried over MgSO₄, and then the solvent was removed by a rotary evaporator. The product was purified by column chromatography (DCM:hexane = 1:3) and recrystallized with methanol to obtain 13.9 g (94%) of Target Compound 3-2-1.

### 2) Preparation of Compound 3-2

After 13.9 g (28.0 mM) of Compound 3-2-1, 6.8 g (28.0 mM) of 4-bromo-1,1'-biphenyl-2,2',3,3',4',5,5',6,6'-D₉ [A'], 0.53 g (2.8 mM) of CuI, 0.32 g (2.8 mM) of trans-1,2-diaminocyclohexane, and 11.9 g (56.0 mM) of K₃PO₄ were dissolved in 140 mL of 1,4-dioxane, the resulting solution was refluxed at 125°C for 8 hours. After the reaction was completed, distilled water and dichloromethane (DCM) were added thereto at room temperature, extraction was performed, the organic material layer was dried over MgSO₄, and then the solvent was removed by a rotary evaporator. The product was purified by column chromatography (DCM:hexane = 1:3) and recrystallized with methanol to obtain 16.1 g (88%) of Target Compound 3-2.

When Compound A and Compound A' are the same, the target compound may be immediately synthesized by adding 2 equivalents of Compound A in Preparation Example 4. That is, when Compound A and Compound A' are the same, the preparation of Compound 3-2-1 may be omitted.

The following target compounds were prepared and synthesized in the same manner as in Preparation Example 4, except that Intermediate A in the following Table 4 was used instead of 4-bromo-1,1'-biphenyl-2,2',3,3',4',5,5',6,6'-D₉ [A] and Intermediate A' in the following Table 4 was used instead of 4-bromo-1,1'-biphenyl-2,2',3,3',4',5,5',6,6'-D₉ [A'] in Preparation Example 4.

**[Table 4]**

| Compound No. | Intermediate A | Intermediate A' | Target compound |
|---|---|---|---|
| 3-3 | 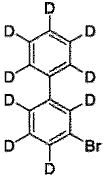 | 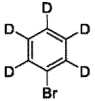 | 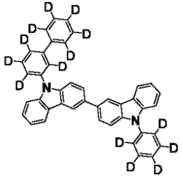 |
| 3-5 | 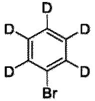 | 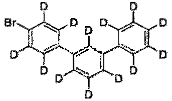 | 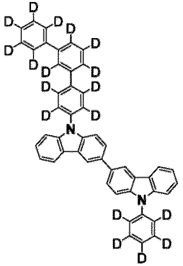 |
| 3-6 | | 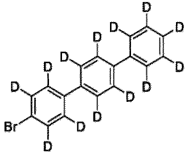 | 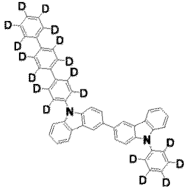 |
| 3-8 | 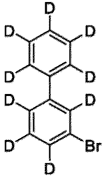 | 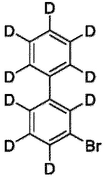 | 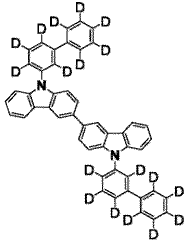 |
| 3-19 | 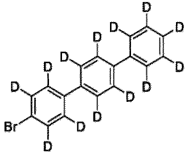 | 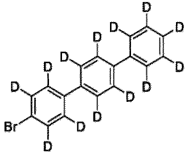 | 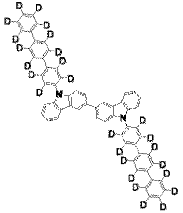 |
| 3-22 | 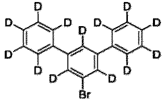 | 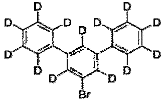 | 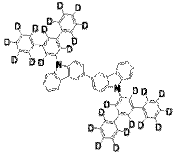 |

### <Preparation Example 5> Preparation of Compound 3-26

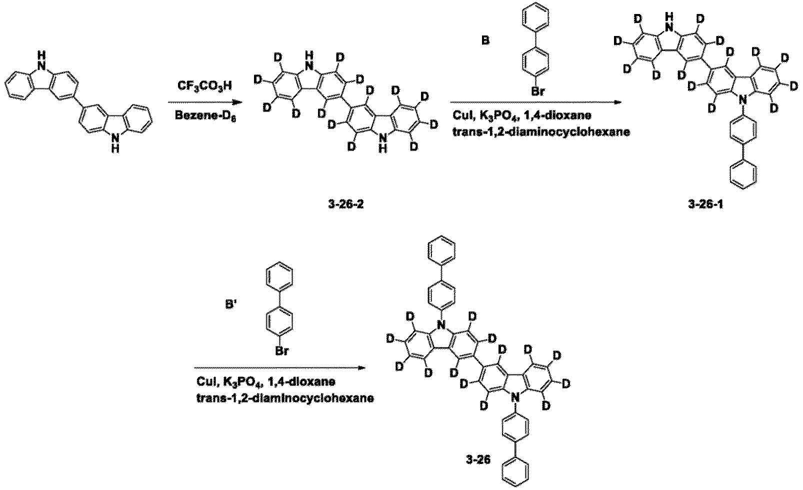

### 1) Preparation of Compound 3-26-2

After 12.0 g (36.2 mM) of 9H,9'H-3,3'-bicarbazole and 9.6 mL (108.6 mM) of triflic acid were dissolved in 140 mL of benzene-d6, the resulting solution was refluxed at room temperature. After the reaction was completed, distilled water and dichloromethane (DCM) were added thereto at room temperature, extraction was performed, the organic material layer was dried over MgSO₄, and then the solvent was removed using a rotary evaporator. The product was purified by column chromatography (DCM:hexane = 1:3) to obtain 8.9 g (71%) of Target Compound 3-26-2.

### 2) Preparation of Compound 3-26-1

After 5.5 g (15.8 mM) of Compound 3-26-2, 3.7 g (15.8 mM) of 4-bromo-1,1'-biphenyl [B], 3.0 g (15.8 mM) of CuI, 1.9 mL (15.8 mM) of trans-1,2-diaminocyclohexane, and 3.3 g (31.6 mM) of K₃PO₄ were dissolved in 100 mL of 1,4-dioxane, the resulting solution was refluxed for 24 hours. After the reaction was completed, extraction was performed by adding distilled water and dichloromethane (DCM) thereto at room temperature, the organic material layer was dried over MgSO₄, and then the solvent was removed using a rotary evaporator. The product was purified by column chromatography (DCM:Hex = 1:3) and recrystallized with methanol to obtain 6.5g (83%) of Target Compound 3-26-1.

### 2) Preparation of Compound 3-26

After 14.0 g (28.0 mM) of Compound 3-26-1, 6.8 g (28.0 mM) of 4-bromo-1,1'-biphenyl [B'], 0.53 g (2.8 mM) of CuI, 0.32 g (2.8 mM) of trans-1,2-diaminocyclohexane, and 11.9 g (56.0 mM) of K₃PO₄ were dissolved in 140 mL of 1,4-dioxane, the resulting solution was refluxed at 125°C for 8 hours. After the reaction was completed, distilled water and dichloromethane (DCM) were added thereto at room temperature, extraction was performed, the organic material layer was dried over MgSO₄, and then the solvent was removed by a rotary evaporator. The product was purified by column chromatography (DCM:hexane = 1:3) and recrystallized with methanol to obtain 15.8g (87%) of Target Compound 3-26.

When Compound B and Compound B' are the same, the target compound may be immediately synthesized by adding 2 equivalents of Compound B in Preparation Example 5. That is, when Compound B and Compound B' are the same, the preparation of Compound 3-26-1 may be omitted.

The following target compounds were prepared and synthesized in the same manner as in Preparation Example 5, except that Intermediate B in the following Table 5 was used instead of 4-bromo-1,1'-biphenyl [B] and Intermediate B' in the following Table 5 was used instead of 4-bromo-1,1'-biphenyl [B'] in Preparation Example 5.

**[Table 5]**

| Compound No. | Intermediate B | Intermediate B' | Target compound |
|---|---|---|---|
| 3-26 | 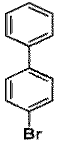 | 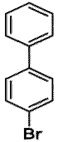 | 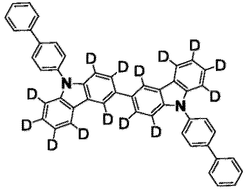 |
| 3-27 | 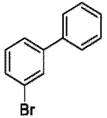 | 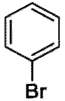 | 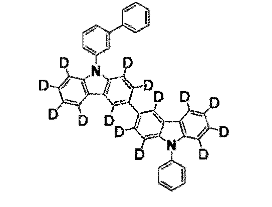 |
| 3-29 | 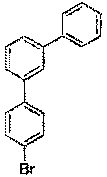 | | 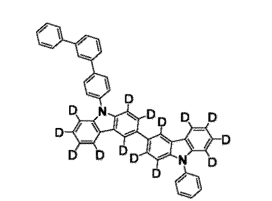 |
| 3-32 | 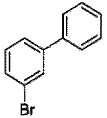 | 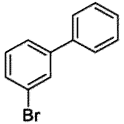 | 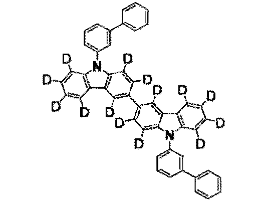 |
| 3-33 | 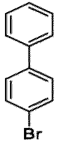 | | 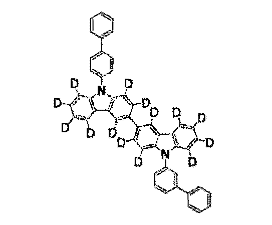 |
| 3-36 | 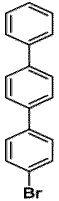 | | 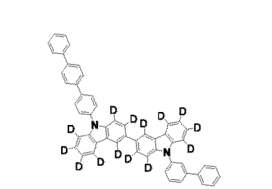 |
| 3-39 | | 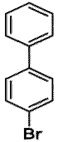 | 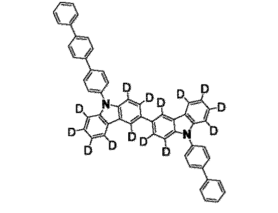 |
| 3-44 | 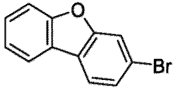 | | 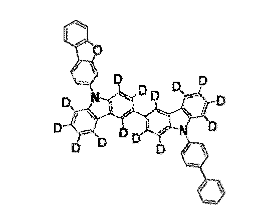 |

### <Preparation Example 6> Preparation of Compound 3-50

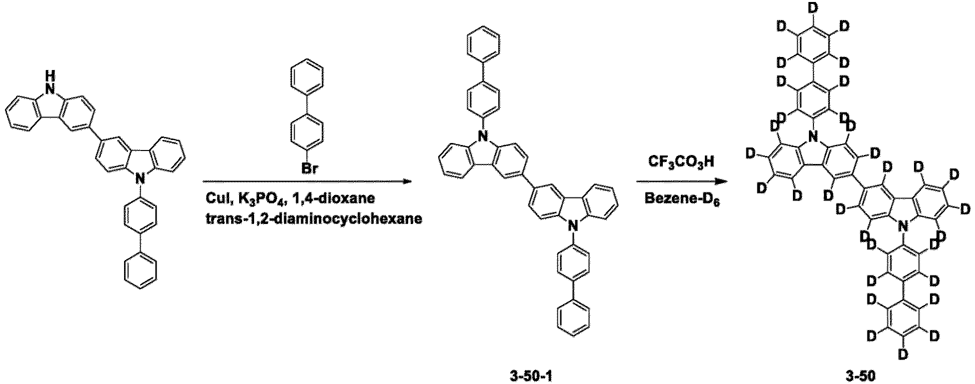

### 1) Preparation of Compound 3-50-1

After 13.6 g (28.0 mM) of 9-([1,1'-biphenyl]-4-yl)-9H,9'H-3,3'-bicarbazole, 6.8 g (28.0 mM) of 4-bromo-1,1'-biphenyl, 0.53 g (2.8 mM) of CuI, 0.32 g (2.8 mM) of trans-1,2-diaminocyclohexane, and 11.9 g (56.0 mM) of K₃PO₄ were dissolved in 140 mL of 1,4-dioxane, the resulting solution was refluxed at 125°C for 8 hours. After the reaction was completed, distilled water and dichloromethane (DCM) were added thereto at room temperature, extraction was performed, the organic material layer was dried over MgSO₄, and then the solvent was removed by a rotary evaporator. The product was purified by column chromatography (DCM:hexane = 1:3) and recrystallized with methanol to obtain 15.9 g (89%) of Target Compound 3-50-1.

### 2) Preparation of Compound 3-50

After 23.0 g (36.2 mM) of Compound 3-50-1 and 9.6 mL (108.6 mM) of triflic acid were dissolved in 140 mL of benzene-d6, the resulting solution was refluxed at room temperature.

After the reaction was completed, distilled water and dichloromethane (DCM) were added thereto at room temperature, extraction was performed, the organic material layer was dried over MgSO₄, and then the solvent was removed using a rotary evaporator. The product was purified by column chromatography (DCM:hexane = 1:3) to obtain 16.9 g (70%) of Target Compound 3-50.

The following target compounds were prepared and synthesized in the same manner as in Preparation Example 6, except that Intermediate C in the following Table 6 was used instead of 9-([1,1'-biphenyl]-4-yl)-9H,9'H-3,3'-bicarbazole and Intermediate C' in the following Table 6 was used instead of 4-bromo-1,1'-biphenyl in Preparation Example 6.

**[Table 6]**

| Compound No. | Intermediate C | Intermediate C' | Target compound |
|---|---|---|---|
| 3-50 | 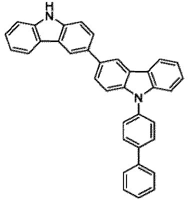 | 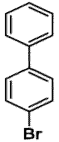 | 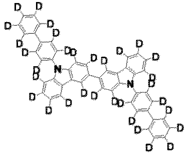 |
| 3-51 | 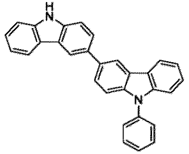 | 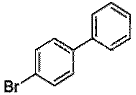 | 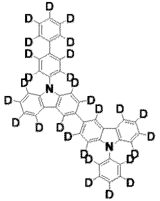 |
| 3-53 | 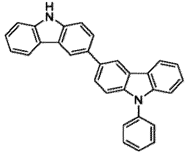 | 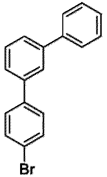 | 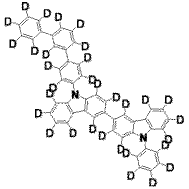 |
| 3-56 | 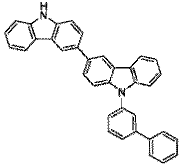 | 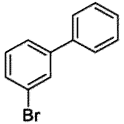 | 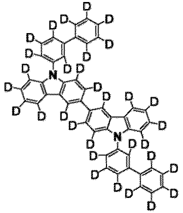 |
| 3-57 | 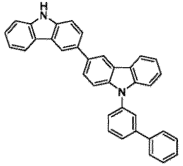 | 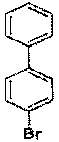 | 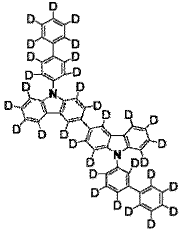 |
| 3-58 | 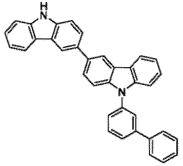 | 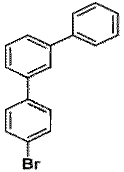 | 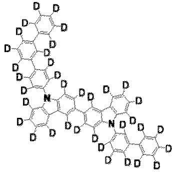 |
| 3-60 | 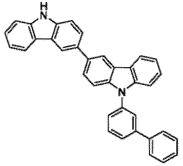 | 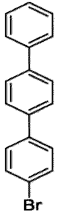 | 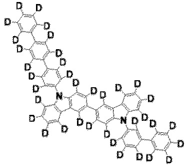 |
| 3-62 | 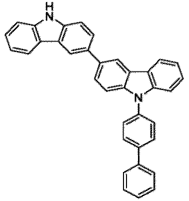 | 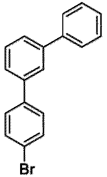 | 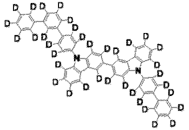 |
| 3-63 | 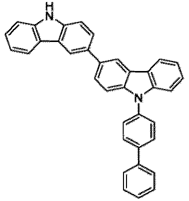 | 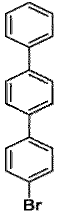 | 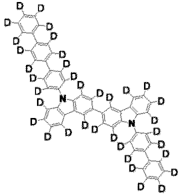 |
| 3-67 | 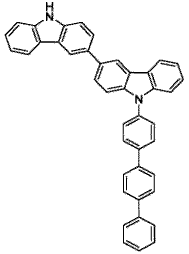 | 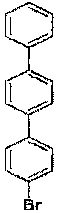 | 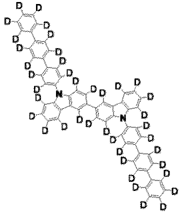 |
| 3-68 | 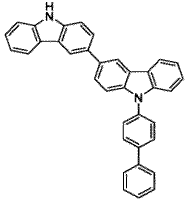 | 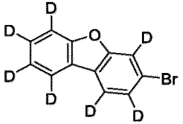 | 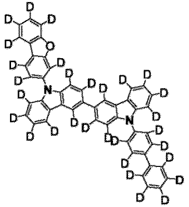 |

The other compounds other than the compounds described in Preparation Examples 1 to 6 and Tables 2 to 6 were also prepared in the same manner as in the above-described Preparation Examples, and synthesis results are shown in the following Tables 7 and 8. The following Table 7 shows the measured values of ¹H NMR(CDCl₃, 200 Mz), and Table 8 shows the measured values of field desorption mass spectrometry (FD-MS).

**[Table 7]**

| Compound No. | ¹H NMR(CDCl₃, 200Mz) |
|---|---|
| 1-1 | δ= 8.36 (4H, d), 7.50 (6H, m) |
| 1-4 | δ= 8.36 (2H, d), 7.96 (2H, d), 7.75 (2H, d), 7.50-7.41 (6H, m), 7.25(2H, d) |
| 1-17 | δ= 8.38 (2H, d), 7.94 (2H, s), 7.75-7.73 (3H, m), 7.61 (2H, d), 7.49-7.41 (6H, m) |
| 1-19 | δ= 7.96 (4H, d), 7.75 (4H, d), 7.49-7.41 (6H, m), 7.25 (2H, d) |
| 1-51 | δ= Deuterium content 100%, no peak |
| 1-77 | δ= 8.36 (2H, d), 8.03-7.98 (2H, m), 7.82-7.76 (2H, m), 7.54-7.50 (4H, m), 7.39-7.31 (2H, m) |
| 1-111 | δ= Deuterium content 100%, no peak |
| 1-113 | δ= 8.36 (4H, d), 7.50 (6H, m) |
| 1-116 | δ= 8.36 (2H, d), 7.96 (2H, d), 7.75 (2H, d), 7.50-7.41 (6H, m), 7.25(2H, d) |
| 1-118 | δ= 8.36 (2H, d), 8.03-7.98 (2H, m), 7.82-7.76 (2H, m), 7.54-7.50 (4H, m), 7.39-7.31 (2H, m) |
| 1-127 | δ= Deuterium content 100%, no peak |
| 1-133 | δ= 8.36 (4H, d), 7.50 (6H, m) |
| 1-142 | δ= Deuterium content 100%, no peak |
| 1-147 | δ= 8.38-8.36 (3H, m), 7.94 (1H, s), 7.75-7.73 (3H, m), 7.61 (2H, d), 7.50-7.41 (6H, m) |
| 1-160 | δ= Deuterium content 100%, no peak |
| 1-161 | δ= 8.36 (4H, d), 7.50 (6H, m) |
| 1-167 | δ= Deuterium content 100%, no peak |
| 1-169 | δ= 8.36 (4H, d), 7.50 (6H, m) |
| 1-178 | δ= Deuterium content 100%, no peak |
| 2-42 | δ= 8.55 (1H, d), 8.30 (1H, d), 8.19-8.13 (2H, m), 7.99-7.89 (12H, m), 7.77-7.75 (5H, m), 7.50-7.35 (8H, m), 7.20-7.16 (2H, m) |
| 3-2 | δ= 8.55 (1H, d), 8.30 (1H, d), 8.19-8.13 (2H, m), 7.99-7.89 (4H, m), 7.77 (1H, d), 7.58-7.50 (2H, m), 7.35 (1H, m), 7.20-7.16 (2H, m) |
| 3-26 | δ= 7.92 (4H, d), 7.91 (4H, d), 7.75 (2H, d), 7.49-7.41 (6H, m) |
| 3-50 | δ= Deuterium content 100%, no peak |

**[Table 8]**

| Compound | FD-MS | Compound | FD-MS |
|---|---|---|---|
| 1-1 | Chemical Formula: C39H10D15N3Exact Mass: 550.299 Molecular Weight: 550.742 | 1-4 | Chemical Formula: C45H14D15N3 Exact Mass: 626.330 Molecular Weight: 626.840 |
| 1-17 | Chemical Formula: C51H18D15N3Exact Mass: 702.362 Molecular Weight: 702.938 | 1-19 | Chemical Formula: C51H18D15N3 Exact Mass: 702.362 Molecular Weight: 702.938 |
| 1-51 | Chemical Formula: C45D29N3Exact Mass: 640.418 Molecular Weight: 640.925 | 1-77 | Chemical Formula: C45H12D15N3O Exact Mass: 640.310 Molecular Weight: 640.823 |
| 1-111 | Chemical Formula: C45D27N3SExact Mass: 668.362 Molecular Weight: 668.957 | 1-113 | Chemical Formula: C45H10D19N3 Exact Mass: 630.355 Molecular Weight: 630.864 |
| 1-116 | Chemical Formula: C51H14D19N3Exact Mass: 706.387 Molecular Weight: 706.962 | 1-118 | Chemical Formula: C51H12D19N3O Exact Mass: 720.366 Molecular Weight: 720.945 |
| 1-127 | Chemical Formula: C45D29N3Exact Mass: 640.418 Molecular Weight: 640.925 | 1-133 | Chemical Formula: C45H10D19N3 Exact Mass: 630.355 Molecular Weight: 630.864 |
| 1-142 | Chemical Formula: C45D29N3Exact Mass: 640.418 Molecular Weight: 640.925 | 1-147 | Chemical Formula: C51H14D19N3 Exact Mass: 706.387 Molecular Weight: 706.962 |
| 1-160 | Chemical Formula: C51D31N3OExact Mass: 732.441 Molecular Weight: 733.018 | 1-161 | Chemical Formula: C45H10D19N3 Exact Mass: 630.355 Molecular Weight: 630.864 |
| 1-167 | Chemical Formula: C45D29N3Exact Mass: 640.418 Molecular Weight: 640.925 | 1-169 | Chemical Formula: C51H10D23N3 Exact Mass: 710.412 Molecular Weight: 710.986 |
| 1-178 | Chemical Formula: C51D33N3Exact Mass: 720.475 Molecular Weight: 721.047 | 2-42 | Chemical Formula: C48H32N2 Exact Mass: 636.257 Molecular Weight: 636.798 |
| 3-2 | Chemical Formula: C48H14D18N2Exact Mass: 654.370 Molecular Weight: 654.908 | 3-26 | Chemical Formula: C48H18D14N2 Exact Mass: 650.344 Molecular Weight: 650.883 |
| 3-50 | Chemical Formula: C48D32N2Exact Mass: 668.457 Molecular Weight: 668.993 | | |

### <Experimental Examples>

### <Experimental Example 1>

### 1) Manufacture of Organic Light Emitting Device

A glass substrate, in which ITO was thinly coated to have a thickness of 1,500 Å, was ultrasonically washed with distilled water. When the washing with distilled water is finished, the glass substrate was ultrasonically washed with a solvent such as acetone, methanol, and isopropyl alcohol, was dried and then was subjected to UVO treatment for 5 minutes by using UV in a UV washing machine. Thereafter, the substrate was transferred to a plasma washing machine (PT, plasma treatment), and then was subjected to plasma treatment in a vacuum state for an ITO work function and in order to remove a residual film, and was transferred to a thermal deposition apparatus for organic deposition.

As the common layers, the hole injection layer 4,4',4'-tris[2-naphthyl(phenyl)amino]triphenylamine (2-TNATA) and the hole transport layer N,N'-di(1-naphthyl)-N,N'-diphenyl-(1,1'-biphenyl)-4,4'-diamine (NPB) were formed on the ITO transparent electrode (positive electrode).

A light emitting layer was thermally vacuum deposited thereon as follows. The light emitting layer was deposited to have a thickness of 360 Å by using a heterocyclic compound of Chemical Formula 1 in the following Table 9 as a host and tris(2-phenylpyridine) iridium (Ir(ppy)₃) as a green phosphorescent dopant to dope the host with Ir(ppy)₃ in an amount of 7%. Thereafter, BCP as a hole blocking layer was deposited to have a thickness of 60 Å, and Alq₃ as an electron transport layer was deposited to have a thickness of 200 Å thereon. Finally, lithium fluoride (LiF) was deposited to have a thickness of 10 Å on the electron transport layer to form an electron injection layer, and then an aluminum (Al) negative electrode was deposited to have a thickness of 1200 Å on the electron injection layer to form a negative electrode, thereby manufacturing an organic electroluminescence device.

Meanwhile, all the organic compounds required for manufacturing an OLED device were subjected to vacuum sublimed purification under 10⁻⁸ to 10⁻⁶ torr for each material, and used for the manufacture of OLED.

The results of measuring the driving voltage, light emitting efficiency, color coordinate (CIE) and service life of the organic light emitting device manufactured according to the present invention are shown in the following Table 9.

**[Table 9]**

| | Light emitting layer Compound | Driving voltage (V) | Light emitting efficiency (cd/A) | Color EL color | Service life (T₉₀) |
|---|---|---|---|---|---|
| Example 1 | 1-1 | 4.69 | 74.4 | | 356 |
| Example 2 | 1-4 | 4.63 | 73.5 | | 358 |
| Example 3 | 1-17 | 4.57 | 77.3 | | 358 |
| Example 4 | 1-19 | 4.65 | 73.6 | | 360 |
| Example 5 | 1-51 | 4.99 | 77.7 | | 397 |
| Example 6 | 1-77 | 4.53 | 73.5 | | 340 |
| Example 7 | 1-111 | 4.79 | 77.9 | | 381 |
| Example 8 | 1-113 | 4.55 | 78.2 | | 374 |
| Example 9 | 1-116 | 4.65 | 73.9 | | 382 |
| Example 10 | 1-118 | 4.55 | 72.8 | | 390 |
| Example 11 | 1-127 | 4.94 | 81.3 | Green | 413 |
| Example 12 | 1-133 | 4.51 | 73.9 | | 326 |
| Example 13 | 1-142 | 4.86 | 77.2 | | 385 |
| Example 14 | 1-147 | 4.56 | 73.2 | | 352 |
| Example 15 | 1-160 | 4.81 | 75.3 | | 399 |
| Example 16 | 1-161 | 4.47 | 74.4 | | 311 |
| Example 17 | 1-167 | 4.88 | 78.9 | | 343 |
| Example 18 | 1-169 | 4.62 | 73.1 | | 363 |
| Example 19 | 1-178 | 4.92 | 76.3 | | 420 |
| Comparative Example 1 | Ref. 1 | 5.56 | 63.6 | | 176 |
| Comparative Example 2 | Ref. 2 | 5.83 | 61.0 | | 181 |
| Comparative Example 3 | Ref. 3 | 5.64 | 62.4 | | 185 |
| Comparative Example 4 | Ref. 4 | 5.92 | 64.2 | | 198 |
| Comparative Example 5 | Ref. 5 | 5.73 | 62.6 | | 193 |
| Comparative Example 6 | Ref. 6 | 5.77 | 61.1 | | 188 |
| Comparative Example 7 | Ref. 7 | 5.74 | 61.3 | | 170 |
| Comparative Example 8 | Ref. 8 | 5.54 | 64.3 | | 199 |

| | | | |
|---|---|---|---|
| 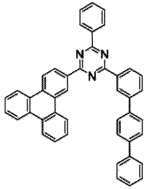 | 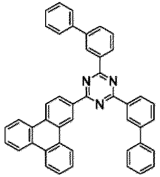 | 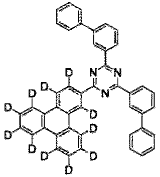 | 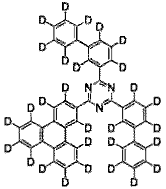 |
| Ref. 1 | Ref. 2 | Ref. 3 | Ref. 4 |
| 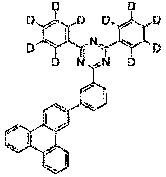 | 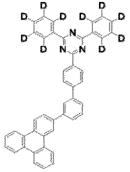 | 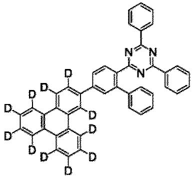 | 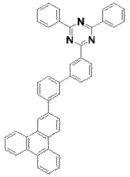 |
| Ref. 5 | Ref. 6 | Ref. 7 | Ref. 8 |

As can be confirmed from the results in Table 9, it could be confirmed that the organic light emitting device using the heterocyclic compound of the present invention as a light emitting layer material has a low driving voltage remarkably improved light emitting efficiency and service life compared to the organic light emitting devices of Comparative Examples 1 to 8.

The heterocyclic compound according to the present invention is a compound substituted with deuterium, and the compounds of Comparative Examples 1 to 8 are compounds substituted with hydrogen or partially substituted with deuterium. Compounds substituted with deuterium, whose atomic mass is twice that of hydrogen, can have lower ground-state energy due to lower zero-point energy and vibrational energy than compounds substituted with hydrogen, and can result in a thin film in an amorphous state due to a reduction in collision caused by intermolecular vibration, and thus, can improve the service life of an organic light emitting device.

Since the compounds substituted with deuterium have low ground-state energy, the stability of the compound is enhanced and the dissociation energy of the C-D bond is high, so that the stability of the molecule may be enhanced, thereby improving the service life of the organic light emitting device.

Specifically, it can be confirmed that Heterocyclic Compound 1-118 substituted with deuterium according to the present invention has improved driving voltage and service life compared to the Comparative Example Compounds Ref. 1, 2, and 8 substituted with hydrogen due to the stability and non-crystallinity of the molecule, and the low vibrational energy of the heterocyclic compound substituted with deuterium may minimize energy loss and facilitate energy transfer to the dopant to improve the light emitting efficiency of the organic light emitting device.

In addition, when Heterocyclic Compounds 1-1 and 1-133 substituted with deuterium according to the present invention are compared with the Comparative Example Compounds Refs. 5 and 6, Compounds 1-1 and 1-133 are compounds in which triphenylene and an aryl group, which is a linker, are substituted with deuterium, and the Comparative Example Compounds Refs. 5 and 6 have an aryl group at the triazine end substituted with deuterium.

In general, the host of an organic light emitting device may generate a radical cation in a substituent having a HOMO orbital due to the excitation of electrons, and when the cation cannot be effectively stabilized, the efficiency and service life of the device may be inhibited.

Specifically, when Compounds 1-1 and 1-133 substituted with deuterium according to the present invention generate a radical cation, the deuterium may improve the service life of the organic light emitting device by stabilizing the radical cation.

In contrast, it was confirmed that Comparative Example Compounds Refs. 5 and 6 had only the aryl group at the triazine end substituted with deuterium, and thus, could not contribute to the stabilization of radical cations.

Therefore, it could be confirmed that the service life of the organic light emitting device using Compound 1-1 or 1-133 in which triphenylene and an aryl group, which is a linker, are substituted with deuterium as the material of the light emitting device is improved.

Furthermore, Heterocyclic Compound 1-127 substituted with deuterium according to the present invention could improve the light emitting efficiency of an organic light emitting device by reducing the loss of energy due to the low vibrational energy of the compound, and the service life of the organic light emitting device may be improved by lowering the ground-state energy.

In contrast, the fully deuterated Compound 1-127 has a high packing density due to vibrational energy lower than Compound 1-113 in which only triphenylene and an aryl group, which is a linker, are substituted with deuterium, which could confirm that the driving voltage could be inhibited by forming an excessive mobility in the light emitting layer of the organic light emitting device to leak holes and electrons to the peripheral layer.

### <Experimental Example 2>

### 1) Manufacture of Organic Light Emitting Device

A glass substrate, in which ITO was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, dried and then was subjected to UVO treatment for 5 minutes using UV in a UV cleaning machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

As the common layers, the hole injection layer 4,4',4"-tris[2-naphthyl(phenyl)amino]triphenylamine (2-TNATA) and the hole transport layer N,N'-di(1-naphthyl)-N,N'-diphenyl-(1,1'-biphenyl)-4,4'-diamine (NPB) were formed on the ITO transparent electrode (positive electrode).

A light emitting layer was thermally vacuum deposited thereon as follows. The light emitting layer was deposited to have a thickness of 360 Å from one supply source after pre-mixing one type of the heterocyclic compound of Chemical Formula 1 and one type of compound of Chemical Formula 2 as hosts, and then was deposited by doping the host with Ir(ppy)₃ as a green phosphorescent dopant in an amount of 7% of the deposition thickness of the light emitting layer. Thereafter, BCP as a hole blocking layer was deposited to have a thickness of 60 Å, and Alq₃ as an electron transport layer was deposited to have a thickness of 200 Å thereon. Finally, lithium fluoride (LiF) was deposited to have a thickness of 10 Å on the electron transport layer to form an electron injection layer, and then an aluminum (Al) negative electrode was deposited to have a thickness of 1,200 Å on the electron injection layer to form a negative electrode, thereby manufacturing an organic electroluminescence device.

In the following Table 10, Examples and Comparative Examples were used as green hosts. As a green phosphorescent dopant, Ir(piq)₂(acac) was used.

Meanwhile, all the organic compounds required for manufacturing an OLED device were subjected to vacuum sublimed purification under 10⁻⁸ to 10⁻⁶ torr for each material, and used for the manufacture of OLED.

For the organic electroluminescence device manufactured as described above, electroluminescence (EL) characteristics were measured by M7000 manufactured by McScience Inc., and based on the measurement result thereof, T₉₀ was measured by a service life measurement device (M6000) manufactured by McScience Inc., when the reference luminance was 6,000 cd/m².

The results of measuring the driving voltage, light emitting efficiency, color coordinate (CIE) and service life of the organic light emitting device manufactured according to the present invention are shown in the following Table 10.

**[Table 10]**

| | Light emitting layer Compound | Ratio | Driving voltage (V) | Effici ency (cd/A) | Color EL color | Service life (T₉₀) |
|---|---|---|---|---|---|---|
| Example 20 | 1-118 : 2-42 | 1 : 8 | 4.62 | 53.7 | Green | 315 |
| Example 21 | | 1 : 5 | 4.58 | 56.1 | | 423 |
| Example 22 | | 1 : 2 | 4.41 | 82.3 | | 565 |
| Example 23 | | 1 : 1 | 4.47 | 70.1 | | 512 |
| Example 24 | | 2 : 1 | 4.38 | 69.4 | | 487 |
| Example 25 | | 5 : 1 | 4.31 | 68.1 | | 420 |
| Example 26 | | 8 : 1 | 4.20 | 67.3 | | 335 |
| Example 27 | 1-19 : 3-2 | 1 : 2 | 4.34 | 82.7 | | 571 |
| Example 28 | | 1 : 1 | 4.42 | 80.2 | | 530 |
| Example 29 | | 2 : 1 | 4.57 | 78.1 | | 508 |
| Example 30 | 1-169 : 3-26 | 1 : 2 | 4.38 | 81.0 | | 615 |
| Example 31 | | 1 : 1 | 4.45 | 79.8 | | 562 |
| Example 32 | | 2 : 1 | 4.58 | 78.4 | | 527 |
| Example 33 | 1-127 : 3-50 | 1 : 2 | 4.59 | 84.3 | | 642 |
| Example 34 | | 1 : 1 | 4.72 | 82.6 | | 622 |
| Example 35 | | 2 : 1 | 4.94 | 80.2 | | 597 |
| Comparative Example 9 | Ref. 8 : 3-2 | 1 : 2 | 5.59 | 71.4 | | 331 |
| Comparative Example 10 | | 1 : 1 | 5.72 | 69.7 | | 311 |
| Comparative Example 11 | | 2 : 1 | 5.94 | 67.3 | | 266 |

In the results of Table 10, it could be confirmed that when the compound represented by Chemical Formula 1, which is the heterocyclic compound of the present invention, is used as an N-type host and the compound represented by Chemical Formula 2 of the present invention is used as a P-type host to be deposited by mixing the two compounds, the light emitting efficiency and service life of the organic light emitting device are improved. From this result, it can be expected that an exciplex phenomenon will occur when the two compounds are mixed and deposited.

The exciplex phenomenon is a phenomenon in which energy with a magnitude of the HOMO energy level of the donor (p-host) and the LUMO energy level of the acceptor (n-host) is released due to an electron exchange between two molecules. When the exciplex phenomenon between two molecules occurs, a reverse intersystem crossing (RISC) occurs, and the internal quantum efficiency of fluorescence may be increased to 100% due to the RISC. When a donor with a good hole transport capacity (p-host) and an acceptor with a good electron transport capacity (n-host) are used as hosts for the light emitting layer, holes are injected into the p-host and electrons are injected into the n-host, so that the driving voltage of the organic light emitting device can be lowered, which can help to improve the service life of the organic light emitting device.

The compound represented by Chemical Formula 2 is a compound substituted with hydrogen, partially substituted with deuterium, or fully substituted with deuterium. Compounds substituted with deuterium, whose atomic mass is twice that of hydrogen, can have lower ground-state energy due to lower zero-point energy and vibrational energy than compounds substituted with hydrogen, and can result in a thin film in an amorphous state due to a reduction in collision caused by intermolecular vibration, and thus, can improve the service life of an organic light emitting device.

Since the compounds substituted with deuterium have low ground-state energy, the stability of the compound is enhanced and the dissociation energy of the C-D bond is high, so that the stability of the molecule may be enhanced, thereby improving the service life of the organic light emitting device.

In the present invention, the compound represented by Chemical Formula 2 serves as a donor and the compound represented by Chemical Formula 1 serves as an acceptor, so that it could be confirmed that when the compounds were used as a host of a light emitting layer, excellent characteristics of the organic light emitting device were exhibited.

## Claims

1. A heterocyclic compound represented by the following Chemical Formula 1: 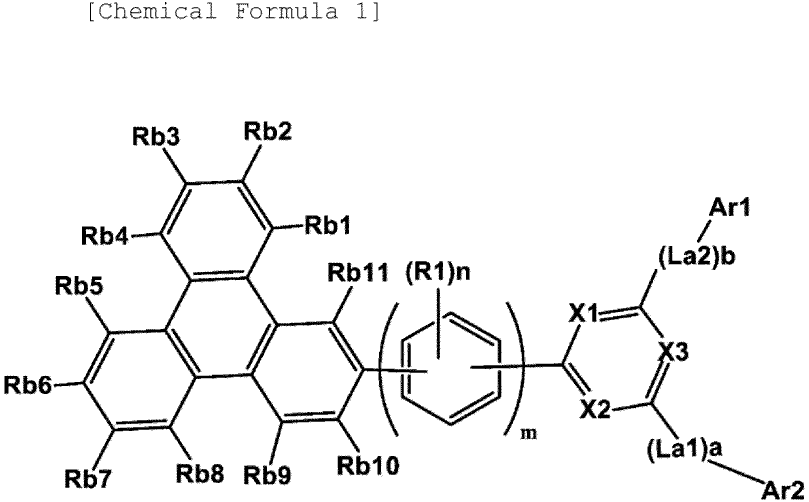 wherein, in Chemical Formula 1,
X1 to X3 are the same as or different from each other, and are each independently N; or CRa1, and at least one is N,
La1 and La2 are the same as or different from each other, and are each independently a direct bond; a substituted or unsubstituted C6 to C60 arylene group; or a substituted or unsubstituted C2 to C60 heteroarylene group,
R1 is hydrogen; or deuterium,
Ra1, Ar1 and Ar2 are the same as or different from each other, and are each independently hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; SiRR'R"; or -P(=O)RR',
Rb1 to Rb11 are the same as or different from each other, and are each independently hydrogen; or deuterium, and at least one of Rb1 to Rb11 is deuterium,
R, R' and R" are the same as or different from each other, and are each independently hydrogen; deuterium; a substituted or unsubstituted C1 to C60 alkyl group; or a substituted or unsubstituted C6 to C60 aryl group,
a, b and m are an integer from 1 to 4,
n is an integer from 0 to 4, and
when a, b, n and m are 2 or higher, substituents in the parenthesis are the same as or different from each other.

2. The heterocyclic compound of claim 1, wherein the 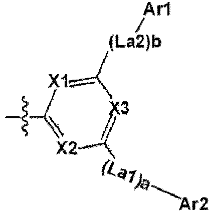 of Chemical Formula 1 is represented by the following Chemical Formula 1-1 or 1-2: 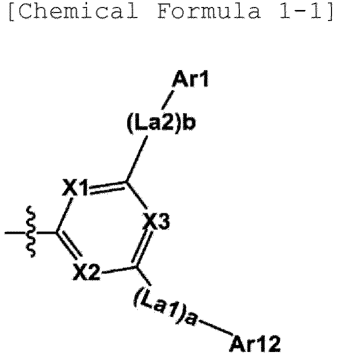 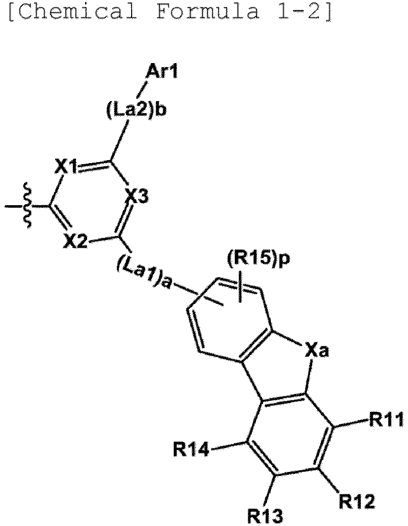 in Chemical Formulae 1-1 and 1-2,
the definitions of X1 to X3, La1, La2, a, b and Ar1 are the same as the definitions in Chemical Formula 1,
Xa is O; or S,
Ar12 is a substituted or unsubstituted C6 to C60 aryl group,
R11 to R15 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; and a substituted or unsubstituted C2 to C60 heteroaryl group, or two or more adjacent groups are bonded to each other to form a substituted or unsubstituted aromatic hydrocarbon ring or a substituted or unsubstituted hetero ring, and
p is an integer from 0 to 3, and when p is 2 or higher, substituents in the parenthesis are the same as or different from each other.

3. The heterocyclic compound of claim 1, wherein Chemical Formula 1 is represented by any one of the following Chemical Formulae 3 to 5: 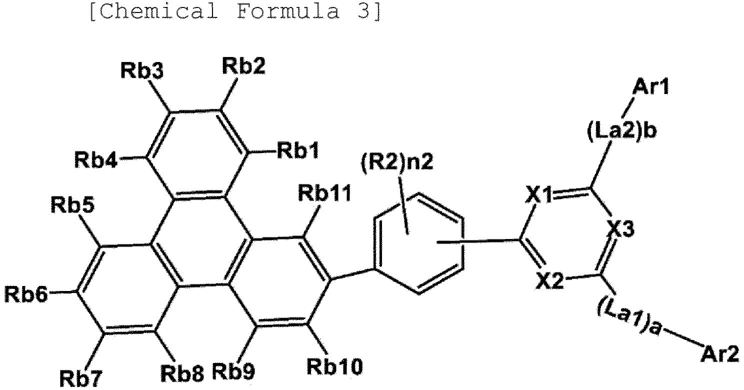 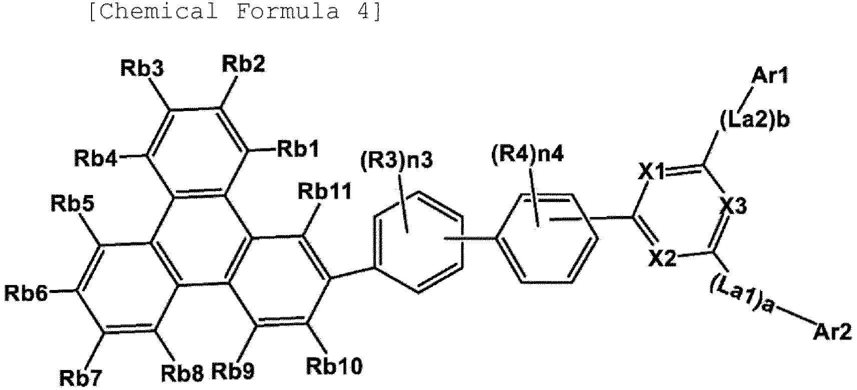 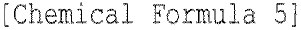 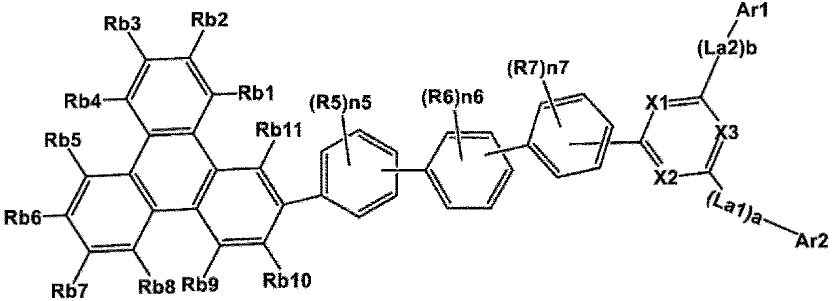 in Chemical Formulae 3 to 5,
the definitions of Rb1 to Rb11, X1 to X3, Ar1, La1, La2, a, b and Ar2 are the same as the definitions in Chemical Formula 1, and
R2 to R7 are the same as or different from each other, and are each independently hydrogen; or deuterium, n2 to n7 are the same as or different from each other, and are each independently an integer of 0 to 4, and when n2 to n7 are 2 or higher, substituents in the parenthesis are the same as or different from each other.

4. The heterocyclic compound of claim 1, wherein Chemical Formula 1 is represented by the following Structural Formulae A to C, a deuterium content of the following Structural Formula A is 1% to 100%, a deuterium content of the following Structural Formula B is 0% to 100%, and a deuterium content of the following Structural Formula C is 0% to 100%: 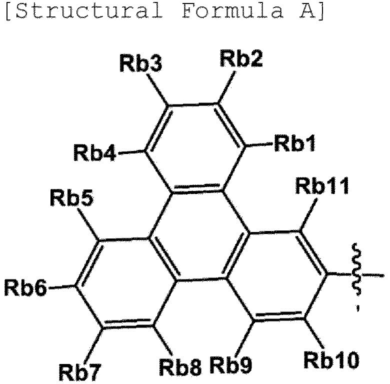 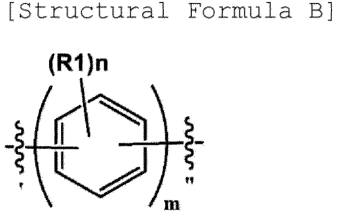 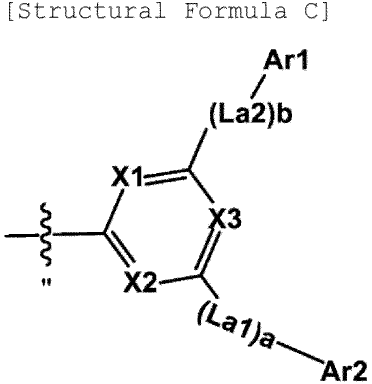 in Structural Formulae A to C,
the definition of each substituent is the same as the definition in Chemical Formula 1, and
the 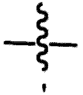 and 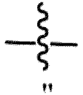 mean a position where moieties are connected to each other, and the same symbols are connected to each other.

5. The heterocyclic compound of claim 1, wherein a deuterium content of Chemical Formula 1 is 70% or more and 90% or less.

6. The heterocyclic compound of claim 4, wherein a deuterium content of Structural Formula A is 70% or more and 90% or less.

7. The heterocyclic compound of claim 1, wherein Chemical Formula 1 is represented by any one of the following compounds:
| | | | |
|---|---|---|---|
| 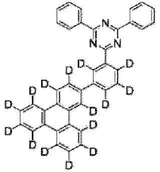 | 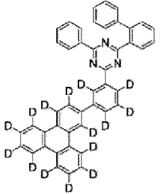 | 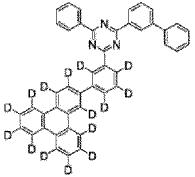 | 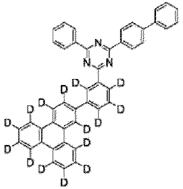 |
| 1-1 | 1-2 | 1-3 | 1-4 |
| 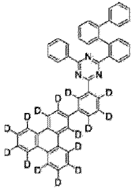 | 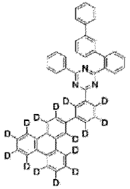 | 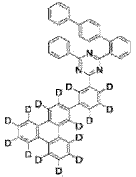 | 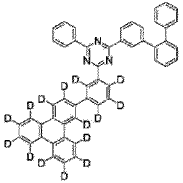 |
| 1-5 | 1-6 | 1-7 | 1-8 |
| 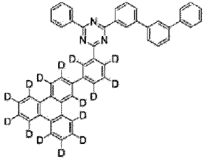 | 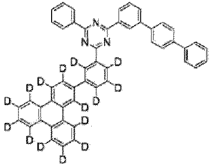 | 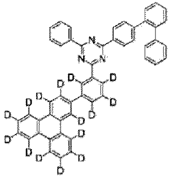 | 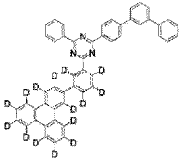 |
| 1-9 | 1-10 | 1-11 | 1-12 |
| 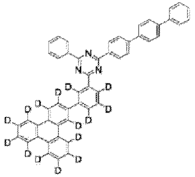 | 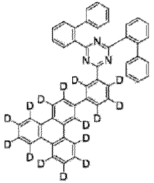 | 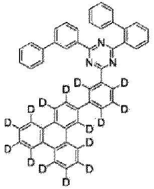 | 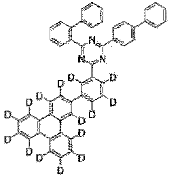 |
| 1-13 | 1-14 | 1-15 | 1-16 |
| 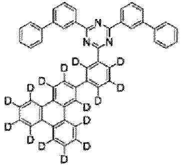 | 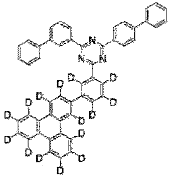 | 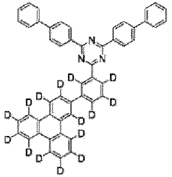 | 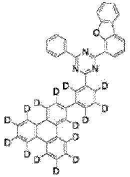 |
| 1-17 | 1-18 | 1-19 | 1-20 |
| 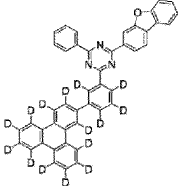 | 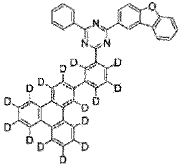 | 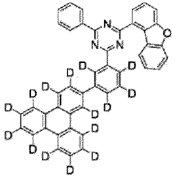 | 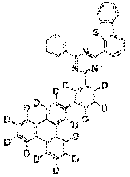 |
| 1-21 | 1-22 | 1-23 | 1-24 |
| 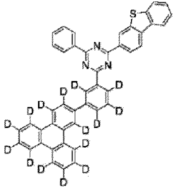 | 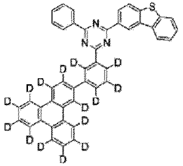 | 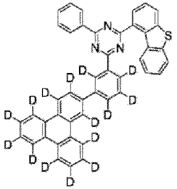 | 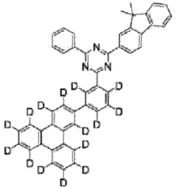 |
| 1-25 | 1-26 | 1-27 | 1-28 |
| 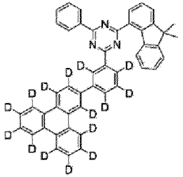 | 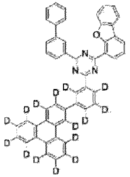 | 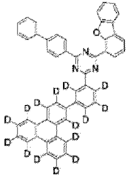 | 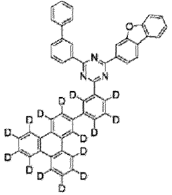 |
| 1-29 | 1-30 | 1-31 | 1-32 |
| 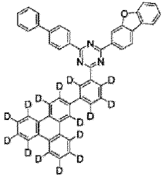 | 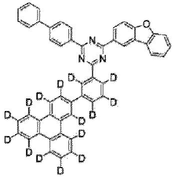 | 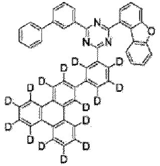 | 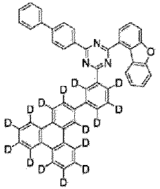 |
| 1-33 | 1-34 | 1-35 | 1-36 |
| 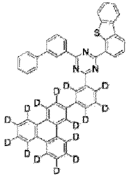 | 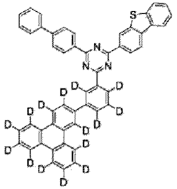 | 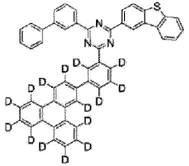 | 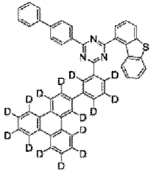 |
| 1-37 | 1-38 | 1-39 | 1-40 |
| 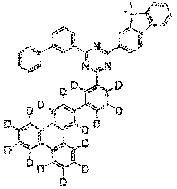 | 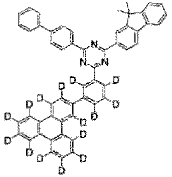 | 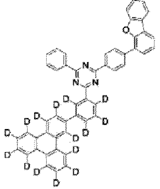 | 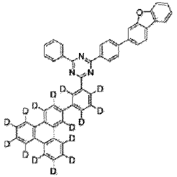 |
| 1-41 | 1-42 | 1-43 | 1-44 |
| 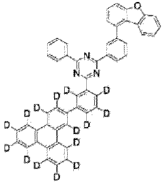 | 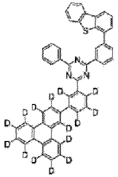 | 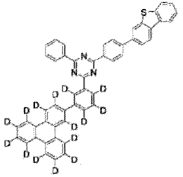 | 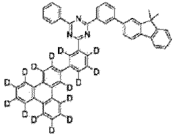 |
| 1-45 | 1-46 | 1-47 | 1-48 |
| 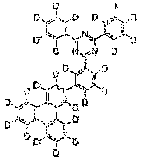 | 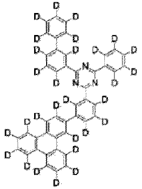 | 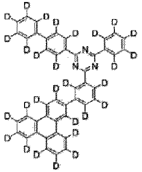 | 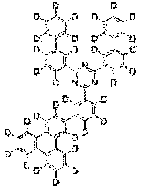 |
| 1-49 | 1-50 | 1-51 | 1-52 |
| 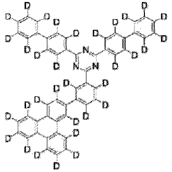 | 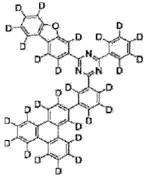 | 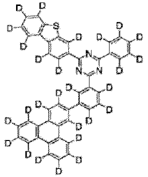 | 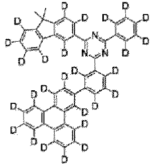 |
| 1-53 | 1-54 | 1-55 | 1-56 |
| 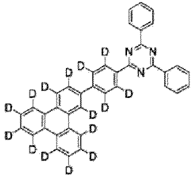 | 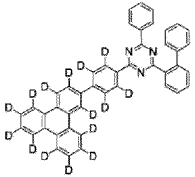 | 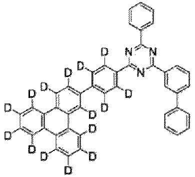 | 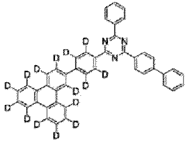 |
| 1-57 | 1-58 | 1-59 | 1-60 |
| 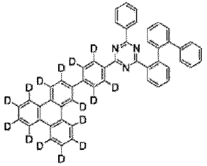 | 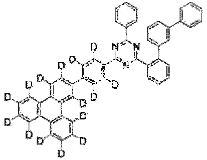 | 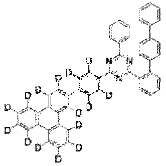 | 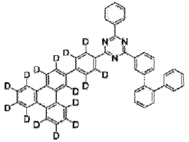 |
| 1-61 | 1-62 | 1-63 | 1-64 |
| 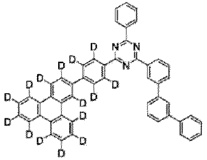 | 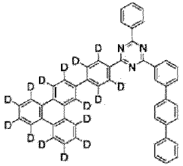 | 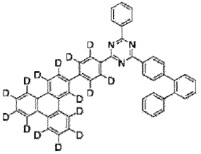 | 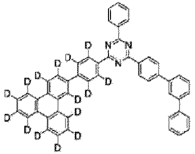 |
| 1-65 | 1-66 | 1-67 | 1-68 |
| 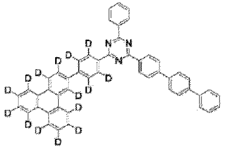 | 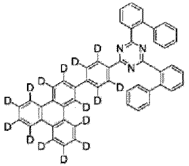 | 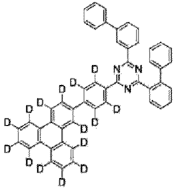 | 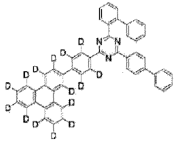 |
| 1-69 | 1-70 | 1-71 | 1-72 |
| 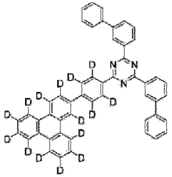 | 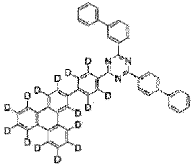 | 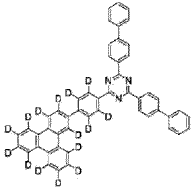 | 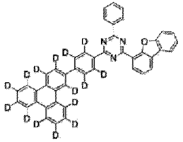 |
| 1-73 | 1-74 | 1-75 | 1-76 |
| 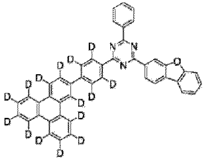 | 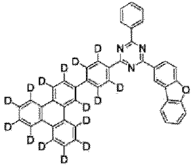 | 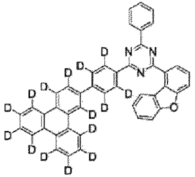 | 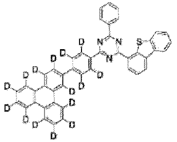 |
| 1-77 | 1-78 | 1-79 | 1-80 |
| 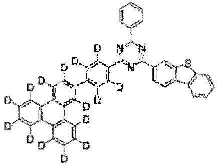 | 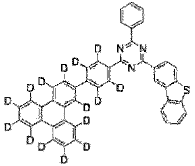 | 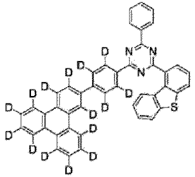 | 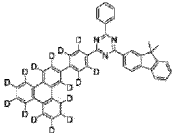 |
| 1-81 | 1-82 | 1-83 | 1-84 |
| 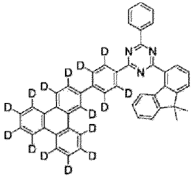 | 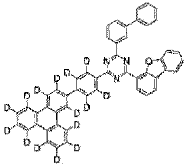 | 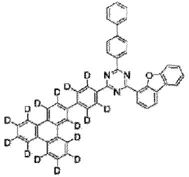 | 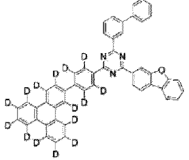 |
| 1-85 | 1-86 | 1-87 | 1-88 |
| 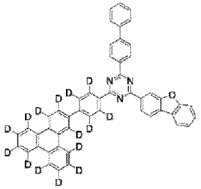 | 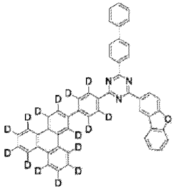 | 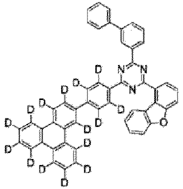 | 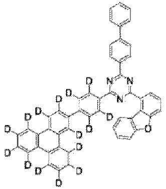 |
| 1-89 | 1-90 | 1-91 | 1-92 |
| 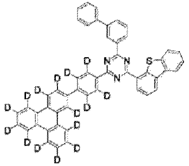 | 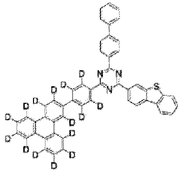 | 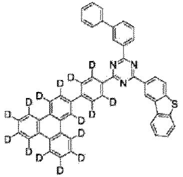 | 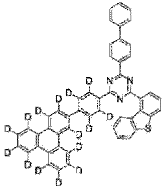 |
| 1-93 | 1-94 | 1-95 | 1-96 |
| 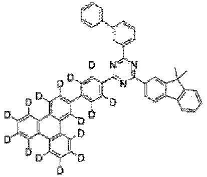 | 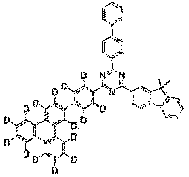 | 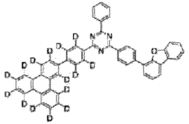 | 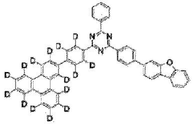 |
| 1-97 | 1-98 | 1-99 | 1-100 |
| 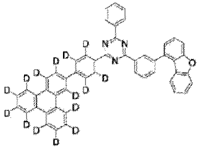 | 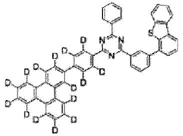 | 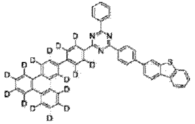 | 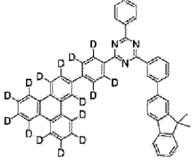 |
| 1-101 | 1-102 | 1-103 | 1-104 |
| 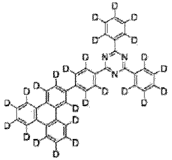 | 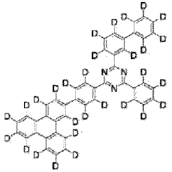 | 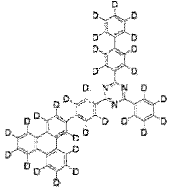 | 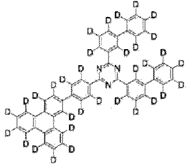 |
| 1-105 | 1-106 | 1-107 | 1-108 |
| 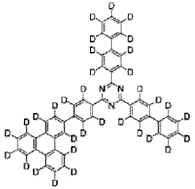 | 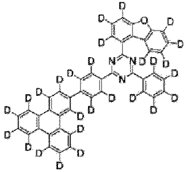 | 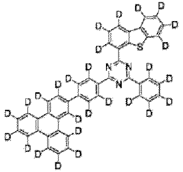 | 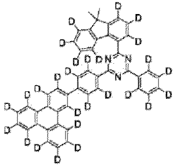 |
| 1-109 | 1-110 | 1-111 | 1-112 |
| 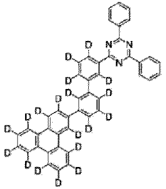 | 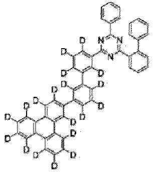 | 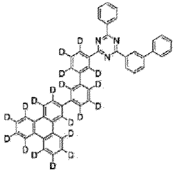 | 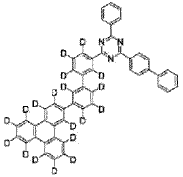 |
| 1-113 | 1-114 | 1-115 | 1-116 |
| 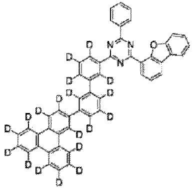 | 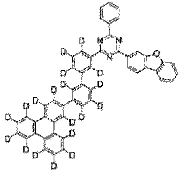 | 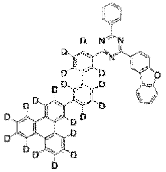 | 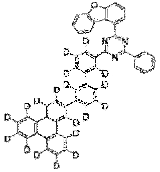 |
| 1-117 | 1-118 | 1-119 | 1-120 |
| 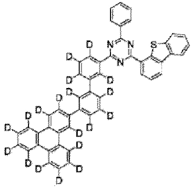 | 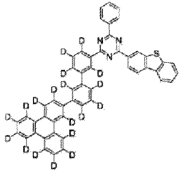 | 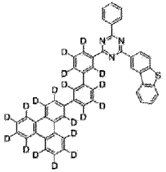 | 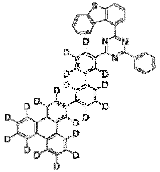 |
| 1-121 | 1-122 | 1-123 | 1-124 |
| 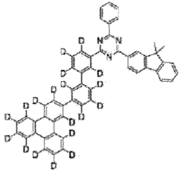 | 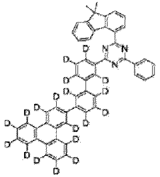 | 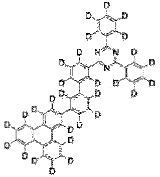 | 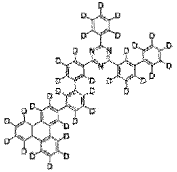 |
| 1-125 | 1-126 | 1-127 | 1-128 |
| 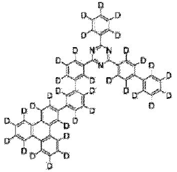 | 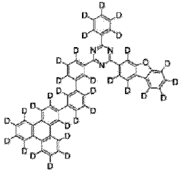 | 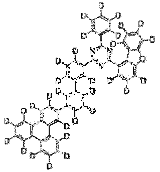 | 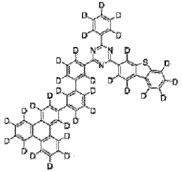 |
| 1-129 | 1-130 | 1-131 | 1-132 |
| 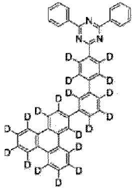 | 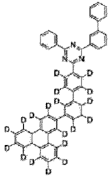 | 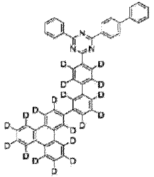 | 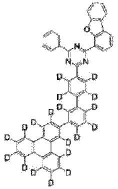 |
| 1-133 | 1-134 | 1-135 | 1-136 |
| 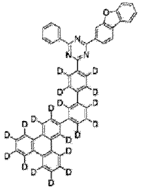 | 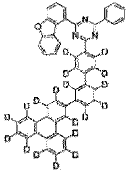 | 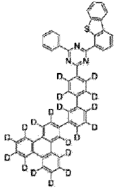 | 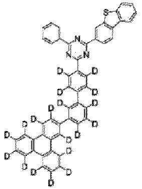 |
| 1-137 | 1-138 | 1-139 | 1-140 |
| 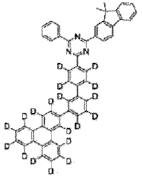 | 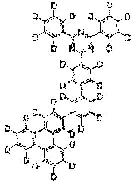 | 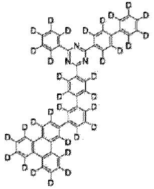 | 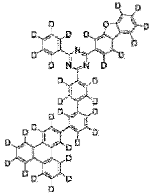 |
| 1-141 | 1-142 | 1-143 | 1-144 |
| 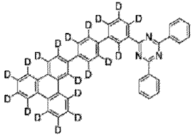 | 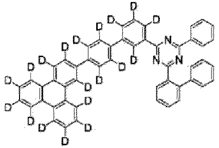 | 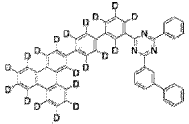 | 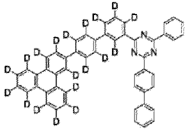 |
| 1-145 | 1-146 | 1-147 | 1-148 |
| 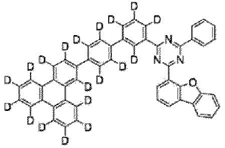 | 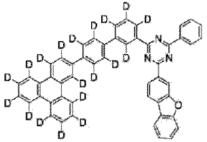 | 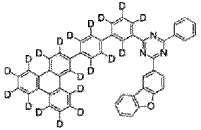 | 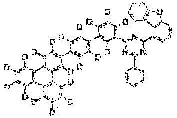 |
| 1-149 | 1-150 | 1-151 | 1-152 |
| 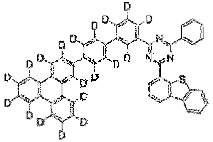 | 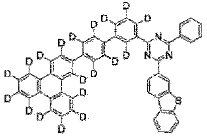 | 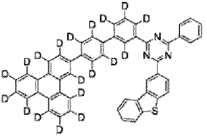 | 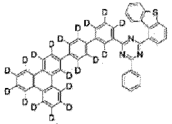 |
| 1-153 | 1-154 | 1-155 | 1-156 |
| 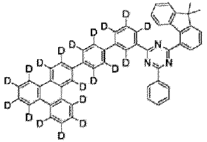 | 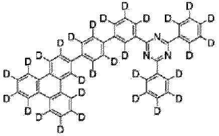 | 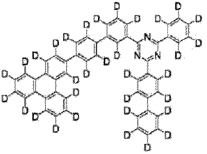 | 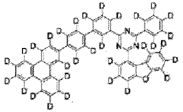 |
| 1-157 | 1-158 | 1-159 | 1-160 |
| 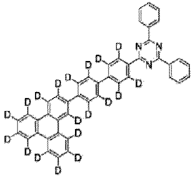 | 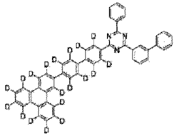 | 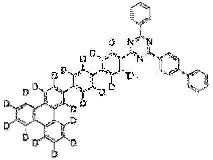 | 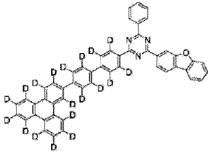 |
| 1-161 | 1-162 | 1-163 | 1-164 |
| 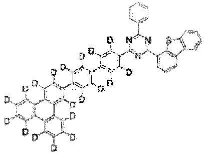 | 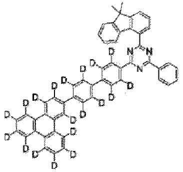 | 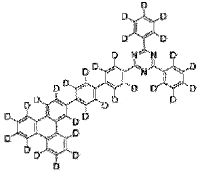 | 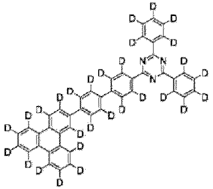 |
| 1-165 | 1-166 | 1-167 | 1-168 |
| 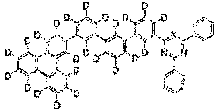 | 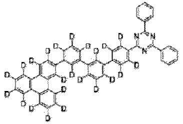 | 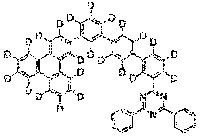 | 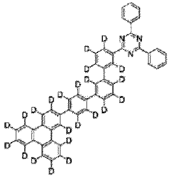 |
| 1-169 | 1-170 | 1-171 | 1-172 |
| 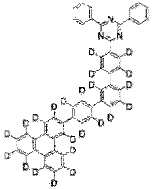 | 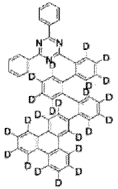 | 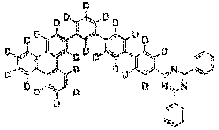 | 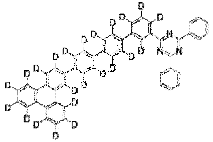 |
| 1-173 | 1-174 | 1-175 | 1-176 |
| 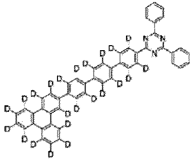 | 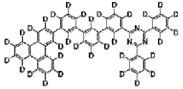 | 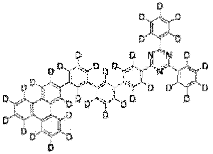 | 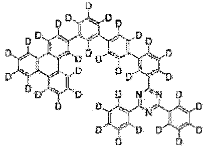 |
| 1-177 | 1-178 | 1-179 | 1-180 |
| 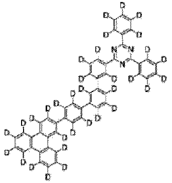 | 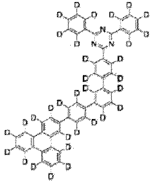 | 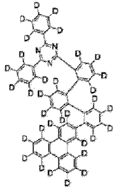 | 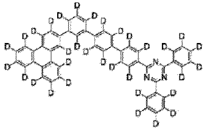 |
| 1-181 | 1-182 | 1-183 | 1-184 |
| 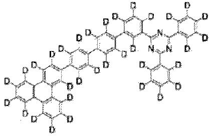 | 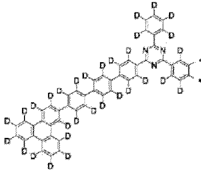 | 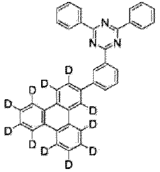 | 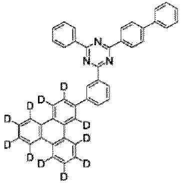 |
| 1-185 | 1-186 | 1-187 | 1-188 |
| 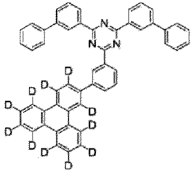 | 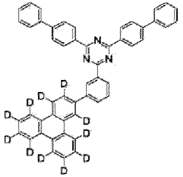 | 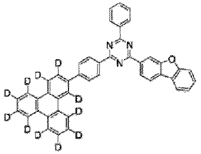 | 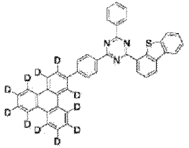 |
| 1-189 | 1-190 | 1-191 | 1-192 |
| 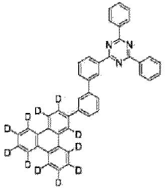 | 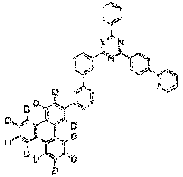 | 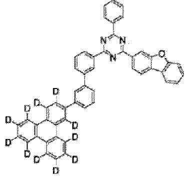 | 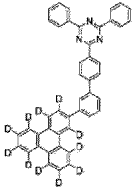 |
| 1-193 | 1-194 | 1-195 | 1-196 |
| 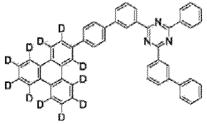 | 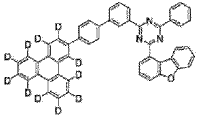 | 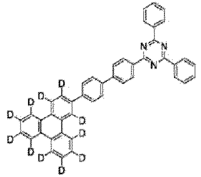 | 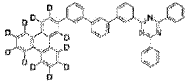 |
| 1-197 | 1-198 | 1-199 | 1-200 |

8. An organic light emitting device comprising:
a first electrode;
a second electrode provided to face the first electrode; and
an organic material layer having one or more layers provided between the first electrode and the second electrode,
wherein the one or more layers of the organic material layer comprise the heterocyclic compound according to any one of claims 1 to 7.

9. The organic light emitting device of claim 8, wherein the organic material layer comprising the heterocyclic compound further comprises a heterocyclic compound represented by the following Chemical Formula 2: 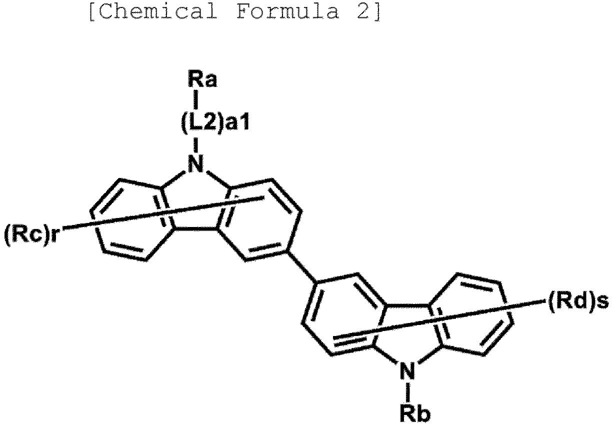 in Chemical Formula 2,
Rc and Rd are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P(=O)RR'; and -NRR', or two or more adjacent groups are bonded to each other to form a substituted or unsubstituted aromatic hydrocarbon ring or a substituted or unsubstituted hetero ring,
L2 is a direct bond; a substituted or unsubstituted C6 to C60 arylene group; or a substituted or unsubstituted C2 to C60 heteroarylene group,
Ra and Rb are the same as or different from each other, and are each independently -CN; -SiRR'R"; -P(=O)RR'; a substituted or unsubstituted C6 to C60 aryl group; or a substituted or unsubstituted C2 to C60 heteroaryl group,
R, R' and R" are the same as or different from each other, and are each independently hydrogen; deuterium; a substituted or unsubstituted C1 to C60 alkyl group; or a substituted or unsubstituted C6 to C60 aryl group,
a1 is an integer from 0 to 4,
r and s are an integer from 0 to 7, and
when a1, s and r are 2 or higher, substituents in the parenthesis are the same as or different from each other.

10. The organic light emitting device of claim 9, wherein the heterocyclic compound represented by Chemical Formula 2 is any one selected from the following compounds:
| | | | |
|---|---|---|---|
| 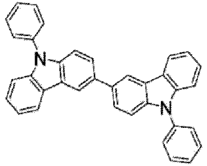 | 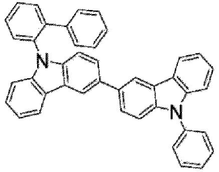 | 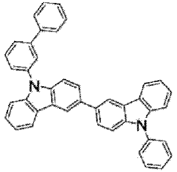 | 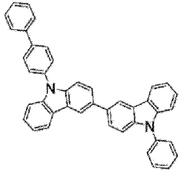 |
| 2-1 | 2-2 | 2-3 | 2-4 |
| 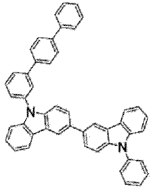 | 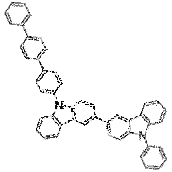 | 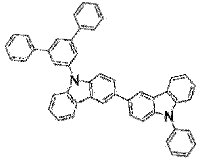 | 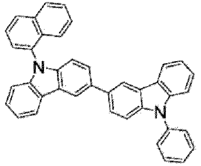 |
| 2-5 | 2-6 | 2-7 | 2-8 |
| 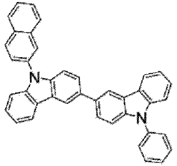 | 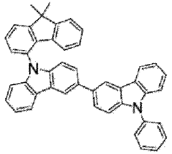 | 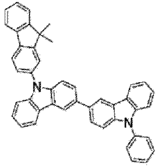 | 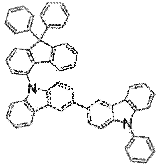 |
| 2-9 | 2-10 | 2-11 | 2-12 |
| 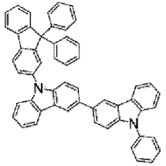 | 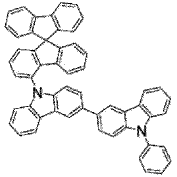 | 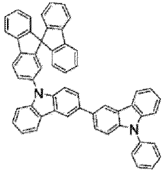 | 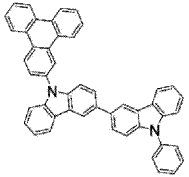 |
| 2-13 | 2-14 | 2-15 | 2-16 |
| 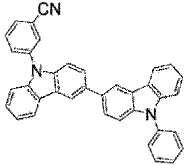 | 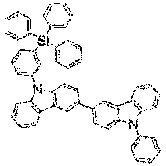 | 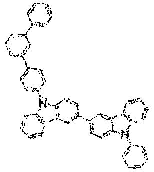 | 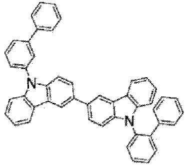 |
| 2-17 | 2-18 | 2-19 | 2-20 |
| 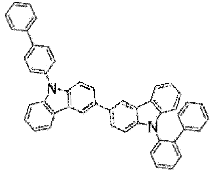 | 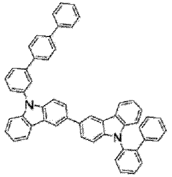 | 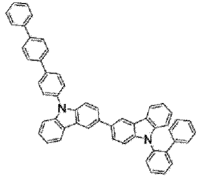 | 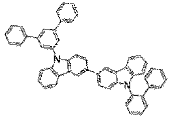 |
| 2-21 | 2-22 | 2-23 | 2-24 |
| 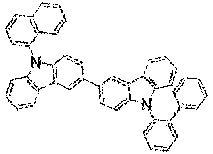 | 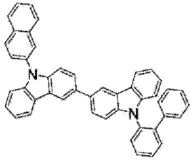 | 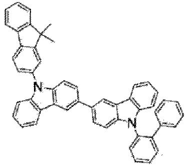 | 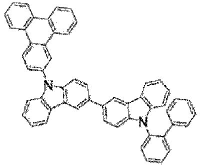 |
| 2-25 | 2-26 | 2-27 | 2-28 |
| 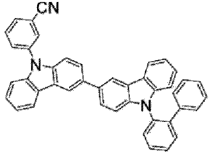 | 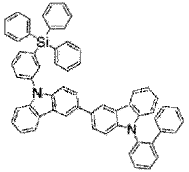 | 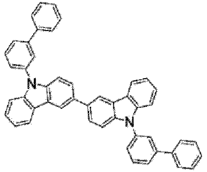 | 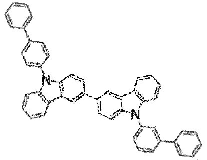 |
| 2-29 | 2-30 | 2-31 | 2-32 |
| 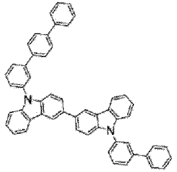 | 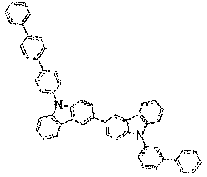 | 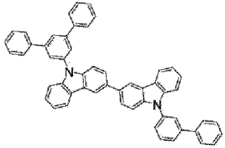 | 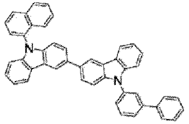 |
| 2-33 | 2-34 | 2-35 | 2-36 |
| 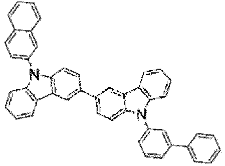 | 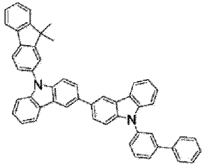 | 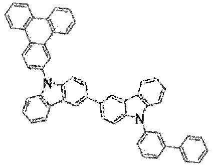 | 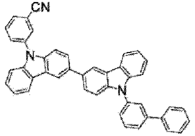 |
| 2-37 | 2-38 | 2-39 | 2-40 |
| 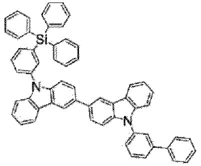 | 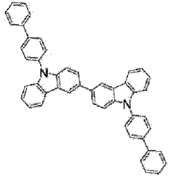 | 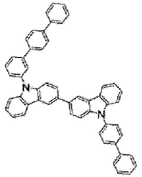 | 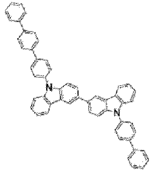 |
| 2-41 | 2-42 | 2-43 | 2-44 |
| 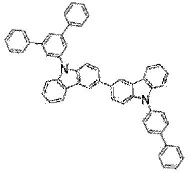 | 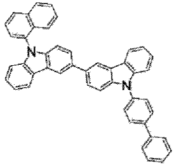 | 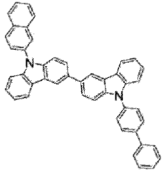 | 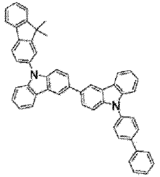 |
| 2-45 | 2-46 | 2-47 | 2-48 |
| 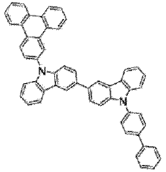 | 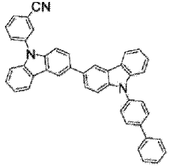 | 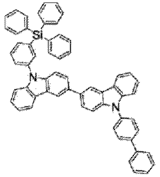 | 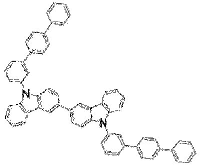 |
| 2-49 | 2-50 | 2-51 | 2-52 |
| 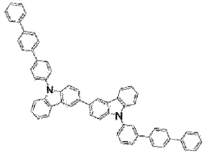 | 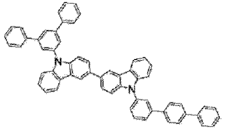 | 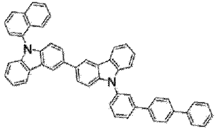 | 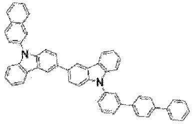 |
| 2-53 | 2-54 | 2-55 | 2-56 |
| 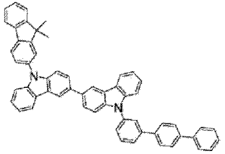 | 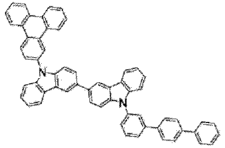 | 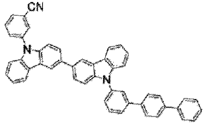 | 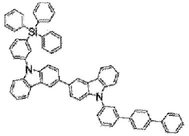 |
| 2-57 | 2-58 | 2-59 | 2-60 |
| 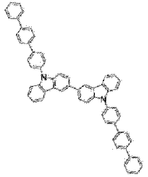 | 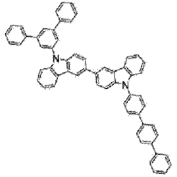 | 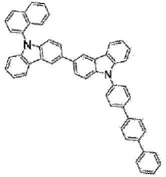 | 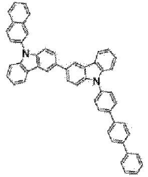 |
| 2-61 | 2-62 | 2-63 | 2-64 |
| 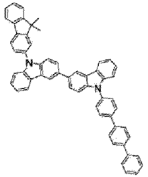 | 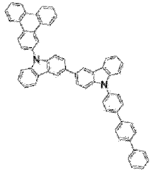 | 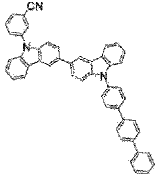 | 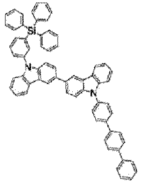 |
| 2-65 | 2-66 | 2-67 | 2-68 |
| 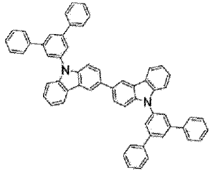 | 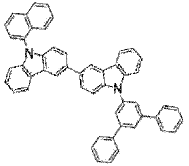 | 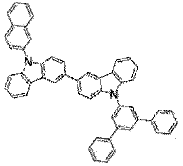 | 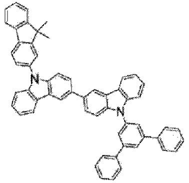 |
| 2-69 | 2-70 | 2-71 | 2-72 |
| 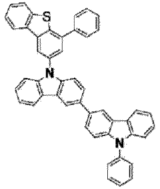 | 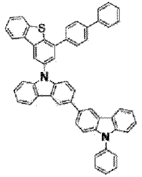 | 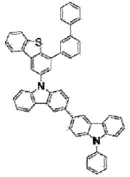 | 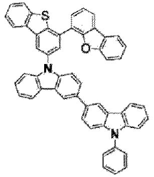 |
| 2-73 | 2-74 | 2-75 | 2-76 |
| 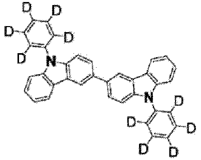 | 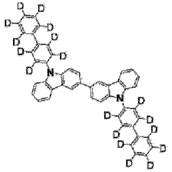 | 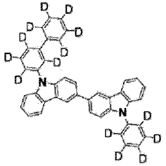 | 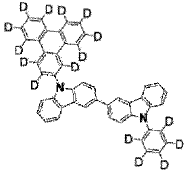 |
| 3-1 | 3-2 | 3-3 | 3-4 |
| 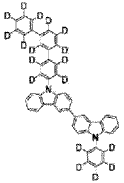 | 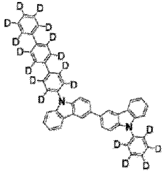 | 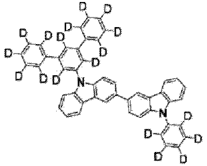 | 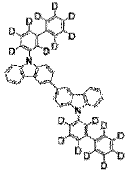 |
| 3-5 | 3-6 | 3-7 | 3-8 |
| 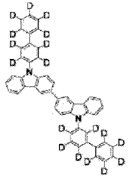 | 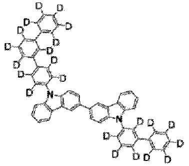 | 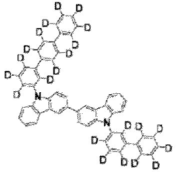 | 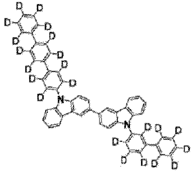 |
| 3-9 | 3-10 | 3-11 | 3-12 |
| 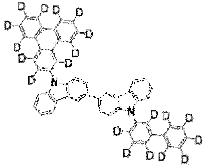 | 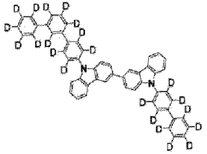 | 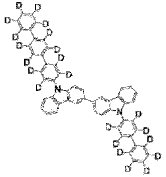 | 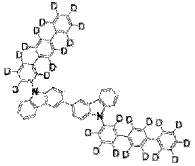 |
| 3-13 | 3-14 | 3-15 | 3-16 |
| 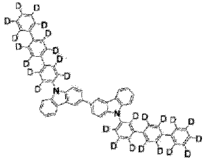 | 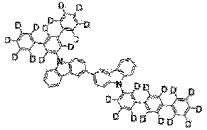 | 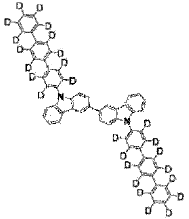 | 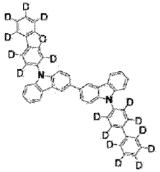 |
| 3-17 | 3-18 | 3-19 | 3-20 |
| 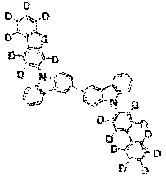 | 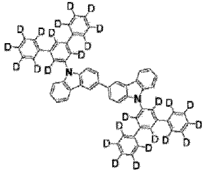 | 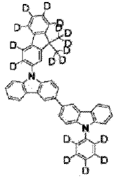 | 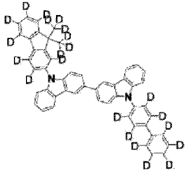 |
| 3-21 | 3-22 | 3-23 | 3-24 |
| 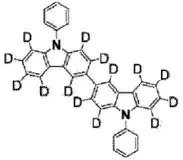 | 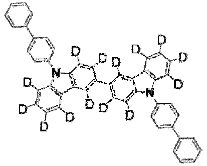 | 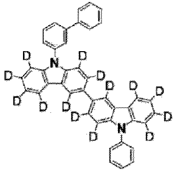 | 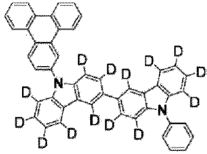 |
| 3-25 | 3-26 | 3-27 | 3-28 |
| 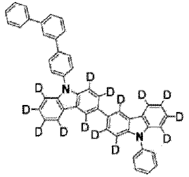 | 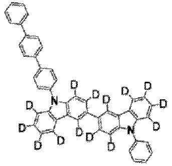 | 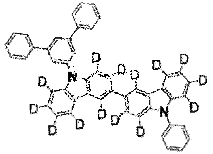 | 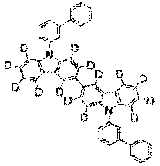 |
| 3-29 | 3-30 | 3-31 | 3-32 |
| 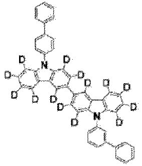 | 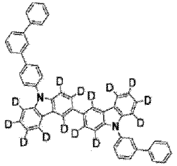 | 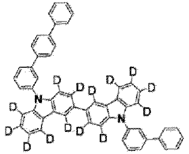 | 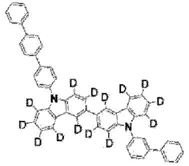 |
| 3-33 | 3-34 | 3-35 | 3-36 |
| 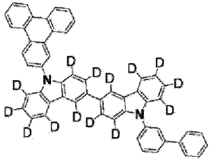 | 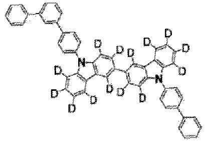 | 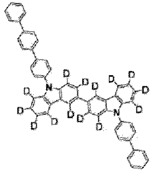 | 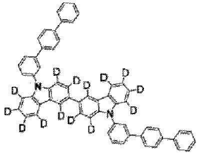 |
| 3-37 | 3-38 | 3-39 | 3-40 |
| 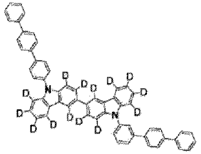 | 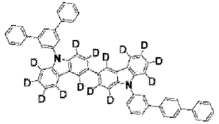 | 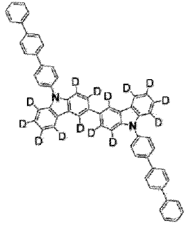 | 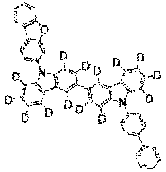 |
| 3-41 | 3-42 | 3-43 | 3-44 |
| 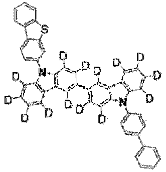 | 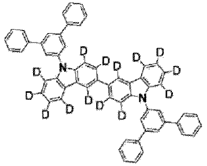 | 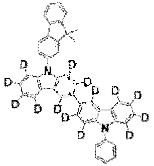 | 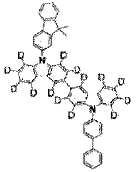 |
| 3-45 | 3-46 | 3-47 | 3-48 |
| 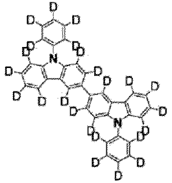 | 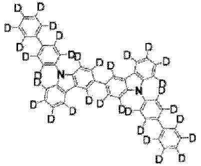 | 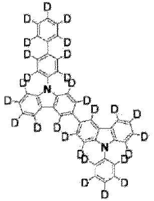 | 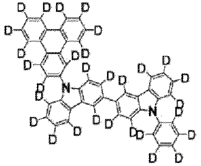 |
| 3-49 | 3-50 | 3-51 | 3-52 |
| 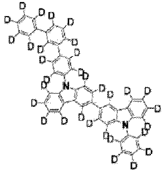 | 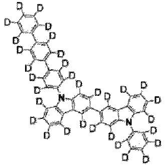 | 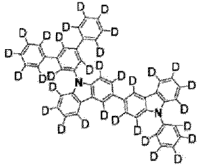 | 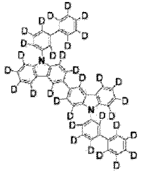 |
| 3-53 | 3-54 | 3-55 | 3-56 |
| 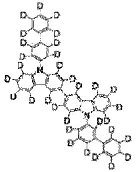 | 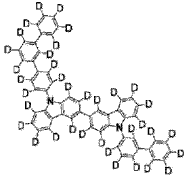 | 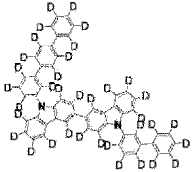 | 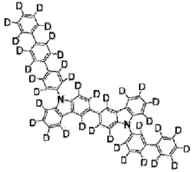 |
| 3-57 | 3-58 | 3-59 | 3-60 |
| 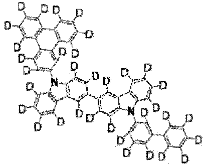 | 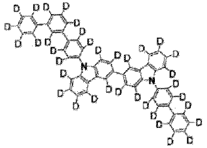 | 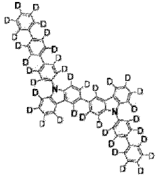 | 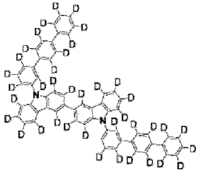 |
| 3-61 | 3-62 | 3-63 | 3-64 |
| 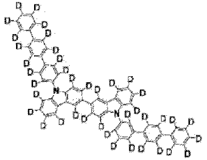 | 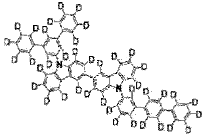 | 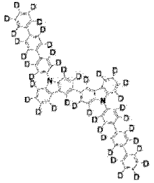 | 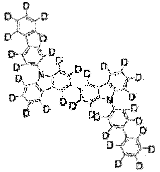 |
| 3-65 | 3-66 | 3-67 | 3-68 |
| 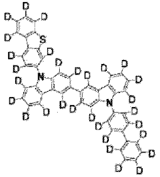 | 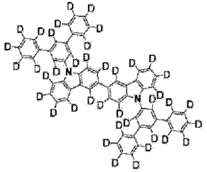 | 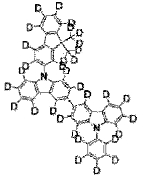 | 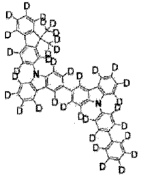 |
| 3-69 | 3-70 | 3-71 | 3-72 |
| 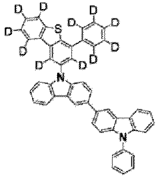 | 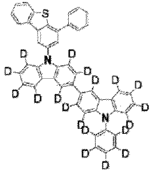 | 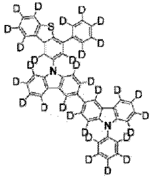 | 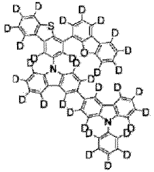 |
| 3-73 | 3-74 | 3-75 | 3-76 |

11. The organic light emitting device of claim 9, wherein a deuterium content of Chemical Formula 2 is 0%, 100% or 10% to 80%.

12. The organic light emitting device of claim 8, wherein the organic material layer comprises a light emitting layer, and the light emitting layer comprises the heterocyclic compound of Chemical Formula 1.

13. The organic light emitting device of claim 8, wherein the organic material layer comprises a light emitting layer, the light emitting layer comprises a host material, and the host material comprises the heterocyclic compound.

14. The organic light emitting device of claim 8, wherein the organic light emitting device further comprises one or two or more layers selected from the group consisting of a light emitting layer, a hole injection layer, a hole transport layer, an electron injection layer, an electron transport layer, an electron blocking layer and a hole blocking layer.

15. A composition for an organic material layer of an organic light emitting device, the composition comprising the heterocyclic compound according to any one of claims 1 to 7, and a heterocyclic compound represented by the following Chemical Formula 2: 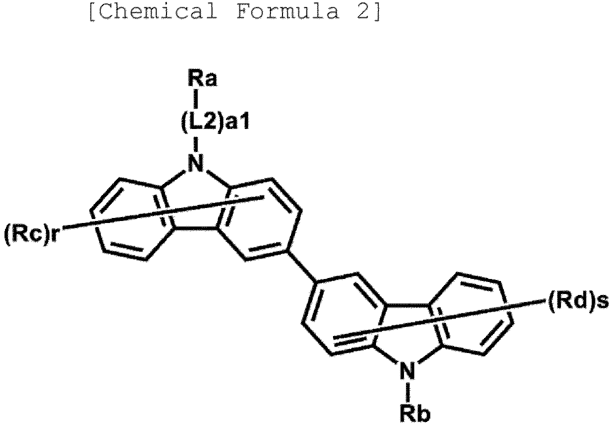 wherein, in Chemical Formula 2,
Rc and Rd are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P(=O)RR'; and -NRR', or two or more adjacent groups are bonded to each other to form a substituted or unsubstituted aromatic hydrocarbon ring or a substituted or unsubstituted hetero ring,
L2 is a direct bond; a substituted or unsubstituted C6 to C60 arylene group; or a substituted or unsubstituted C2 to C60 heteroarylene group,
Ra and Rb are the same as or different from each other, and are each independently -CN; -SiRR'R"; a substituted or unsubstituted C6 to C60 aryl group; or a substituted or unsubstituted C2 to C60 heteroaryl group,
R, R' and R" are the same as or different from each other, and are each independently hydrogen; deuterium; a substituted or unsubstituted C1 to C60 alkyl group; or a substituted or unsubstituted C6 to C60 aryl group,
a1 is an integer from 0 to 4,
r and s are an integer from 0 to 7, and
when a1, s and r are 2 or higher, substituents in the parenthesis are the same as or different from each other.

16. The composition of claim 15, wherein a weight ratio of the heterocyclic compound : the heterocyclic compound represented by Chemical Formula 2 in the composition is 1 : 10 to 10 : 1.
